(19) Europäisches Patentamt
European Patent Office
Office européen des brevets

(11) EP 0 559 046 B1

(12) **EUROPÄISCHE PATENTSCHRIFT**

(45) Veröffentlichungstag und Bekanntmachung des Hinweises auf die Patenterteilung:
**11.07.2001 Patentblatt 2001/28**

(51) Int Cl.$^7$: **C07D 211/26**, C07D 265/30, C07K 5/06, C07D 401/12, C07D 413/12

(21) Anmeldenummer: **93102767.6**

(22) Anmeldetag: **22.02.1993**

(54) **N-amidopiperidinyl (3/4)-oder N-amidino-1,4-oxazinyl(2)-substituierte Sulfonamide, Verfahren zur Herstellung und Verwendung als Thrombin-Inhibitoren**

N-amidinopiperidinyl(3/4)- or N-amidino-1,4-Oxazinyl(2)-substituted sulfonamides, process for their preparation and use as thrombin inhibitors

Sulfonamides substitués par un groupe N-amidinopipéridinyl(3/4)-ou N-amidino-1,4-oxazinyl(2), procédé pour leur préparation et utilisation comme inhibiteur de la thrombine

(84) Benannte Vertragsstaaten:
**AT BE CH DE DK ES FR GB GR IE IT LI LU MC NL PT SE**

(30) Priorität: **06.03.1992 CH 72892**
**21.01.1993 CH 18093**

(43) Veröffentlichungstag der Anmeldung:
**08.09.1993 Patentblatt 1993/36**

(73) Patentinhaber: **F. Hoffmann-La Roche AG**
**4002 Basel (CH)**

(72) Erfinder:
- **Ackermann, Jean**
  **CH-4056 Basel (CH)**
- **Banner, David**
  **CH-4054 Basel (CH)**
- **Gubernator, Klaus**
  **W-7800 Freiburg (DE)**
- **Hilpert, Kurt**
  **CH-4114 Hofstetten (CH)**
- **Schmid, Gérard**
  **CH-4468 Kienberg (CH)**

(74) Vertreter: **Braun, Axel et al**
**F.Hoffmann-La Roche AG**
**Patent Department (PLP),**
**124 Grenzacherstrasse**
**4070 Basel (CH)**

(56) Entgegenhaltungen:
**EP-A- 0 097 630**
**EP-A- 0 381 033**
**EP-A- 0 462 960**
**EP-A- 0 468 231**
**EP-A- 0 502 536**
**FR-A- 2 506 306**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 559 046 B1

Printed by Jouve, 75001 PARIS (FR)

## Beschreibung

Die Erfindung betrifft neue Sulfonamidocarboxamide der Formel

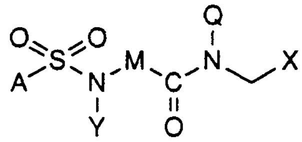 I

worin

| | |
|---|---|
| X | eine Gruppe der Formel $X^1$ oder $X^2$: |

($X^1$) 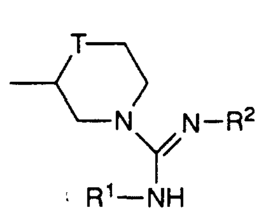 ($X^2$) 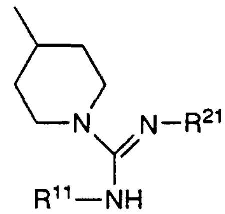

| | |
|---|---|
| T | $CH_2$ oder O, |
| $R^1$, $R^2$, $R^{11}$ und $R^{21}$ | unabhängig voneinander H oder COO-nieder-Alkyl, |
| Y | H oder, falls X eine Gruppe $X^2$ ist oder falls X eine Gruppe $X^1$ ist, in der zumindest eins von $R^1$ und $R^2$ nicht H ist, dann Y auch $CH_2COOH$ oder $SO_2$-A' sein kann, |
| A und A' | Aryl, Heteroaryl, Heterocyclyl, Alkyl oder Cycloalkyl, |
| Q | H, nieder-Alkyl oder nieder-Alkyl(OH, COOH oder COO-niederalkyl), |
| M | eine Gruppe der Formel $M^1$ ist oder, falls X eine Gruppe $X^2$ ist, oder falls X eine Gruppe $X^1$ ist und zumindest eins von $R^1$, $R^2$ und Q nicht H ist, und/oder falls A Alkyl oder Cycloalkyl ist, dann M auch eine Gruppe einer der Formeln $M^2$ bis $M^8$ sein kann, |

($M^1$) 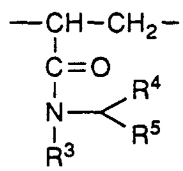 ($M^2$) 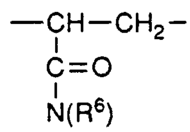

$-CH_2CH(NH(CO)_{1-2}R^7)-$ ($M^3$)

$-CH_2CH$(NHC(O)O-Benzyl)- ($M^4$)

$=CH(CH_2)_{1-2}R^7$ ($M^5$)

$=CHCH_2C(O)R^8$ ($M^6$)

$=CHCH_2NH(CO)_{1-2}R^7$ ($M^7$)

$=CHCH_2NHC(O)O$-Benzyl ($M^8$)

| | |
|---|---|
| $R^3$ | H, nieder-Alkyl oder -Alkenyl, Aryl, Heteroaryl, Cycloalkyl oder (Aryl, Heteroaryl oder Cycloalkyl)-niederalkyl, |
| $R^4$ | H, nieder-Alkyl, Aryl, Cycloalkyl, oder (Aryl oder Cycloalkyl)-niederalkyl, |
| $R^5$ | H, nieder-Alkyl oder gegebenenfalls über nieder-Alkylen gebundenes COOH, COO-nieder-Alkyl, nieder-Alkanoyl, OH, nieder-Alkanoyloxy, nieder-Alkoxy, Aryl-niederalkoxy, $CONH_2$, $CONHCH_2CH_2OH$, CONHOH, $CONHOCH_3$, CONHO-Benzyl, $CONHSO_2$-nieder-Alkyl, $CONHCH_2CH_2$-Aryl, CONH-Cycloalkyl, $CONHCH_2$-Heteroaryl, $NH_2$, NHCOO-nieder-Alkyl, NHCOO-nieder-Aralkyl, $NHSO_3H$, ($NHSO_2$ oder $NHSO_3$)-nieder-Alkyl, NH-nieder-Alkanoyl, NHCOCOOH, NHCOCOO-nieder-Alkyl, NH-Cycloalkyl, NH-(3,4-Dioxo-2-hydroxy-cyclobut-1-enyl), NH-[2-nieder-(Alkoxy oder -Alkenyloxy)-3,4-dioxocyclobut-1-enyl], $NHCH_2$-Heteroaryl, NHCOCO-(Aryl oder nieder-Alkyl), $NHCOCH_2Cl$, $NHCOCH_2O$-Aryl, $NHCOCH_2$-Aryl, NHCO-(Aryl oder Heteroaryl), $NHPO_3(R^9,R^{10})$, Heteroaryl oder gegebenenfalls durch O oder S unterbrochenes und gegebenenfalls durch bis zu 2 Substituenten aus der Gruppe von nieder-Alkyl, COOH, COO-nieder-Alkyl, $CH_2OH$ und $CH_2O$-Benzyl substituiertes $CON(CH_2)_{4-9}$, |
| $R^9$ und $R^{10}$ | H, nieder-Alkyl oder Phenyl, wobei falls Q, $R^1$, $R^2$, $R^3$ und $R^5$ gleichzeitig H sind, $R^4$ nicht Phenyl sein soll, |
| $N(R^6)$ | Benzylamino oder gegebenenfalls durch O oder S unterbrochenes und gegebenenfalls durch bis zu 2 Substituenten aus der Gruppe von nieder-Alkyl, COOH, COO-nieder-Alkyl, $CH_2OH$, $CH_2O$-Benzyl substituiertes $N(CH_2)_{4-9}$, |
| $R^7$ und $R^8$ | Aryl, Heteroaryl, Cycloalkyl oder Heterocyclyl, oder |
| $R^8$ | gegebenenfalls durch bis zu 2 Substituenten aus der Gruppe von Oxo, COO-nieder-Alkyl, $(CH_2)_{0-1}OH$, $(CH_2)_{0-1}OCO$-nieder-Alkyl, $CONH_2$, CONH-nieder-Alkyl oder CON(nieder-Alkyl)$_2$ substituiertes $N(CH_2)_{4-9}$ sind, |

wobei der Ausdruck "nieder" Gruppen mit bis zu 6 C-Atomen, "Aryl" gegebenenfalls durch Halogen, nieder-Alkyl, nieder-Alkoxy, OH, Phenyl, $CF_3$, $OCF_3$, Cyclopentyl, CN, COOH, $COOCH_3$, $COOC_2H_5$, $CONH_2$ oder Tetrazolyl substituiertes Phenyl oder 1- oder 2-Naphthyl, "Heteroaryl" gegebenenfalls durch nieder-Alkyl oder Halogen substituierte 5- bis 10-gliedrige aromatische Gruppen, die aus einem oder 2 Ringen bestehen und ein oder mehrere N-und/oder O-Atom(e) enthalten, und "Heterocyclyl" gegebenenfalls durch nieder-Alkyl substituierte 5- bis 10-gliedrige nicht aromatische, teilweise oder vollständig gesättigte Gruppen, die ein oder zwei Ringe und zumindest ein Heteroatom enthalten, bezeichnen, sowie Hydrate oder Solvate und physiologisch verträgliche Salze davon.

Die in den Patentanmeldung EP-A-0 468 231, EP-A-0-502 536, EP-A-0 381 033, EP-A-0 462 960, FR-A-2 506 306 und EP-A-0 097 630 beschriebenen Verbindungen unterscheiden sich strukturell von den vorliegenden Verbindungen der Formel I.

Ferner betrifft die Erfindung Verfahren zur Herstellung der obigen Verbindungen, pharmazeutische Präparate, die solche Verbindungen enthalten, sowie die Verwendung dieser Verbindungen bei der Herstellung von pharmazeutischen Präparaten.

Beispiele von physiologisch verwendbaren Salzen der Verbindungen der Formel I sind Salze mit physiologisch verträglichen Mineralsäuren, wie Salzsäure, Schwefelsäure, schweflige Säure oder Phosphorsäure; oder mit organischen Säuren, wie Methansulfonsäure, p-Toluolsulfonsäure, Essigsäure, Trifluoressigsäure, Zitronensäure, Fumarsäure, Maleinsäure, Weinsäure, Bernsteinsäure oder Salicylsäure. Die Verbindungen der Formel I mit sauren Gruppen, wie die Carboxygruppe, können auch Salze mit physiologisch verträglichen Basen bilden. Beispiele solcher Salze sind Alkalimetall-, Erdalkalimetall-, Ammonium- und Alkylammoniumsalze, wie das Na-, K-, Ca- oder Tetramethylammoniumsalz. Die Verbindungen der Formel I können auch in Form von Zwitterionen vorliegen.

Die Verbindungen der Formel I können solvatisiert, insbesondere hydratisiert sein. Die Hydratisierung kann im Zuge des Herstellungsverfahrens erfolgen oder allmählich als Folge hygroskopischer Eigenschaften einer zunächst wasserfreien Verbindung der Formel I auftreten.

Die Verbindungen der Formel I enthalten zumindest zwei asymmetrische C-Atome und können daher als Diastereomerengemisch oder als optisch reine Verbindung vorliegen.

Im Rahmen der Erfindung bezeichnet der Ausdruck "nieder" Gruppen, die 1 bis 6, vorzugsweise 1 bis 4 C-Atome enthalten. So bezeichnet nieder-Alkyl allein oder in Kombination geradkettige oder verzweigte, 1 bis 6, vorzugsweise 1 bis 4 C-Atome enthaltende Gruppen, wie Methyl, Aethyl, Propyl, Isopropyl, Butyl, Isobutyl, t-Butyl, 2-Butyl und Pentyl. Als Alkylgruppen A sind die nieder-Alkylgruppen bevorzugt. Ein Beispiel für nieder-Alkenyl ist Allyl.

Aryl bezeichnet Gruppen, wie Phenyl und 1- oder 2-Naphthyl, gegebenenfalls mit einem oder mehreren Substituenten, wie Halogen, z. B. Chlor, oder nieder-Alkyl oder Alkoxy, z. B. $CH_3$, t-Butyl, OH, $OCH_3$, Phenyl, $CF_3$, $OCF_3$, Cyclopentyl, CN, COOH, $COOCH_3$, $COOC_2H_5$, $CONH_2$ oder Tetrazolyl.

Heteroarylgruppen sind 5- bis 10-gliedrige aromatische Gruppen, die aus einem oder 2 Ringen bestehen und ein oder mehrere N- und/oder O-Atom(e) enthalten. Beispiele davon sind 2-, 3- oder 4-Pyridyl auch in Form ihrer N-Oxyde, Tetrazolyl, Oxadiazolyl, Pyrazinyl und Chinolyl. Sie können substituiert sein, z. B. durch nieder-Alkyl, wie $CH_3$, oder Halogen, wie Chlor.

Cycloalkylgruppen enthalten 3 bis 8 C-Atome. Beispiele davon sind Cyclopropyl, Cyclopentyl und Cyclohexyl.

Heterocyclyl bezeichnet 5- bis 10-gliedrige nicht aromatische, teilweise oder vollständig gesättigte Gruppen, wie Tetrahydrochinolyl, die ein oder zwei Ringe und zumindest ein Heteroatom, z.B. ein N-Atom, enthalten und gegebenenfalls durch einem oder mehreren Substituenten, wie nieder-Alkyl, z. B. Methyl, substituiert sind.

Beispiele von gegebenenfalls durch O unterbrochenes Tetra- bis Nonamethyleniminogruppen $N(CH_2)_{4-9}$ sind Hexahydroazepin und Morpholino.

Beispiele von Verbindungen der Formel I sind diejenigen, worin

| | |
|---|---|
| X | eine Gruppe $X^1$ ist, in der die Guanidinogruppe ungeschützt ist, |
| Y | H, |
| A | Aryl, Heteroaryl oder Heterocyclyl, |
| Q | die obige Bedeutung hat und |
| M | entweder eine Gruppe $M^1$, in der $R^3$ und $R^4$ die obige Bedeutung haben, wobei falls Q, $R^3$ und $R^5$ gleichzeitig H sind, $R^4$ nicht H oder Phenyl sein soll, und |
| $R^5$ | H, nieder-Alkyl oder gegebenenfalls über nieder-Alkylen gebundenes COOH, COO-nieder-Alkyl, nieder-Alkanoyl, OH, nieder-Alkanoyloxy, $NH_2$, NHCOO-nieder-Alkyl, $NHSO_3H$, ($NHSO_2$ oder $NHSO_3$)-nieder-Alkyl, NH-nieder-Alkanoyl, NHCOCOOH, NHCOCOO-nieder-Alkyl oder $NHPO_3(R^9,R^{10})$, oder falls Q nicht H ist, dann |
| M | auch eine Gruppe $M^2$ sein kann, in der $N(R^6)$ gegebenenfalls durch COOH oder COO-nieder-Alkyl substituiertes $N(CH_2)_{4-9}$ ist. |

Weitere Beispiele von Verbindungen der Formel I sind diejenigen, worin Y H, X eine Gruppe $X^1$ und M eine Gruppe $M^1$ ist und, falls zumindest eins von $R^1$ und $R^2$ (in $X^1$) nicht H ist und/oder falls Q nicht H ist und/oder falls A Alkyl oder Cycloalkyl ist, dann M auch eine Gruppe $M^2$ sein kann.

Weitere Beispiele von Verbindungen der Formel I sind diejenigen, worin Y H, X eine Gruppe $X^2$ und M eine Gruppe $M^1$ oder $M^2$ ist;

ferner diejenigen, worin Y H, X eine Gruppe $X^1$ und M eine Gruppe $M^5$ oder $M^6$ ist, mit der Bedingung, dass zumindest eins von $R^1$ und $R^2$ (in $X^1$) nicht H ist und/oder dass Q nicht H ist und/oder dass A Alkyl oder Cycloalkyl ist;

ferner diejenigen, worin Y H, X eine Gruppe $X^1$ und M eine Gruppe $M^3$ oder $M^7$ ist, mit der Bedingung, dass zumindest eins von $R^1$ und $R^2$ (in $X^1$) nicht H ist und/oder dass Q nicht H ist und/oder dass A Alkyl oder Cycloalkyl ist;

ferner diejenigen, worin Y und Q H sind, X eine Gruppe $X^1$ und M eine Gruppe $M^1$ ist und, falls zumindest eins von $R^1$ und $R^2$ (in $X^1$) nicht H ist und/oder falls A Alkyl oder Cycloalkyl ist, dann M auch eine Gruppe $M^2$ sein kann.

Bevorzugte Verbindungen der Formel I sind diejenigen, worin Y H, Q nieder-Alkyl(OH, COOH oder COO-niederalkyl), X eine Gruppe $X^1$ und M eine Gruppe $M^1$ oder $M^2$ ist;

ferner diejenigen, worin X eine Gruppe $X^1$, T $CH_2$, eins von $R^1$ und $R^2$ H und das andere H oder COO-(Methyl, Ethyl, Isobutyl oder t-Butyl) ist;

ferner diejenigen, worin X eine Gruppe $X^1$, T O, eins von $R^1$ und $R^2$ H und das andere H oder $COOC_2H_5$ ist;

ferner diejenigen, worin X eine Gruppe $X^2$ und $R^{11}$ und $R^{21}$ H sind.

Ferner ist A vorzugsweise Naphthyl, Methylchinolyl, Methyltetra-hydrochinolyl, Methyl, Pyridyl oder durch t-Butyl, $CF_3$, Phenyl, Cyclopentyl, Carboxy, Methoxycarbonyl, Ethoxycarbonyl, $OCF_3$, CN, $CONH_2$ oder Tetrazolyl substituiertes Phenyl,

und Q ist vorzugsweise H, $CH_3$, $CH_2COOH$, $CH_2CH_2OH$ oder $CH_2COOC_2H_5$.

Falls M eine Gruppe $M^1$ ist, ist $R^3$ vorzugsweise H, $CH_3$, Propyl, Isopropyl, Butyl, Pentyl, Allyl, Cyclopropyl, Cyclopentyl, Cyclohexyl, Cyclopropylmethyl, Cyclohexylmethyl, Pyridylmethyl oder gegebenenfalls durch Chlor oder

Methoxy substituiertes Benzyl und $R^4$ H, Isopropyl, 2-Butyl, Isobutyl, Phenyl, Benzyl oder Cyclohexyl.

In einer Gruppe $M^1$ ist ferner $R^5$ vorzugsweise eine Gruppe $(CH_2)_{0-2}$-$R^{50}$ und $R^{50}$ H, OH, $C(CH_3)_2OH$, $COCH_3$, $OCOCH_3$, COO(H, $CH_3$ oder $C_2H_5$), $NHCOOCH_3$, $NHCOCH_3$, Tetrazolyl, $CONH_2$, Methyloxadiazolyl, $OCH_3$, Benzyloxy, Morpholinocarbonyl, $CONHOCH_3$, CONHO-Benzyl, $CONHSO_2CH_3$, $CONHCH_2$-Pyridyl, CONH-Cyclopropyl, $CONHCH_2CH_2$-$C_6H_3(OH)_2$, $CONHCH_2CH_2OH$, NHCOCOOH, $NHCOCOOCH_3$, $NHCOCOOC_2H_5$, $NHSO_3H$, $NHSO_2CH_3$, NHCOO-Benzyl, $NHCOCH_2Cl$, $NHCOCH_2OC_6H_5$, $NHCOCOC_6H_5$, $NHCOCOCH_3$, NHCO-Pyridyl, NHCO-Pyridyl-N-oxid, NHCO-Pyrazinyl, $NHCOCH_2C_6H_3(OH)_2$, $NHPO(OC_6H_5)_2$, $NHPO(OC_2H_5)_2$, NH-(3,4-Dioxo-2-hydroxycyclobut-1-enyl) oder NH-(2-Allyloxy-3,4-dioxocyclobut-1-enyl).

Falls M eine Gruppe $M^2$ ist, ist $N(R^6)$ vorzugsweise Hexamethylenimino.

Beispiele von bevorzugten Verbindungen der Formel I sind folgende:

(S)-N4-[(S)-1-(Amino-imino-methyl)piperidin-3-ylmethyl]-N1-carboxymethyl-N1-cyclopentyl-2-(naphthalin-2-sulfonylamino)succinamid,

[(S)-3-[(S)-2-(Amino-imino-methyl)piperidin-3-ylmethylcarbamoyl]-2-(naphthalen-2-sulfonylamino)propionyl]-propyl-aminoessigsäure,

N-[N4-[[(S)-1-Amidino-3-piperidinyl]methyl]-N2-(2-naphthylsulfonyl)-L-asparaginyl]-N-(o-chlorbenzyl)glycin,

[2-[[(S)-3-[(S)-1-(Amino-imino-methyl)piperidin-3-ylmethylcarbamoyl]-2-(naphthalen-2-sulfonylamino)-propionyl]-butyl-amino]äthyl]oxamsäure,

(S)-N4-[(S)-1-(Amino-imino-methyl)piperidin-3-ylmethyl]-N1-butyl-2-(naphthalen-2-sulfonylamino)-N1-(2-sulfoamino-äthyl)-succinamid,

[(S)-3-[(S)-1-(Amino-imino-methyl)piperidin-3-ylmethylcarbamoyl]-2-(4 -t-butylphenylsulfonylamino)-propionyl-cyclopropyl-amino]-essigsäure

2-[(S)-2-[(S)-1-Amino-imino-methyl)-piperidin-3-ylmethylcarbamoyl]-1-[cyclopropyl-(2-carboxy-ethyl)-carbamoyl]-ethylsulfamoyl]-benzoesäure,

3-[[(S)-3-[(S)-1-(Amino-imino-methyl)-piperidin-3-ylcarbamoyl]-2-(4-cyano-phenylsulfonylamino)-propionyl]-cyclopropyl-amino]-propionsäure,

(S)-N(4)-[4-(Amino-imino-methyl)-morpholin-2-ylmethyl]-N(1)-cyclopropyl-N(1)-[2-(tetrazol-5-yl)-ethyl]-2-(naphthalin-2-ylsulfonyl)-succinamid,

[[(S)-3-[4-(Amino-imino-methyl)-morpholin-2-ylmethylcarbamoyl]-2-(naphthalin-2-yl-sulfonyl)-propionyl]-cyclopropyl-Amino]-essigsäure-ethylester,

[[(S)-3-[4-(Amino-imino-methyl)-morpholin-2-ylmethylcarbamoyl]-2-(naphthalen-2-yl-sulfonyl)-propionyl]-cyclopropyl-amino]-essigsäure,

2-[[(S)-3-[(S)-1-(Amino-imino-methyl)-piperidin-3-ylmethylcarbamoyl]-2-(naphthalen-2-ylsulfonylamino)-propionyl]-cyclopropyl-amino]-ethyl-sulfaminsäure,

(S)-N4-[(S)-1-(Amino-imino-methyl)-piperidin-3-ylmethyl]-N1-(2-chloroacetylamino-ethyl)-N1-cyclopropyl-2-(naphthalen-2-ylsulfonylamino)-succinamid,

(S)-N4-[(S)-1-(Amino-imino-methyl)-piperidin-3-ylmethyl]-N1-cyclopropyl-2-(naphthalen-2-ylsulfonylamino)-N1-(2-phenoxyacetylamino-ethyl)-succinamid,

(S)-N4-[(S)-1-(Amino-imino-methyl)-piperidin-3-ylmethyl]-N1-cyclopropyl-2-(naphthalen-2-ylsulfonylamino)-N1-[2-(2-oxo-2-phenyl-acetylamino)-ethyl]-succinamid,

(S)-N4-[(S)-1-(Amino-imino-methyl)-piperidin-3-ylmethyl]-N1-cyclopropyl-2-(naphthalen-2-ylsulfonylamino)-N1-[2-(2-oxo-propionylamino)-ethyl]-succinamid,

(S)-N4-[(S)-1-(Amino-imino-methyl)-piperidin-3-ylmethyl]-N1-cyclopropyl-2-(naphthalen-2-ylsulfonylamino)-N1-[2-(pyridin-3-ylcarbonylamino)-ethyl]-succinamid,

(S)-N4-[(S)-1-(Amino-imino-methyl)-piperidin-3-ylmethyl]-N1-cyclopropyl-2-naphthalen-2-ylsulfonylamino-N1-[2-(1-oxy-nicotinoylamino)-ethyl]-succinamid.

Besonders bevorzugt sind:

N-[N4-[[(S)-1-Amidino-3-piperidinyl]methyl]-N2-(2-naphthylsulfonyl)-L-asparaginyl]-N-cyclopropylglycin,

(S)-[[3-[(S)-1-(Amino-imino-methyl)piperidin-3-ylmethylcarbamoyl]-2-(naphthalin-2-sulfonylamino)propionyl]cyclopropylamino]propionsäure,

[(S)-3-[(S)-1-(Amino-imino-methyl)piperidin-3-ylmethylcarbamoyl]-2-(4-trifluormethyl-phenylsulfonylamino)-propionyl-cyclopropyl-amino]essigsäure,

3-[[(S)-3-[(S)-1-(Amino-imino-methyl)-piperidin-3-ylcarbamoyl]-2-(4-carbamoyl-phenylsulfonylamino)-propionyl]-cyclopropyl-amino]-propionsäure,

(S)-N4-[(S)-1-(Amino-imino-methyl)-piperidin-3-ylmethyl]-N1-cyclopropyl-2-(naphthalen-2-ylsulfonylamino)-N1-[2-(pyrazin-2-ylcarbonyl-amino)-ethyl]-succinamid,

(S)-N4-[(S)-1-(Amino-imino-methyl)-piperidin-3-ylmethyl]-N1-cyclopropyl-N1-[2-(3,4-dihydroxy-phenyl)-ethylcarbamoylmethyl]-2-(naphthalen-2-ylsulfonylamino)-succinamid.

Die Verbindungen der Formel I werden in an sich bekannter Weise dadurch hergestellt, dass man

a) eine Säure der Formel

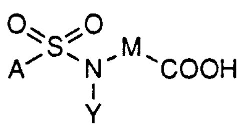

II

mit einem Amin der Formel

$$Q\text{-}NHCH_2\text{-}X \qquad III$$

oder einem Salz davon unter intermediärem Schutz von in den Gruppen A, Y und M (in II) und Q (in III) enthaltenen funktionellen Gruppen umsetzt oder

b) ein Amin der Formel

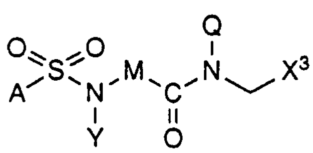

IV

worin $X^3$ eine Gruppe $X^{31}$ oder $X^{32}$ ist:

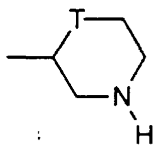 $(X^{31})$ 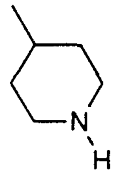 $(X^{32})$

mit einem Amidinierungsmittel umsetzt und

c) gewünschtenfalls eine in der Gruppe M oder Q einer Verbindung der Formel I enthaltene reaktionsfähige Gruppe funktionell abwandelt, und

d) gewünschtenfalls eine Verbindung der Formel I in ein physiologisch verträgliches Salz überführt oder ein Salz einer Verbindung der Formel I in die freie Säure oder Base überführt.

Zweckmässig wird die Säure II in einem Lösungsmittel, wie Dimethylformamid (DMF) oder Methylenchlorid, in Gegenwart einer Base, wie 4-Aethylmorpholin, Triäthylamin, Aethyldiisopropylamin oder 1,8-Diazabicyclo(5.4.0) undec-7-en (DBU), mit einem Salz einer Verbindung der Formel III, z. B. einem Trifluoracetat, Bisulfit, Nitrat, Hydrochlorid oder Hydrojodid, und mit Benzotriazol-1-yloxy-tris(dimethylamino)phosphoniumhexafluorophosphat (BOP) bei Raumtemperatur umgesetzt. In den Verbindungen II und III enthaltene, intermediär zu schützende funktionelle Gruppen wie COOH, $NH_2$ und OH können in Form von nieder-AlkylOCO-Gruppen, von BenzylOCO- oder Azidgruppen bzw. von Benzyloxygruppen geschützt werden. Die Aufspaltung einer geschützten Carboxygruppe, wie $COOCH_3$ oder $COOC_2H_5$, zu COOH kann mit Natronlauge in Aethanol erfolgen. Die Ueberführung der BenzylOCONH- oder $N_3$-Gruppe in die freie Aminogruppe kann man durch katalytische (Pd/C) Hydrierung in Aethanol durchführen.

In der Verfahrensvariante b) kann man die Verbindung IV in einem Lösungsmittel, wie DMF oder Methanol, in Gegenwart einer Base, wie Triäthylamin, mit Formamidinsulfonsäure oder 3,5-Dimethyl-1-pyrazolylformamidiniumnitrat, zweckmässig bei einer Temperatur bis zu 50°C umsetzen.

Als funktionelle Abwandlungen in der Variante c) kann man folgende nennen:

1. die Verseifung einer Estergruppe, wie Aethoxycarbonyl, z. B. in Aethanol oder Methanol, mittels einer Base, wie wässrigem NaOH, oder die Verseifung einer Estergruppe, wie Acetoxy, z. B. in THF, mittels einer-Base, wie wässrigem LiOH;

2. die Hydrierung der Doppelbindung in einer Alkylengruppe, z. B. in Aethanol und Wasser in Gegenwart von Pd/C;

3. die Hydrierung einer Arylgruppe zur entsprechenden Cycloalkylgruppen, z. b. in Aethanol in Gegenwart von Essigsäure und Pd/C;

4. die Spaltung eines Aethers, wie eines Benzyläthers, zum entsprechenden Alkohol, z.B. mittels einer Lösung von Bortribromid in Methylenchlorid;

5. die Verätherung eines Alkohols, z.B. mit einem nieder-Alkylhalogenid, wie Methyljodid, in Gegenwart einer Lösung von DBU in THF;

6. die Ueberführung einer Carbonsäure in das Carbonsäureamid durch Reaktion mit einem Amin, wie Morpholin, z.B. in DMF in Gegenwart von BOP und 4-Aethylmorpholin;

7.

a) die Umwandlung eines Amins in ein Quadratsäurederivat davon, z.B. durch Reaktion mit 3,4-Bis(2-propenyloxy)-3-cyclobuten-1,2-dion in THF bei 0° und gewünschtenfalls

b) die katalytische Abspaltung der 2-Propenylgruppe aus dem unter a) erhaltenen Quadratsäurederivat, z.B. mittels Palladium(II)-acetat in Acetonitril und Wasser in Gegenwart von Triethylphosphit und dann Natrium-2-ethylcapronat.

Die N-sulfonierten Aminosäuren der Formel II lassen sich durch Reaktion eines entsprechenden reaktionsfähigen Sulfonsäurederivats, wie des Sulfochlorids A-$SO_2Cl$, mit dem entsprechenden intermediär geschützten Aminosäurederivat HN(Y)-M-COO-t-Butyl, z.B. wie in der EP-A-468231 beschrieben, herstellen. Die Spaltung des t-Butylesters zur erwünschten Säure II kann man mit Trifluoressigsäure in $CH_2Cl_2$ oder mit Salzsäure in Ethylacetat durchführen.

Ferner lassen sich die Aminosäuren II, worin M eine Gruppe $M^1$ ist nach folgendem Reaktionsschema (1), (2), (3) herstellen:

(1)

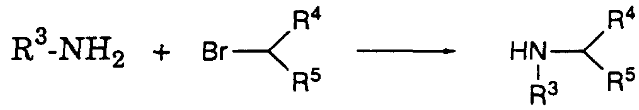

V

(2) V

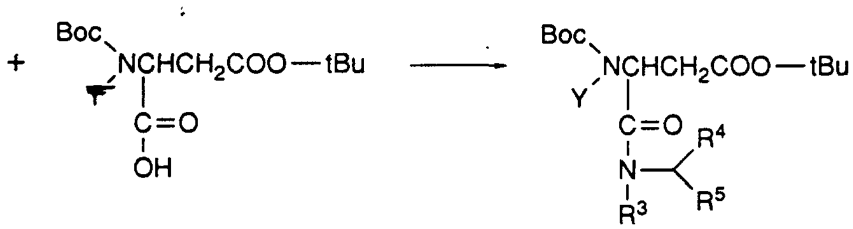

VI

(tBu = t-Butoxy, Boc = tBu-OCO)

(3)

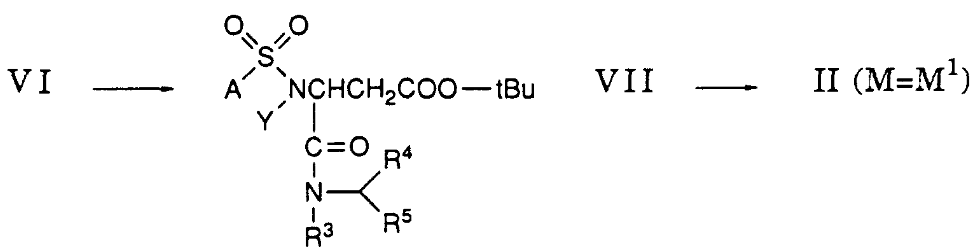

VI

VII

II ($M=M^1$)

Die Reaktion (1) kann man in einem Lösungsmittel, wie Toluol, bei erhöhter Temperatur durchführen. Die Reaktion (2) wird zweckmässig wie die weiter oben beschriebene Reaktion von II mit III durchgeführt. Die Reaktion VI → VII wird so bewerkstelligt, dass man zunächst die in VI enthaltene Boc-Gruppe vom N-Atom abspaltet, z. B. in Acetonitril oder Dioxan mit p-Toluolsulfonsäure, und die erhaltene Verbindung mit einem Sulfochlorid A-$SO_2Cl$ in Dioxan umsetzt. Die Hydrolyse der Ester VII zu den Säuren II kann mittels Trifluoressigsäure in Methylenchlorid erfolgen.

Die Herstellung eines Esters VII, in dem $R^5$ Tetrazolyl ist, verläuft via entsprechendem Ester in dem $R^5$ für Cyano steht. Die Umwandlung der Cyanogruppe in die Tetrazolylgruppe kann man in DMF mittels Ammoniumchlorid und Natriumazid durchführen.

Die Ausgangsguanidine III, in denen X eine Gruppe $X^1$ und $R^1$, $R^2$ und Q H sind, können wie in der EP-A-468231 beschrieben hergestellt werden, z.B. ausgehend von 3-Picolylamin bzw. von 2-Aminomethyl-4-benzylmorpholin je nach dem ob Guanidine III mit T = $CH_2$ bzw. T = O erwünscht sind. Zur Herstellung eines optisch aktiven Guanidins III kann man wie im Beispiel 36B beschrieben vorgehen. N-(3-Pyridylmethyl)benzamid wird katalytisch (Pd/C) in Aethanol und Salzsäure zum (RS)-N-Piperidin-3-ylmethyl-benzamid hydriert. Durch Salzbildung mit D-Mandelsäure in Methylenchlorid kann nach Zugabe von Diäthyläther das (R)-N-Piperidin-3-ylmethyl-benzamid-mandelat kristallisiert werden. Dieses kann dann in DMF mit Triethylamin und Formamidinsulfonsäure amidiniert werden. Durch Erhitzen einer Lösung des erhaltenen Mandelats in konzentrierter Salzsäure kann das (S)-Guanidin der Formel III, worin X die Gruppe $X^1$ und Q = $R^1$ = $R^2$ = H sind, erhalten werden.

Das Guanidin III mit X = $X^2$ und Q, $R^{11}$ und $R^{21}$ = H lässt sich in Analogie zu demjenigen mit X = $X^1$, T = $CH_2$ und Q, $R^1$ und $R^2$ = H herstellen, z.B. nach folgendem Reaktionsschema (4) und wie im nachfolgenden Beispiel 67a) b) beschrieben:

(4) 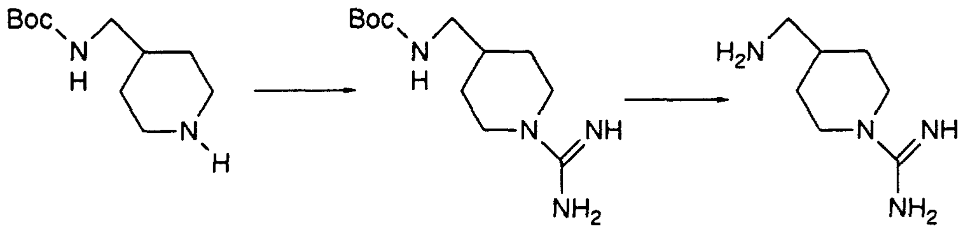 III'

Guanidine III, worin eins von $R^1$ und $R^2$ bzw. eins von $R^{11}$ und $R^{21} \neq H$ ist, lassen sich z.B. via Verbindungen des Typs VIII, IX, X im folgendem Reaktionsschema (5) und wie in Beispiel 48a)b)c) beschrieben, herstellen:

(5) 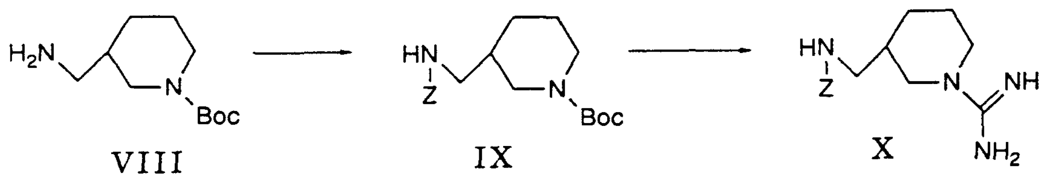

VIII IX X

So wird das Amin VIII in Hexan und Wasser mit Tetrabutylammoniumhydrogensulfat und 1N Natronlauge und dann mit Benzylchloroformiat umgesetzt. Aus der erhaltenen Verbindung IX wird die Boc-Gruppe mit einer Lösung von Salzsäure in Essigester abgespalten. Das Produkt wird in DMF mit Triäthylamin und Formamidinsulfonsäure in die Verbindung X übergeführt. Zum Schutz der Amidinogruppe in der Verbindung X wird letztere z.B. in Methylenchlorid mit Chlorameisensäureethylester umgesetzt. Durch hydrokatalytische Abspaltung der Z-Gruppe erhält man das Piperidinderivat III, worin X die Gruppe $X^1$, und eins von $R^1$ und $R^2$ Ethoxycarbonyl ist. In analoger Weise kann man das entsprechende Morpholinderivat III (T = O) herstellen.

Zum Schutz der in einem Guanidin III enthaltenen Amidinogruppe mit einer Boc-Gruppe kann man ein Guanidin des Typs X mit Di-t-butyl-dicarbonat (anstatt Chlorameisensäureäthylester) in Dioxan umsetzen.

Guanidine III mit $Q \neq H$ lassen sich z.B. nach folgenden Reaktionen (6) und (7) wie im nachstehenden Beispiel 9a) bis d) beschrieben herstellen;

(6) 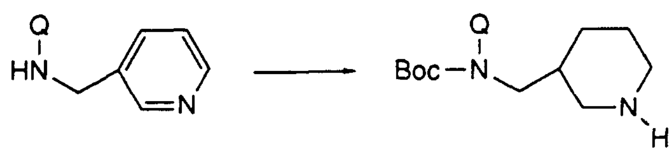 XI

(7) XI 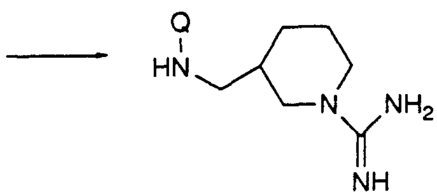 III"

Guanidine III mit $Q \neq H$ und T = O erhält man durch

a) Umsetzung des 2-Aminomethyl-4-benzylmorpholins (J. Med. Chem. 33, 1990, 1406 - 1413) mit Di-t-butyldicarbonat in Dioxan,

b) Umsetzung des erhaltenen mit Boc geschützten Amins mit NaH und einem Bromid Q-Br in DMF,

c) Abspaltung der Benzylgruppe aus dem erhaltenen Produkt durch Hydrierung in Aethanol in Gegenwart von Pd/C und

d) Amidinierung des erhaltenen Morpholinderivats wie weiter oben für die Verbindung IV beschrieben und Abspaltung der Boc-Gruppe.

Die Ausgangsamine IV stellt man z.B. nach folgender Reaktion (8) her, worin W eine Schutzgruppe, wie Boc oder Z, ist.

(8) 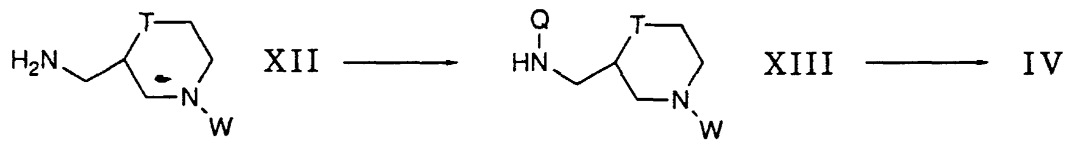

Zur Herstellung einer Verbindung IV, worin Q ≠ H ist, wird ein primäres Amin XII [herstellbar aus 3-Hydroxymethylpiperidin wie in der EP-A-468231 und im nachstehenden Beispiel 12 a) bis g) beschrieben] wird in einem Lösungsmittel, wie Methylenchlorid, mit einer Base, wie der Hünig-Base und einem Bromid Q-Br zum sekundären Amin XIII umgesetzt. Eine Säure II wird dann mit diesem Amin XIII (wie bei der obigen Kopplung II + III beschrieben) gekoppelt. Die Schutzgruppe Boc wird dann mittels Trifluoressigsäure in Methylenchlorid, p-Toluolsulfonsäure in Acetonitril oder einer Lösung von Chlorwasserstoff in Aethylacetat abgespalten. Die Abspaltung der Schutzgruppe Z wird durch Hydrierung in Aethanol in Gegenwart von Pd/C bewerkstelligt. Zur Herstellung einer Verbindung IV, worin Q = H ist, wird die Säure II mit dem Amin XII (anstatt XIII) gekoppelt.

Die Herstellung eines Ausgangsamins IV, in dem M eine Gruppe $M^1$ und $R^5$ NHCOCOO-nieder Alkyl ist, verläuft via eine Verbindung der Formel IV, in der $R^5$ für eine Azidogruppe steht. Die Umwandlung von Azido in NHCOCOO-nieder Alkyl kann durch katalytische Hydrierung (Pd/C in Methanol) gefolgt durch Umwandlung der entstandenen Aminogruppe in NHCOCOO-nieder Alkyl durch Reaktion mit einem Oxalsäure-mono-niederalkylesterchlorid in Gegenwart von Pyridin in Methylenchlorid erfolgen. Wird an Stelle von einem Oxalsäure-mono-niederalkylesterchlorid Pyrazincarbonsäure in Gegenwart von Hünig's Base in Methylenchlorid verwendet, so erhält man eine Verbindung IV in der M = $M^1$ und $R^5$ = NHCO-Pyrazinyl ist.

Zudem enthalten manche der nachfolgenden Beispiele detaillierte Angaben betreffend die Herstellung bestimmter Verbindungen der Formeln II, III und IV. Die Verbindungen der Formel III, worin X eine Gruppe $X^1$ und zumindest eins von $R^1$, $R^2$ und Q nicht H ist oder worin X eine Gruppe $X^2$ ist, sowie die Verbindungen der Formel IV, worin M eine Gruppe $M^1$ ist oder, falls $X^3$ eine Gruppe $X^{32}$ ist, oder falls X eine Gruppe $X^{31}$ ist und gleichzeitig Q nicht H ist und/oder falls A Alkyl oder Cycloalkyl ist, dann M auch eine der Gruppen $M^2$ bis $M^8$ sein kann, sind neu und als solche ebenfalls Gegenstand der vorliegenden Erfindung.

Die Verbindungen der Formel I, ihre Solvate und ihre Salze hemmen sowohl die durch Thrombin induzierte Plättchenaggregation als auch die durch Thrombin induzierte Gerinnung von Fibrinogen im Blutplasma. Die besagten Verbindungen beeinflussen sowohl die Plättchen induzierte als auch die plasmatische Blutgerinnung. Sie verhindern damit insbesondere die Entstehung von Gerinnungsthromben aber auch von plättchenreichen Thromben und können bei der Bekämpfung bzw. Verhütung von Krankheiten, wie Thrombose, Apoplexie, Herzinfarkt, Entzündung und Arteriosklerose verwendet werden. Ferne haben diese Verbindungen einen Effekt auf Tumorzellen und verhindern die Bildung von Metastasen. Somit können sie auch als Antitumor-Mittel eingesetzt werden.

Eine unterschiedliche Hemmung von Thrombin und anderen Serin-Proteasen durch die obigen Verbindungen ist erstrebenswert, um Verbindungen mit einer möglichst hohen Spezifität zu erhalten und damit mögliche Nebenwirkungen zu vermeiden. Nebst anderen getesteten Serin-Proteasen wurde das Verhältnis der Hemmung von Trypsin zur Hemmung von Thrombin als generelles Mass der Spezifität einer Verbindung genommen (q in der nachstehenden Tabelle), weil Trypsin als unspezifischste Serin-Protease durch verschiedenste Hemmer leicht gehemmt werden kann. Um die Hemmung von Thrombin und Trypsin, trotz Benutzung unterschiedlicher Substrate, direkt vergleichen zu können, wurde als Mass der Hemmung die von Substrat- und Enzymkonzentration unabhängige Hemmkonstante $K_i$ ermittelt.

Zum Nachweis der Hemmung der katalytischen Aktivität der obigen Proteasen können spezifische chromogene Peptidsubstrate benutzt werden. Die Hemmung der amidolytischen Aktivität von Thrombin und Trypsin durch die obigen Verbindungen wurde wie nachstehend beschrieben nachgewiesen.

Die Messungen wurden auf Mikrotiterplatten bei Raumtemperatur durchgeführt. Dafür wurden pro Vertiefung der Platte 150 µl Puffer (50 mM Tris, 100 mM NaCl, 0,1 % Polyaethylenglykol; pH 7,8) mit 50 µl der in DMSO gelösten und im Puffer verdünnten Hemmsubstanz vermischt und 25 µl humanes Thrombin (0,5 nM Endkonz.) zugegeben. Nach 10 Minuten Inkubation wurde die Reaktion durch Zugabe von chromogenem Substrat S-2238 (H-D-Phe-Pip-Arg-Paranitroanilin von Kabivitrum; 10 oder 50 µM Endkonz.) gestartet und die Hydrolyse des Substrates spektrophotometrisch auf einem kinetischen Mikrotiter-Plattenleser während 5 Minuten verfolgt. Nach graphischer Dartstellung der

Hemmkurven wurden die $K_i$-Werte nach der in Biochem. J. 55, 1955, 170-171 beschriebenen Methode bestimmt. Die Hemmung von Trypsin erfolgte analog, jedoch unter Verwendung des Substrates S-2251 (H-D-Val-Leu-Lys-Paranitroanilin) in 200 und 750 µM Endkonzentration.

Die Resultate sind aus folgender Tabelle ersichtlich:

| Produkt von Beispiel | $K_i$ (nM) Thrombin | $K_i$ (nM) Trypsin | q |
|---|---|---|---|
| 2k | 0,40 | 7700 | 19250 |
| 4a | 0,27 | 1900 | 7143 |
| 4i | 0,82 | 5000 | 6098 |
| 4k | 0,30 | 8100 | 27000 |
| 41 | 0,56 | 5000 | 8929 |
| 5 | 0,22 | 4300 | 19545 |
| 15a | 0.85 | 6100 | 7176 |
| 15b | 0,99 | 2100 | 2121 |
| 16 | 0,81 | 2100 | 2593 |
| 26b | 0,25 | 130 | 524 |
| 29 | 0,44 | 1800 | 4091 |
| 30 | 0,75 | 2400 | 3200 |
| 31c | 2,20 | 7700 | 3500 |

Die Verbindungen der Formel I haben eine geringe Toxizität. So haben die Produkte der in der Tabelle aufgezählten Beispiele bei intravenöser Verabreichung eine LD50 von 125 - 500 mg/kg an der Maus.

Wie eingangs erwähnt, sind Arzneimittel enthaltend eine Verbindung der Formel I, ein Solvat oder Salz davon, ebenfalls Gegenstand der vorliegenden Erfindung, weiterhin auch ein Verfahren zur Herstellung derartiger Arzneimittel, welches dadurch gekennzeichnet ist, dass man eine oder mehrere andere therapeutisch wertvolle Stoffe in galenische Darreichungsform bringt. Die Dragées, Hart- und Weichgelatinekapseln, Lösungen, Emulsionen oder Supensionen, oder rektal, z. B. in Form von Suppositorien, oder als Spray verabreicht werden. Die Verabreichung kann aber auch parenteral, z. B. in Form von Injektionslösungen, erfolgen.

Zur Herstellung von Tabletten, Lacktabletten, Dragées und Hartgelatinekapseln kann der Wirkstoff mit pharmazeutisch inerten, anorganischen oder organischen Excipientien vermischt werden. Als solche Excipientien kann man für Tabletten, Lacktabletten, Dragées und Hartgelatinekapseln z. B. Lactose, Maisstärke oder Derivate davon, Talk, Stearinsäure oder deren Salze, verwenden. Für Weichgelatinekapseln eignen sich als Excipientien z. B. vegetabile Oele, Wachse, Fette, halbfeste und flüssige Polyole; je nach Beschaffenheit des Wirkstoffs sind jedoch bei Weichgelatinekapseln überhaupt keine Excipientien erforderlich. Zur Herstellung der Lösungen und Sirupe eignen sich als Excipientien z. B. Wasser, Polyole, Saccharose, Invertzucker und Glucose, für Injektionslösungen eignen sich z. B. Wasser, Alkohole, Polyole, Glycerin und vegetabile Oele, und für Suppositorien eignen sich natürliche oder gehärtete Oele, Wachse, Fette, halbflüssige oder flüssige Polyole. Die pharmazeutischen Präparate können daneben noch Konservierungsmittel, Lösungsvermittler, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten.

Für die Bekämpfung bzw. Verhütung der weiter oben genannten Krankheiten kann die Dosierung des Wirkstoffes innerhalb weiter Grenzen variieren und ist natürlich in jedem Einzelfall den individuellen Gegebenheiten anzupassen. Im allgemeinen dürfte bei oraler oder parenteraler, z.B. intravenöser oder subkutaner Verabreichung eine Dosis von etwa 0,1 bis 20 mg/kg, vorzugsweise von etwa 0,5 bis 4 mg/kg, pro Tag für den Erwachsenen angemessen sein, wobei aber die soeben angegebene obere Grenze auch über- oder unterschritten werden kann, wenn sich dies als angezeigt erweisen sollte.

Beispiel 1

Eine Lösung von 0,85 g t-Butyl-(S)-N-cyclohexyl-N-[(äthoxycarbonyl)-methyl]-3-(2-naphthylsulfonamido)succinamat in 21 ml Methylenchlorid wird bei 0°C mit 2,4 ml Trifluoressigsäure versetzt und bei Raumtemperatur gerührt. Der nach Eindampfen der Lösung erhaltene Schaum wird in 13 ml DMF gelöst, dann mit 0,98 ml 4-Aethylmorpholin, 0,68 g Benzotriazol-1-yloxy-tris(dimethylamino)phosphoniumhexafluorophosphat und 0,43 g (S)-1-Amidino-3-(aminomethyl)piperidin-dihydrochlorid versetzt und bei Raumtemperatur gerührt. Das Reaktionsgemisch wird eingedampft und der Rückstand mit Essigester, dann mit Essigester-Aceton-Essigsäure-Wasser 16:2:1:1 an Kieselgel chromatographiert. Man isoliert 0,85 g N-[N4-[[(S)-1-Amidino-3-piperidinyl]methyl]-N2-(2-naphthylsulfonyl)-L-asparaginyl]-N-cyclohexylglycin-äthylester-diacetat,
Fab-MS: 629,3 $(M+H)^+$.

Herstellung des Ausgangsmaterials:

a) Zu einer Lösung von 2,89 g N-Boc-L-Asparaginsäure-β-t-butylester in 50 ml DMF gibt man 3,78 ml 4-Aethylmorpholin, 4,42 g Benzotriazol-1-yloxytris(dimethylamino)phosphonium-hexafluorophosphat (BOP) und eine Lösung von 2,25 g N-Cyclohexylglycin-äthylester (J. Heterocycl. Chem. 23, 1986, 929-933) in 8 ml DMF. Das Reaktionsgemisch wird bei Raumtemperatur gerührt, dann eingedampft und der Rückstand zwischen Essigester und Wasser verteilt. Die organische Phase wird getrocknet, eingedampft und der Rückstand mit Essigester-Hexan 1:1 an Kieselgel chromatographiert. Man isoliert so 4,5 g t-Butyl-(S)-3-(1-t-butoxyformamido)-N-cyclohexyl-N-[(äthoxycarbonyl)methyl]succinamat,
Fab-MS: 457 $(M+H)^+$.

b) Eine Lösung von 2,1 g des Produktes von a) in 22 ml Acetonitril wird mit 2,2 g p-Toluolsulfonsäure-monohydrat unter Rühren versetzt. Dann wird die Lösung eingedampft und getrocknet. 2,4 g des Rückstands werden in 45 ml Dioxan gelöst und mit einer Lösung von 1,56 g β-Naphthylsulfochlorid in 15 ml Dioxan versetzt. Dazu gibt man 1,9 g Natriumbicarbonat in 19 ml Wasser. Nach Rühren wird das Reaktionsgemisch auf Eis gegossen und mit Essigester extrahiert. Die organische Phase wird mit Wasser gewaschen, dann getrocknet und eingedampft. Der Rückstand wird mit Hexan-Essigester 4:1 an Kieselgel chromatographiert. Man erhält 0,85 g t-Butyl-(S)-N-cyclohexyl-N-[(äthoxycarbonyl)methyl]-3-(2-naphthylsulfonamido)succinamat,
Fab-MS: 547 $(M+H)^+$.

Beispiel 2

2.A) Analog Beispiel 1 stellt man folgende Verbindungen her:

a) N-[N4-[[(S)-1-Amidino-3-piperidinyl]methyl]-N2-(2-naphthylsulfonyl)-L-asparaginyl]-N-cyclopropylglycin-äthylester-acetat, MS (Ion-Spray): 587,3 $(M+H)^+$,

b) [[(S)-3-[(S)-1-(Amino-imino-methyl)piperidin-3-ylmethylcarbamoyl]-2-(naphthalin-2-sulfonylamino)propionyl]benzylamino]essigsäureäthylesteracetat, MS (Ion-Spray): 637,3 $(M+H)^+$,

c) N-[N4-[[(S)-1-Amidino-3-piperidinyl]methyl]-N2-(2-naphthylsulfonyl)-L-asparaginyl]-N-cyclohexylglycin-methylester-acetat, MS (Ion-Spray): 629,4 $(M+H)^+$,

d) N-[N4-[[(S)-1-Amidino-3-piperidinyl]methyl]-N2-(2-naphthylsulfonyl)-L-asparaginyl]-N-methylglycin-äthylester-hydrochlorid, MS (Ion-Spray): 561,5 $(M+H)^+$,

e) N-[N4-[[(S)-1-Amidino-3-piperidinyl]methyl]-N2-(2-naphthylsulfonyl)-L-asparaginyl]-N-isopropylglycin-äthylester-hydrochlorid, MS (Ion-Spray): 589,0 $(M+H)^+$,

f) (S)-[N-Allyl-[3-[(S)-1-amino-imino-methyl)piperidin-3-ylmethylcarbamoyl]-2-(naphthalin-2-sulfonylamino)propionyl]amino]essigsäureäthylester-hydrochlorid, MS (Ion-Spray): 587,0 $(M+H)^+$,

g) N-[(S)-3-[(S)-1-(Amino-imino-methyl)piperidin-3-ylmethylcarbamoyl]-2-(naphthalin-2-sulfonylamino)propionyl]butylamino]essigsäure-äthylesterhydrochlorid, MS (Ion-Spray): 603,2 $(M+H)^+$,

h) N-[N4-[[(S)-1-Amidino-3-piperidinyl]methyl]-N2-(2-naphthylsulfonyl)-L-asparaginyl]-N-(cyclopropylmethyl)glycin-äthylester-hydrochlorid, MS (Ion-Spray): 601,2 $(M+H)^+$,

i) (S)-N4-[(S)-1-(Amino-imino-methyl)piperidin-3-ylmethyl]-N1-äthoxycarbonylmethyl-N1-cyclopentyl-2-(naphthalin-2-sulfonylamino)succinamidhydrochlorid, MS (Ion-Spray): 615,2 $(M+H)^+$,

j) N-[N4-[[(S)-1-Amidino-3-piperidinyl]methyl]-N2-(2-naphthylsulfonyl)-L-asparaginyl]-L-leucin-äthylester-hydrochlorid, MS (Ion-Spray): 603,0 $(M+H)^+$,

k) N-[N4-[[(S)-1-Amidino-3-piperidinyl]methyl]-N2-(2-naphthylsulfonyl)-L-asparaginyl]-N-cyclopropyl-β-alanin-äthylester-hydrochlorid, MS (Ion-Spray): 601,3 $(M+H)^+$,

l) (S)-3-[Allyl-[3-[(S)-1-(amino-imino-methyl)piperidin-3-ylmethylcarbamoyl]-2-(naphthalin-2-sulfonylamino) propionyl]amino]propionsäureäthylester-hydrochlorid, MS (Ion-Spray): 601,2 $(M+H)^+$,

m) (S)-N4-[(S)-1-(Amino-imino-methyl)piperidin-3-ylmethyl]-N1-butyl-N1-(2-äthoxycarbonyläthyl)-2-(naphthalen-2-ylsulfonylamino)-succinamidhydrochlorid, MS (Ion-Spray): 617,5 $(M+H)^+$,

n) (S)-3-[(S)-1-(Amino-imino-methyl)piperidin-3-ylmethylcarbamoyl]-2-(naphthalen-2-ylsulfonylamino)-N-pentyl-propionylaminoessigsäureäthylester-hydrochlorid (1:1), MS (Ion-Spray): 617,1 $(M+H)^+$.

2.B) Herstellung der Ausgangsmaterialien:

2.B) a) Zu einer Lösung von 15,4 ml Cyclopropylamin in 100 ml Toluol gibt man 14,0 ml 3-Brompropionsäure-äthylester und erhitzt das Reaktionsgemisch 3 Stunden auf 90°. Anschliessend wird von ausgefallenem Salz filtriert und das Filtrat destilliert. Man erhält 9,5 g N-Cyclopropyl-β-alanin-äthylester, Fab-MS: 157 $(M+H)^+$.

2.B) b) Analog dem Verfahren von 2.B)a) stellt man unter Verwendung von Allylamin bzw. Butylamin anstelle von Cyclopropylamin folgende Verbindungen her:

1) N-Allyl-β-alanin-äthylester, Fab-MS: 157 $(M^+)$,

2) 3-Butylaminopropionsäure-äthylester, Fab-MS: 173 $(M^+)$.

2.B)c) Analog Beispiel Ia) jedoch unter Verwendung von N-substituiertem Glycinester an Stelle von N-Cyclohexylglycin-äthylester erhält man folgende Triester:

2.B)c)1) t-Butyl-(S)-2-(2-t-butoxyformamido)-N-cyclopropyl-N-[(äthoxycarbonyl)methyl]succinamat, Fab-MS: 415 $(M+H)^+$,

2.B)c)2) (S)-N-Benzyl-3-t-butoxycarbonylamino-N-äthoxycarbonylmethylsuccinamsäure-t-butylester, MS (Ion-Spray): 465,2 $(M+H)^+$,

2.B)c)3) (S)-3-t-Butoxycarbonylamino-N-cyclohexylmethyl-N-methoxycarbonylmethyl-succinamsäure-t-butylester, MS (Ion-Spray): 457,3 $(M+H)^+$.

2.B)d) Analog Beispiel 1b) erhält man folgende Daten:

2.B)d)1) (S)-N-Cyclopropyl-N-äthoxycarbonylmethyl-3-(naphthalen-2-sulfonylamino)succinamsäure-t-butylester, Fab-MS: 505 $(M+H)^+$,

2.B)d)2) (S)-N-Benzyl-N-äthoxycarbonylmethyl-3-(naphthalen-2-ylsulfonylamino)-succinamsäure-t-butylester, MS (Ion Spray): 555,2 $(M+H)^+$,

2.B)d)3) (S)-N-Cyclohexylmethyl-N-methoxycarbonylmethyl-3-naphthalen-2-ylsulfonylamino-succinamsäure-t-butylester, MS (Ion Spray): 547,2 $(M+H)^+$.

2.B)e) Zu einer Lösung von 5,78 g N-Boc-L-Asparaginsäure-β-t-butylester in 100 ml Methylenchlorid gibt man nacheinander 10,6 ml 4-Aethylmorpholin, 4,6 g N-(Dimethylaminopropyl)-N'-äthylcarbodiimid-hydrochlorid, 244 mg 4-Dimethylaminopyridin und 3,1 g Sarcosinäthylester-hydrochlorid. Nach Rühren wird das Reaktionsgemisch auf eiskalte 5%ige Kaliumhydrogensulfat-10%ige Kaliumsulfat-Lösung gegossen und mit Essigester extrahiert. Die organische Phase wird mit Wasser gewaschen, dann getrocknet, eingedampft und der Rückstand mit Hexan-Essigester (3:1) an Kieselgel chromatographiert. Man erhält 6,8 g (S)-3-t-Butoxycarbonylamino-N-äthoxycarbonylmethyl-N-methylsuccinamsäure-t-butylester, MS (Ion-Spray): 389,4 $(M+H)^+$.

2.B)f) Analog 2.B)e) jedoch unter Verwendung von N-substituierten Glycinestern an-Stelle von Sarcosinäthylester erhält man folgende Triester:

2.B)f)1) (S)-3-t-Butoxycarbonylamino-N-äthoxycarbonylmethyl-N-isopropyl-succinamsäure-t-butylester, MS (Ion-Spray): 417,1 $(M+H)^+$,

2.B)f)2) t-Butyl-(S)-N-allyl-N-[(äthoxycarbonyl)methyl]-3-(1-t-butoxyformamido)succinamat, MS (Ion-Spray): 415,2 $(M+H)^+$,

2.B)f)3) N-[N,3-Bis-t-butoxycarbonyl)-L-alanyl]-N-butylglycin-äthylester, MS (Ion-Spray): 431,2 $(M+H)^+$,

2.B)f)4) N-[N,3-Bis(t-butoxycarbonyl)-L-alanyl]-N-(cyclopropylmethyl)glycin-äthylester, MS (Ion-Spray): 428,2 $M^+$,

2.B)f)5) (S)-3-t-Butoxycarbonylamino-N-cyclopentyl-N-äthoxycarbonylmethyl-succinamsäure-t-butylester, MS (Ion-Spray): 443,3 $(M+H)^+$,

2.B)f)6) (S)-3-t-Butoxycarbonylamino-N4-cyclobutyl-N4-äthoxycarbonylmethyl-succinamsäure-t-butylester, MS (Ion-Spray): 429,2 $(M+H)^+$,

2.B)f)7) (S)-3-t-Butoxycarbonylamino-N-t-butyl-N-äthoxycarbonylmethylsuccinamsäure-t-butylester, MS (Ion-Spray): 431,2 $(M+H)^+$,

2.B)f)8) (S)-3-t-Butoxycarbonylamino-N-äthoxycarbonylmethyl-N-pentylsuccinamsäure-t-butylester, MS (Ion-Spray): 445,3 $(M+H)^+$.

2.B)g) Analog 2.B)e) jedoch unter Verwendung von L-Leucinäthylester an Stelle von Sarcosinäthylester erhält man den N-[N,3-Bis(t-butoxycarbonyl)-L-alanyl]-L-leucin-äthylester, Fab-MS: 431,2 $(M+H)^+$.

2.B)h) Analog 2.B)e) jedoch unter Verwendung der nach Beispiel 2.B)a) und b) hergestellten Ester erhält man folgende Triester:

2.B)h)1) 1) (S)-3-t-Butoxycarbonylamino-N-cyclopropyl-N-(2-äthoxycarbonyl-äthyl)-succinamsäure-t-butylester, MS: 429 $(M+H)^+$,

2.B)h)2) (S)-3-t-Butoxycarbonylamino-N-allyl-N-(2-äthoxycarbonyläthyl)succinamsäure-t-butylester, MS: 429 $(M+H)^+$,

2.B)h)3) (S)-3-t-Butoxycarbonylamino-N-butyl-N-(2-äthoxycarbonyläthyl)-succinamsäure-t-butylester, MS (Ion-Spray): 445,6 $(M+H)^+$.

2.B)i) Eine Lösung von 6,7 g (S)-3-t-Butoxycarbonylamino-N-äthoxycarbonylmethyl-N-methylsuccinamsäure-t-butylester in 80 ml Dioxan wird mit 8,2 g p-Toluolsolfonsäure-monohydrat versetzt. Nach Rühren werden 43,1 ml 1N Natronlauge, 4,34 g Natriumbicarbonat und eine Lösung von 7,8 g 2-Naphthylsulfochlorid in 37 ml Dioxan zugegeben. Nach Rühren wird das Reaktionsgemisch auf eiskalte 5%ige Kaliumhydrogensulfat-10%ige Kaliumsulfat-Lösung gegossen und mit Essigester extrahiert. Die organische Phase wird mit verdünnter Kochsalzlösung gewaschen, dann getrocknet und eingedampft. Nach Chromatographie an Kieselgel mit Hexan-Essigester (3:1) isoliert man 2,0 g t-Butyl-(S)-N-[(äthoxycarbonyl)-methyl]-N-methyl-3-(2-naphthylsulfonamido)succinamat, MS (Ion-Spray): 479,9 $(M+H)^+$.

2.B)j) Analog 2.B)i) jedoch unter Verwendung der Triester der Beispiele 2.B)f), g) und h) an Stelle der Triester des Beispiels 2.B)e) erhält man folgende Diester:

2.B)j)1) N-[3-(t-Butoxycarbonyl)-N-(2-naphthylsulfonyl)-L-alanyl]-N-isopropylglycin-äthylester, Fab-MS: 433 (M-t-Butoxy),

2.B)j)2) N-Allyl-N-[3-(t-butoxycarbonyl)-N2-(2-naphthylsulfonyl)-L-alanyl]glycin-äthylester, MS (Ion-Spray): 505,0 $(M+H)^+$,

2.B)j)3) (S)-N-Butyl-N-äthoxycarbonylmethyl-3-(naphthalene-2-sulfonylamino)-succinamsäure-t-butylester, MS (Ion-Spray): 54,3 $(M+H)^+$,

2.B)j)4) N-(Cyclopropylmethyl)-N-[4-t-butoxycarbonyl)-N-(2-naphthylsulfonyl)-L-alanyl]glycin-äthylester, Fab-MS: 445 (M-t-Butoxy),

2.B)j)5) (S)-N-Cyclopentyl-N-äthoxycarbonylmethyl-3-(naphthalen-2-sulfonylamino)-succinamsäure-t-butylester, MS (Ion-Spray): 533,0 $(M+H)^+$,

2.B)j)6) (S)-N-Cyclobutyl-N-äthoxycarbonylmethyl-3-(naphthalen-2-sulfonylamino)-succinamsäure-t-butylester, MS (Ion-Spray): 519,1 $(M+H)^+$,

2.B)j)7) (S)-N-t-Butyl-N-äthoxycarbonylmethyl-3-(naphthalen-2-sulfonylamino)-succinamsäure-t-butylester, MS (Ion-Spray): 521,1 $(M+H)^+$,

2.B)j)8) (S)-2-[(S)-3-t-Butoxycarbonyl-2-(naphthalen-2-sulfonylamino)-propionylamino]-4-methylpentansäureäthylester, MS (Ion-Spray): 521,0 $(M+H)^+$,

2.B)j)9) N-[3-(t-Butoxycarbonyl)-N-(2-naphthylsulfonyl)-L-alanyl]-N-cyclopropyl-β-alanin-äthylester, MS (Ion-Spray): 517,1 (M-H)-,

2.B)j)10) N-Allyl-N-[O-t-butyl-N-(naphthalen-2-yl-sulfonyl)-L-aspartyl]-β-alanin-äthylester, MS (Ion-Spray): 519,4 $(M+H)^+$,

2.B)j)11) (S)-N-Butyl-N-(2-äthoxycarbonyläthyl)-3-(naphthalen-2-ylsul-fonylamino)-succinamsäure-t-butylester, Fab-MS: 479 (M-Isobutylester),

2.B)j)12) (S)-N-Aethoxycarbonylmethyl-3-(naphthalen-2-ylsulfonylamino)-N-pentylsuccinamsäure-t-butylester, Fab-MS: 479 (M-Isobutylester).

Beispiel 3

Eine Lösung von 0,85 g N-[N4-[[(S)-2-Amidino-3-piperidinyl]methyl]-N2-(2- naphthylsulfonyl)-L-asparaginyl]-N-cyclohexylglycin-äthylester-diacetat (Beispiel 1) in 6 ml Aethanol wird mit 6,0 ml IN Natronlauge versetzt. Nach Rühren werden 6,0 ml 1N Salzsäure zugegeben, die Lösung wird eingedampft und der Rückstand mit Acetonitril-Wasser an einer RP-18 Säule chromatographiert. Man erhält 0,25 g N-[N4-[[(S)-1-Amidino-3-piperidinyl]methyl]-N2-(2-naphthylsulfonyl)-L-asparaginyl]-N-cyclohexylglycin, MS (Ion-Spray): 601,3 $(M+H)^+$.

Beispiel 4

Analog Beispiel 3, jedoch ausgehend von den Estern von Beispiel 2.A) erhält man folgende Säuren:

a) N-[N4-[[(S)-1-Amidino-3-piperidinyl]methyl]-N2-(2-naphthylsulfonyl)-L-asparaginyl]-N-cyclopropylglycin, MS (Ion-Spray): 559,0 $(M+H)^+$,

b) [[(S)-3-[(S)-1-(Amino-imino-methyl)piperidin-3-ylmethylcarbamoyl]-2-(naphthalin-2-sulfonylamino)-propionyl]-benzylamino]-essigsäure, MS (Ion-Spray): 609,1 $(M+H)^+$,

c) N-[N4-[[(S)-1-Amidino-3-piperidinyl]methyl]-N2-(2-naphthylsulfonyl)-L-asparaginyl]-N-cyclohexylglycin, MS (Ion-Spray): 615,4 $(M+H)^+$,

d) N-[N4-[[(S)-1-Amidino-3-piperidinyl]methyl]-N2-(2-naphthylsulfonyl)-L-asparaginyl]-N-methylglycin, MS (Ion-Spray): 532,9 $(M+H)^+$,

e) N-[N4-[[(S)-1-Amidino-3-piperidinyl]methyl]-N2-(2-naphthylsulfonyl)-L-asparaginyl]-N-isopropylglycin, MS (Ion-Spray): 561,2 $(M+H)^+$,

f) [[(S)-3-[(S)-1-(Amino-imino-methyl)piperidin-3-ylmethylcarbamoyl]-2-(naphthalin-2-sulfonylamino)propionyl]allylamino]essigsäure, MS (Ion-Spray): 557,2 (M-H)-,

g) [[(S)-3-[(S)-1-(Amino-imino-methyl)piperidin-3-ylmethylcarbamoyl]-2-(naphthalin-2-sulfonylamino)propionyl] butylamino]essigsäure, MS (Ion-Spray): 575,3 $(M+H)^+$,

h) N-[N4-[[(S)-1-Amidino-3-piperidinyl]methyl]-N2-(2-naphthylsulfonyl)-L-asparaginyl]-N-(cyclopropylmethyl)glycin, MS (Ion-Spray): 573,3 $(M+H)^+$,

i) (S)-N4-[(S)-1-(Amino-imino-methyl)piperidin-3-ylmethyl]-N1-carboxymethyl-N1-cyclopentyl-2-(naphthalin-2-sulfonylamino)succinamid, MS (Ion-Spray): 587,2 $(M+H)^+$,

j) N-[N4-[[(S)-1-Amidino-3-piperidinyl]methyl]-N2-(2-naphthylsulfonyl)-L-asparaginyl]-L-leucin, MS (Ion-Spray): 575,1 $(M+H)^+$,

k) (S)-[[3-[(S)-1-(Amino-imino-methyl)piperidin-3-ylmethylcarbamoyl]-2-(naphthalin-2-sulfonylamino)propionyl] cyclopropylamino]propionsäure, MS (Ion-Spray): 573,,2 $(M+H)^+$,

l) (S)-3-[Allyl-[3-[(S)-1-(amino-imino-methyl)piperidin-3-ylmethylcarbamoyl]-2-(naphthalin-2-sulfonylamino)propionyl]amino]propionsäure, MS (Ion-Spray): 573,3 $(M+H)^+$,

m) 3-[(S)-3-[(S)-[1-(Amino-imino-methyl)piperidin-3-ylmethylcarbamoyl]-N-butyl-2-(naphthalen-2-ylsulfonylamino)propionylamino]propionsäure, MS (Ion-Spray): 589,4 $(M+H)^+$,

n) [(S)-[3-[(S)-1-(Amino-imino-methyl)piperidin-3-ylmethylcarbamoyl]-2-(naphthalen-2-ylsulfonylamino)-propionyl]-pentyl-aminoessigsäure, MS (Ion-Spray): 589,5 $(M+H)^+$.

Beispiel 5

Eine Lösung von 50 mg [[(S)-3-[(S)-1-(Amino-imino-methyl)piperidin-3-ylmethylcarbamoyl]-2-(naphthalin-2-sulfonylamino)-propionyl]allylamino]-essigsäure (Beispiel 4.f) in 4 ml Aethanol und 1 ml Wasser wird mit 10 mg Pd/C versetzt und unter Normalbedingungen hydriert. Nach 4 Stunden wird vom Katalysator filtriert und das Filtrat eingedampft. Man erhält 50 mg [(S)-3-[(S)-2-(Amino-imino-methyl)piperidin-3-ylmethylcarbamoyl]-2-(naphthalen-2-sulfonylamino)propionyl]-propyl-aminoessigsäure, MS (Ion-Spray): 561,3 $(M+H)^+$.

Beispiel 6

Analog Beispiel 5 erhält man aus

(S)-3-[Allyl-[3-[(S)-1-(amino-imino-methyl)piperidin-3-ylmethyl-carbamoyl]-2-(naphthalin-2-sulfonylamino)propionyl]amino]propionsäure (Beispiel 4.1)

3-[(S)-3-[(S)-1-(Amino-imino-methyl)piperidin-3-ylmethylcarbamoyl]-2-(naphthalen-2-sulfonylamino)-propionyl]-propyl-amino]propionsäure, MS (Ion-Spray): 575,2 $(M+H)^+$.

Beispiel 7

A) Analog Beispiel 1 erhält man aus den Diester der Beispiele 2.B)i) und j) einerseits und an Stelle des (S)-1-Amidino-3-(aminomethyl)piperidindihydrochlorids: aus dem rac-2-Aminomethyl-4-morpholinecarboxamidintrifluoracetat andererseits folgende Ester:

a) 3-[N-[(S)-3-[(R,S)-4-(amino- imino-methyl)-morpholin-2-ylmethylcarbamoyl]-2-(naphthalen-2-sulfonylamino)-propionyl]-cyclopropylamino]-propionsäure-äthylester-trifluoroacetat (1:1), MS (Ion-Spray): 603,4 $(M+H)^+$,

b) 3-[N-Allyl-[(S)-3-(R,S)-4-(amino-imino- methyl)-morpholin-2-ylmethylcarbamoyl]-2-(naphthalen-2-sulfonylamino)-propionyl]-amino]-propionsäure-äthylester-trifluoroacetat (1:1), MS (Ion-Spray): 603,5 $(M+H)^+$.

B) Das Ausgangstrifluoroacetat wird wie folgt hergestellt:

a) Eine Lösung von 23,3 g rac-2-(Aminomethyl)-4-benzylmorpholin in 250 ml Dioxan wird mit 27,1 g Di-t-butyldicarbonat in 250 ml Dioxan versetzt. Nach Rühren wird das Lösungsmittel abgedampft und der Rückstand mit Methylenchlorid-Essigester 3:1 an Kieselgel chromatographiert. Das Produkt wird aus Methylenchlorid-Hexan umkristallisiert. Man erhält 25,6 g t-Butyl-rac-[(4-benzyl-2-morpholinyl)methyl]-carbamat.

b) Eine Lösung des Produkts von a) in 500 ml Essigester und 50 ml Essigsäure wird mit 2,6 g Pd/C versetzt und während 5 Stunden unter Normalbedingungen hydriert. Nach Filtration und Eindampfen wird der Rückstand in 230 ml DMF gelöst, mit 46 ml Triäthylamin und 10,8 g Formamidinsulfonsäure versetzt. Nach Rühren wird das Reaktionsgemisch eingedampft und der Rückstand zwischen Essigester und Wasser verteilt. nach Trocknen der organischen Phase und Eindampfen erhält man das t-Butyl-rac-[(4-amidino-2-morpholinyl)methyl]carbamat-hemisulfit.

c) 6,5 g des unter b) erhaltenen Materials werden in 50 ml Methylenchlorid suspendiert und bei 0° mit 20 ml TFA versetzt. Dann wird das Reaktionsgemisch eingedampft und mit Aethylenchlorid und Toluol azeotropiert. Man isoliert rac-2-(Aminomethyl)-4-morpholincarboxamidintrifluoracetat.

Beispiel 8

Analog Beispiel 3 erhält man aus den Estern von Beispiel 7 folgende Säuren:

a) 3-[(S)-3-[(R,S)-4-Amino-imino-methylmorpholin-2-ylmethylcarbamoyl]-2-(naphthalen-2-sulfonylamino)-N-cyclopropyl-propionylamino]-propionsäure, MS (Ion-Spray): 575,5 $(M+H)^+$,

b) 3-[N-Allyl-[(S)-3-[(R,S)-4-(amino-imino-methyl)morpholin-2-ylmethylcarbamoyl]-2-(naphthalen-2-sulfonylamino)-propionyl]-amino]-propionsäure, MS (Ion-Spray): 575,4 $(M+H)^+$.

Beispiel 9

Eine Lösung von 1,4 g N-[3-(t-Butoxycarbonyl)-N-(2-naphthylsulfonyl)-L-alanyl]-N-cyclopropyl-β-alanin-äthylester (Beispiel 2B)j)9) in 23 ml Methylenchlorid wird bei 2° mit 4,1 ml Trifluoressigsäure versetzt. Nach 5 Stunden Rühren bei Raumtemperatur wird die Lösung eingedampft, der Rückstand in 23 ml DMF gelöst, mit 1,7 ml 4-Aethylmorpholin,1,2 g BOP und 0,8 g rac-3-[[(2-Hydroxyäthyl)amino]methyl]-1-piperidincarboxamidin-dihydrochlorid versetzt. Nach Rühren wird die Reaktionslösung eingedampft und der Rückstand mit Wasser-Acetonitril an einer RP-18 Säule chromatographiert. Man erhält 0,7 g 3-[(S)-3-[(R,S)-N-(1-Amino-imino-methyl-piperidin-3-ylmethyl)-2-hydroxyäthylcarbamoyl]-2-(naphthalen-2-sulfonylamino)-N-cyclopropyl-propionylamino]-propionsäure-äthylester-hydrochlorid (1:1) MS (Ion-Spray): 645,5 $(M+H)^+$.

Herstellung des Ausgangspiperidincarboxamidins:

a) Zu einer Lösung von 20 g N-(2-Hydroxyäthyl)-3-picolylamin in 250 ml Dioxan gibt man eine Lösung von 31,6 g Di-t-butyl-dicarbonat in 100 ml Dioxan. Nach Rühren dampft man das Reaktionsgemisch ein und chromatographiert den Rückstand mit Essigester an Kieselgel. Man erhält 29,8 g t-Butyl-(2-hydroxyäthyl)(3-pyridylmethyl)carbamat, EI-MS: 253 $(M+H)^+$.

b) Das unter a) erhaltene Material wird in 150 ml Aethanol gelöst, mit 3 g Ruthenium auf Aluminiumoxid versetzt und während 24 Stunden bei 60° und unter 100 bar hydriert. Nach Filtration erhält man quantitativ t-Butylrac-(2-hydroxyäthyl)-(3-piperidinylmethyl)-carbamat, EI-MS: 259 $(M+H)^+$.

c) Eine Lösung des Produktes von b) in 500 ml DMF wird mit 51 ml Triäthylamin und 12,1 g Formamidinsulfonsäure versetzt. Nach Rühren wird ausgefallenes Material abfiltriert, in Aethanol-Wasser 1:1 gelöst, filtriert und das Filtrat eingedampft und mit Aethanol azeotropiert. Der Rückstand wird mit Aether aufgeschlämmt und abgenutscht. Man erhält 24,1 g t-Butyl-rac-[(1-amidino-3-piperidinyl)methyl](2-hydroxyäthyl)carbamat-hemisulfit, Fab-MS: 301 (M+H).

d) 10,0 g des unter c) erhaltenen Produktes werden in 90 ml Methylenchlorid und 10 ml Methanol gelöst, bei 0° mit 100 ml einer 4 molaren Salzsäure-Lösung in Essigester versetzt. Nach Rühren wird das Reaktionsgemisch eingedampft, man erhält quantitativ rac-3-[[(2-Hydroxyäthyl)-amino]methyl]-1-piperidincarboxamidin-dihydrochlorid, Fab-MS: 201 $(M+H)^+$.

Beispiel 10

Analog Beispiel 3 erhält man aus dem Ester von Beispiel 9 das
(S)-N4-[R,S)-1-Amino-imino-methyl-piperidin-3-ylmethyl]-N1-(2-carboxyäthyl)-N1-cyclopropyl-N4-(2-hydroxyäthyl)-2-(naphthalen-2-ylsulfonylamino)-succinamid, MS (Ion-Spray): 617,5 $(M+H)^+$.

Beispiel 11

Eine Lösung von 0,45 g t-Butyl-(S)-hexahydro-β-(2-naphthylsulfonamido)-γ-oxo-1H-azepin-1-butyrat in 3 ml Methylenchlorid wird bei 0° mit 1,5 ml Trifluoressigsäure versetzt. Nach Rühren wird die Lösung eingedampft, mit Toluol azeotropiert und getrocknet. Der Rückstand wird in 8 ml DMF gelöst, mit 0,38 ml 4-Ethylmorpholin, 0,49 g BOP und 0,3 g rac-3-[[(2-Hydroxyäthyl)amino]methyl]-1-piperidincarboxamidin-dihydrochlorid (Bsp. 9d) versetzt. Nach Rühren wird das Reaktionsgemisch eingedampft und mit Essigester-Aceton-Essigsäure-Wasser (6:2:1:1) an Kieselgel chromatographiert. Die Produkt enthaltenden Fraktionen werden eingedampft und der Rückstand mit Methanol-Wasser (9:1) über Dowex (Acetatform) filtriert. Man erhält 0,4 g (S)-N-[[(RS)-1-Amidino-3-piperidinyl]methyl]-N-(2-hydroxyäthyl)hexahydro-β-2-naphthylsulfonamido-1H-1-azepinbutyramid-diacetat, MS (Ion-Spray): 587,2 $(M+H)^+$.

Herstellung des Ausgangsmaterials:

a) Zu einer Lösung von 2,89 g N-Boc-L-Asparaginsäure-β-t-butylester in 50 ml Methylenchlorid gibt man 1,37 ml Hexamethylenimin, 2,3 g N-(Dimethylaminopropyl)-N'-äthylcarbodiimid-hydrochlorid und 122 mg Dimethylaminopyridin. Nach Rühren giesst man das Reaktionsgemisch auf eiskalte 5%ige Kaliumhydrogensulfat-10%ige Kaliumsulfat-lösung und extrahiert mit Methylenchlorid. Die organische Phase wird mit Wasser gewaschen, dann getrocknet und eingedampft. Der Rückstand wird mit Hexan-Essigester an Kieselgel chromatographiert. Man erhält 2,9 g t-Butyl-(S)-β-(1-t-butoxyformamido)hexahydro-γ-oxo-1H-azepin-1-butyrat, Fab-MS: 371,2 $(M+H)^+$.

b) Eine Lösung von 1,02 g des Produktes von a) in 10 ml Dioxan wird mit 1,31 g p-Toluolsulfonsäure-monohydrat versetzt. Nach Rühren werden unter Eiskühlung nacheinander 6,9 ml IN Natronlauge, eine Lösung von 0,93 g 2-Naphthylsulfochlorid in 5 ml Dioxan und 0,7 g Natriumbicarbonat zugegeben. Nach Rühren wird das Reaktionsgemisch auf eiskalte 5%ige Kaliumhydrogensulfat-10%ige Kaliumsulfat-lösung gegossen und mit Essigester extrahiert. Die organische Phase wird mit Wasser gewaschen, dann getrocknet und eingedampft. Nach Chromatographie mit Hexan-Essigester (2:1) an Kieselgel isoliert man 0,55 g t-Butyl-(S)-hexahydro-β-(2-naphthylsulfonamido)-γ-oxo-lH-azepin-1-butyrat, MS (Ion-Spray): 483,1 $(M+H)^+$.

Beispiel 12

Zu einer Lösung von 1,9 g (S)-3-[N-[(S)-4-(Azepan-1-yl)-3-(naphthalin-2-sulfonylamino)-4-oxobutyryl]-N-äthoxycarbonylmethylaminomethyl]-piperidin-1-carbonsäure-t-butylester in 20 ml Acetonitril gibt man 1,3 g p-Toluolsulfonsäure-monohydrat. Nach Rühren wird die Lösung eingedampft, der Rückstand getrocknet, in 25 ml DMF gelöst und mit 1,9 ml Triäthylamin und 450 mg Formamidinsulfonsäure versetzt. Nach Rühren wird das Reaktionsgemisch eingedampft und der Rückstand mit Wasser-Acetonitril an einer RP-18-Säule chromatographiert. Man erhält 0,7 g [[(R)-1-(Amino-imino-methyl)-piperidin-3-ylmethyl]-[(S)-4-(azepan-1-yl)-3-(naphthyl-2-sulfonylamino)-4-oxo-butyryl] amino]essigsäureäthylesterhemisulfit, MS (Ion-Spray): 629,2 $(M+H)^+$.

Herstellung des Ausgangsesters:

a) Zu einer Lösung von 92,9 g rac-3-Hydroxymethylpiperidin in 1500 ml Dioxan wird eine Lösung von 211,2 g Di-t-butyl-dicarbonat in 500 ml Dioxan zugegeben, so dass die Temperatur 25°C nicht übersteigt. Das Reaktionsgemisch wird gerührt und dann eingedampft. Der Rückstand wird in 800 ml Hexan aufgeschlämmt und filtriert. Man erhält 120,7 g rac-N-t-Butyloxycarbonyl-3-hydroxymethylpiperidin, Smp. 78°C.

b) Eine Lösung von 100 g des Produkts von a) in 4000 ml Methylenchlorid wird mit 56,2 ml Pyridin versetzt und auf 0°C abgekühlt. Dazu tropft man 58,3 ml Buttersäurechlorid, so dass die Temperatur 10°C nicht übersteigt. Nach Rühren wird die Suspension abfiltriert, das Filtrat eingedampft und der Rückstand in Essigester aufgenommen. Die organische Phase wird mit wässriger 10% $CuSO_4$-Lösung gewaschen, getrocknet und eingedampft. Der Rückstand wird durch Kieselgel filtriert und mit Hexan-Essigester (8:2) eluiert. Man erhält 119,7 g rac-3-(Butyroxymethyl)-1-piperidincarbonsäure-t-butylester.

c) 116,6 g des Produkts von b) werden in 2 l 0,1M Natriumchloridlösung und 80 ml 0,1M Natriumphosphat-Puffer pH 7,0 emulgiert. Der pH wird mit 1,0N Natronlauge auf 7,0 gestellt und die Reaktion durch Zugabe von 1,00 g

aus Pseudomonas fluorescens gewonnener Lipoproteinlipase (Lipase P-30, Amano) in 10 ml 0,1M Natriumchloridlösung gestartet. Der pH wird unter Rühren durch Zugabe von 2,0N Natronlauge bei 7,0 gehalten. Nach 14 Stunden wird die Reaktion durch Zugabe von 500 ml Methylenchlorid beendet, das Reaktionsgemisch mit Methylenchlorid extrahiert und die organische Phase getrocknet und eingedampft. Chromatographie des Rückstands über Kieselgel mit Hexan-Essigester ergibt 36,6 g (S)-3-Hydroxymethyl-1-piperidincarbonsäure-t-butylester, Smp. 89-90°C, $[\alpha]_{365}^{25}$ = +53,5° (c=1,0, EtOH).

d) Die 65,7 g Ester-Fraktion von c) werden in 1,15 l 0,1M Natriumchloridlösung und 45 ml 0,1M Natriumphosphat-Puffer (pH 7,0) emulgiert und mit 0,50 g Lipase P-30 in 5 ml 0,1M Natriumchloridlösung versetzt. Der pH wird unter Rühren durch Zugabe von 2,0N Natronlauge bei 7,0 gehalten. Nach 40 Stunden wird die Reaktion durch Zugabe von 400 ml Methylenchlorid beendet, das Reaktionsgemisch mit Methylenchlorid extrahiert und die organische Phase getrocknet und eingedampft. Chromatographie des Rückstands über Kieselgel mit Hexan-Essigester ergibt 49,5 g (R)-3-(Butyryloxymethyl)-1-piperidincarbonsäure-t-butylester. Dieser wird in 250 ml Aethanol gelöst, mit 88 ml 2N Natronlauge versetzt, über Nacht gerührt und dann eingedampft. Der Rückstand wird in 200 ml Methylenchlorid aufgenommen und mit Wasser gewaschen, die wässrige Phase mit Methylenchlorid extrahiert und die organische Phase getrocknet und eingeengt. Chromatographie des Rückstands über Kieselgel mit Hexan-Essigester ergibt 33,7 g (R)-3-Hydroxymethyl-1-piperidincarbonsäure-t-butylester, $[\alpha]_{365}^{25}$ = -60,7° (c=1,0, EtOH).

e) Eine Lösung von 5,0 g des Produkts von d) in 100 ml Pyridin wird mit 5,4 g p-Chlorsulfonylchlorid versetzt. Das Reaktionsgemisch wird gerührt, dann eingedampft, in 200 ml Essigester aufgenommen und mit Wasser und wässriger 10% $CuSO_4$-Lösung gewaschen. Die organische Phase wird getrocknet und eingedampft. Der Rückstand wird über Kieselgel nitriert und mit Hexan-Essigester (8:2) eluiert. Man erhält 6,5 g (R)-3-(p-Chlorphenylsulfonyloxymethyl)-1-piperidincarbonsäure-t-butylester.

f) Eine Lösung des Produkts von e) in 50 ml DMF wird mit 3,25 g Natriumazid versetzt. Das Reaktionsgemisch wird 15 Stunden bei 50°C gerührt und eingedampft. Der Rückstand wird in Wasser und Aether aufgenommen und mit Wasser gewaschen. Die Aetherphase wird getrocknet und eingedampft. Man erhält 4,0 g (R)-3-Azidomethyl-1-piperidincarbonsäure-t-butylester.

g)1) Eine Lösung des Produkts von f) in 100 ml Aethanol wird in Gegenwart von 0,6 g Platinoxid unter 1 bar Wasserstoff hydriert. Dann wird das Reaktionsgemisch über Kieselgel filtriert und mit Methanol eluiert. Man erhält 3,4 g (S)-3-Aminomethyl-1-piperidincarbonsäure-t-butylester, $[\alpha]_{D}^{25}$ = -17,7° (c=0,6, EtOH).

g)2) Analog e), f) und g)1) erhält man aus (S)-3-Hydroxymethyl-1-piperidincarbonsäure-t-butylester den (R)-3-Aminomethyl-1-piperidincarbonsäure-t-butylester, $[\alpha]_{D}^{25}$ = +23,0° (c=0,4 EtOH).

h) Eine Lösung von 4,0 g des Produkts von g) in 300 ml Methylenchlorid wird unter Argon mit 9,6 ml Hünigs Base und 2,08 ml Bromessigsäureäthylester versetzt. Nach Rühren wird die Lösung eingedampft, der Rückstand in Essigester aufgeschlämmt, filtriert und das Filtrat gegen Wasser geschüttelt. Die organische Phase wird getrocknet, eingedampft und der Rückstand mit Hexan-Essigester (1:1) an Kieselgel chromatographiert. Man erhält 2,3 g N-[[(S)-1-t-Butoxycarbonyl)-3-piperidinyl]methyl]glycinäthylester, EI-MS: 243 (M-t-butyl).

i) Eine Lösung von 1,65 g t-Butyl-(S)-hexahydro-β-(2-naphthylsulfonamido)-γ-oxo-1H-azepin-1-butyrat (Beispiel 11b) in 50 ml Methylenchlorid wird bei 0°C mit 5,5 ml Trifluoressigsäure versetzt. Nach Rühren wird eingedampft. Der Rückstand wird in 31 ml DMF gelöst, mit 2,3 ml 4-Aethylmorpholin, 1,60 g BOP und einer Lösung von 1,3 g des Produktes von h) in 2 ml DMF versetzt. Nach Rühren wird das Reaktionsgemisch eingedampft, der Rückstand in Essigester aufgenommen und gegen Wasser geschüttelt. Nach Trocknen-der organischen Phase, Eindampfen und Chromatographie des Rückstandes mit Hexan-Essigester an Kieselgel erhält man 2,0 g (S)-3-[N-[(S)-4-(Azepan-1-yl)-3-(naphthalin-2-sulfonylamio)-4-oxobutyryl]-N-äthoxycarbonylmethylaminomethyl]-piperidin-1-carbonsäure-t-butylester, MS (Ion-Spray): 687,3 $(M+H)^+$.

Beispiel 13

Eine Lösung von 1,0 g des Esterproduktes von Beispiel 12 in 10 ml Methanol wird mit 9,0 ml 1N Natronlauge versetzt. Nach Rühren gibt man 9,0 ml 1N Salzsäure zu, dampft die Lösung ein, chromatographiert den Rückstand mit Wasser-Acetonitril an einer RP-18-Säule und erhält so 0,5 g [[(R)-1-(Amino-imino-methyl)piperidin-3-ylmethyl]-[(S)-4-(azepan-1-yl)-3-(naphthyl-2-sulfonylamino)-4-oxo-butyryl]amino]essigsäure, MS (Ion-Spray): 601,2 $(M+H)^+$.

Beispiel 14

A) Analog Beispiel 1 erhält man:

a) aus dem (S)-N-Cyclopropyl-N-äthoxycarbonylmethyl-3-(4-trifluormethyl-phenylsulfonylamino)-succinamsäure-t-butylester,
das [(S)-3-[(S)-1-(Amino-imino-methyl)piperidin-3-ylmethylcarbamoyl]-2-(4-trifluormethyl-phenylsulfonylamino)propionyl-cyclopropyl-amino]-essigsäure-äthylester-acetat (1:1), MS (Ion-Spray): 605,4 $(M+H)^+$,

b) aus dem (S)-3-(4-t-Butyl-phenylsulfonylamino)-N-cyclopropyl-N-äthoxycarbonylmethyl-succinamsäure-t-butylester,
das [(S)-3-[(S)-1-(Amino-imino-methyl)piperidin-3-ylmethylcarbamoyl]-2-(4-t-butylphenylsulfonylamino)-propionyl-cyclopropyl-amino]essigsäureäthylester-acetat (1:1), MS (Ion-Spray): 593,5 $(M+H)^+$,

c) aus dem (S)-3-(Biphenyl-4-ylsulfonylamino)-N-cyclopropyl-N-äthoxycarbonylmethyl-succinamsäure-t-butylester,
das (S)-3-[(S)-1-(Amino-imino-methyl)piperidin-3-ylmethylcarbamoyl]-2-(biphenyl-4-ylsulfonylamino)-N-cyclopropyl-propionylaminoessigsäureäthylester-hydrochlorid (1:2), MS (Ion-Spray): 613,4 $(M+H)^+$,

d) aus dem (S)-N-Cyclopropyl-N-äthoxycarbonylmethyl-3-(3-methylchinolin-8-yl-sulfonylamino)-succinamsäure-t-butylester,
das (S)-3-[(S)-1-(Amino-imino-methyl)piperidin-3-ylmethylcarbamoyl]-N-cyclopropyl-2-(3-methylchinolin-8-ylsulfonylamino)-propionylaminoessigsäure-äthylester-acetat (1:1), MS (Ion-Spray): 602,2 $(M+H)^+$.

B) Die Ausgangsdiester erhält man aus t-Butyl-(S)-2-(1-t-butoxyformamido)-N-cyclopropyl-N-[(äthoxycarbonyl) methyl]succinamat (Beispiel 2B)c)1), analog Beispiel 1b), jedoch unter Verwendung der entsprechenden Arylsulfochloriden anstelle von β-Naphthylsulfochlorid:

a) (S)-N-Cyclopropyl-N-äthoxycarbonylmethyl-3-(4-trifluormethyl-phenylsulfonylamino)-succinamsäure-t-butylester, MS (Ion-Spray): 523,0 $(M+H)^+$,

b) (S)-3-(4-t-Butyl-phenylsulfonylamino)-N-cyclopropyl-N-äthoxycarbonylmethyl-succinamsäure-t-butylester, MS (Ion Spray): 511,1 $(M+H)^+$,

c) (S)-3-(Biphenyl-4-ylsulfonylamino)-N-cyclopropyl-N-äthoxycarbonylmethyl-succinamsäure-t-butylester, MS (Ion-Spray): 531,4 $(M+H)^+$,

d) (S)-N-Cyclopropyl-N-äthoxycarbonylmethyl-3-(3-methylchinolin-8-ylsulfonylamino)-succinamsäure-t-butylester, MS (Ion-Spray): 520,2 $(M+H)^+$.

Beispiel 15

Analog Beispiel 3 erhält man aus den Estern von Beispiel 14A) folgende Säuren:

a) [(S)-3-[(S)-1-(Amino-imino-methyl)piperidin-3-ylmethylcarbamoyl]-2-(4-trifluormethyl-phenylsulfonylamino)-propionyl-cyclopropyl-amino]essigsäure, MS (Ion-Spray): 577,4 $(M+H)^+$,

b) [(S)-3-[(S)-1-(Amino-imino-methyl)piperidin-3-ylmethylcarbamoyl]-2-(4-t-butylphenylsulfonylamino)-propionyl-cyclopropyl-amino]-essigsäure, MS (Ion-Spray): 565,2 $(M+H)^+$,

c) (S)-3-[(S)-1-(Amino-imino-methyl)piperidin-3-ylmethylcarbamoyl]-2-(biphenyl-4-ylsulfonylamino)-N-cyclopropyl-propionylamino-essigsäure, MS (Ion-Spray): 585,4 $(M+H)^+$,

d) (S)-3-[(S)-1-(Amino-imino-methyl)piperidin-3-ylmethylcarbamoyl]-N-cyclopropyl-2-(3-methylchinolin-8-ylsulfonylamino)-propionylamino-essigsäure, MS (Ion-Spray): 574,4 $(M+H)^+$.

Beispiel 16

Eine Lösung von 0,34 g des Produkts von Beispiel 15A)d) in 25 ml Aethanol wird mit 1 ml Essigsäure und 0,1 g Pd/C versetzt und unter Normalbedingungen hydriert. Nach Filtration und Eindampfen des Filtrats erhält man 0,12 g

N-[(S)-3-[(S)-1-(Amino-imino-methyl)piperidin-3-ylmethylcarbamoyl]-2-(3-methyl-1,2,3,4-tetrahydrochinolin-8-ylsulfonylamino)-propionyl]-N-cyclopropyl-aminoessigsäure-acetat (1:1), MS (Ion-Spray): 578,4 $(M+H)^+$.

Beispiel 17

Analog Beispiel 1 erhält man aus dem (S)-N-Cyclopropyl-3-(naphthalen-2-ylsulfonylamino)-N-(3-oxo-butyl)-succinamsäure-t-butylester,
das (S)-N4-[(S)-1-(Amino-imino-methyl)piperidin-3-ylmethyl]-N1-cyclopropyl-2-(naphthalen-2-ylsulfonylamino)-N1-(3-oxobutyl)-succinamidhydrochlorid (1:1), MS (Ion-Spray): 571,2 $(M+H)^+$.

Herstellung des Ausgangsmaterials:

a) Zu einer Lösung von 10 g N-Cyclopropyl-β-alanin-äthylester in 100 ml Dioxan tropft man unter Kühlung eine Lösung von 13,9 g Di-t-butyl-dicarbonat in 140 ml Dioxan. Nach Rühren wird das Reaktionsgemisch eingedampft. Man erhält nach Trocknen des Rückstandes 16 g 3-(t-Butoxycarbonyl-cyclopropyl-amino)propionsäure-äthylester, Fab-MS: 201 (M-Isobutylen).

b) Zu einer Lösung von 15,7 g des Produktes von a) in 160 ml THF werden bei 0-3° 42 ml einer 1,6 molaren Methyllithium-Lösung in Aether zugetropft. Nach Rühren bei Raumtemperatur wird auf 0° abgekühlt und weitere 34,5 ml einer 1,6 molaren Methyllithium-Lösung in Aether zugetropft. Nach Rühren giesst man die Reaktionslösung auf eiskalte 5%ige Kaliumhydrogensulfat-10%ige Kaliumsulfatlösung und extrahiert mit Essigester. Die organische Phase wird mit Kochsalzlösung gewaschen, dann getrocknet und eingedampft und der Rückstand mit Hexan-Essigester 4:1 an Kieselgel chromatographiert. Man erhält in einer ersten Fraktion 8,3 g Cyclopropyl(3-hydroxy-3-methylbutyl)carbamidsäure-t-butylester Fab-MS: 187 (M-Isobutylen).

c) Aus dem Chromatogramm von b) isoliert man in einer zweiten Fraktion 1,7 g Cyclopropyl-3-oxobutyl-carbamidsäure-t-butylester, Fab-MS: 171 (M-Isobutylen).

d) Eine Lösung von 18,2 g des Produktes von b) in 80 ml Essigester wird mit 40 ml einer 4 molaren Salzsäurelösung in Essigester versetzt. Nach Rühren filtriert man das ausgefallene Material ab und wäscht mit Essigester. Man erhält nach Trocknen 3,6 g 4-Cyclopropylamino-2-methylbutan-2-ol-hydrochlorid, Fab-MS: 143 $M^+$.

e) Eine Lösung von 3,1 g des Produktes von c) in 30 ml Essigester wird mit 30 ml einer 4 molaren Salzsäurelösung in Essigester versetzt. Nach Rühren wird eingedampft und getrocknet. Man erhält 2,3 g 4-Cyclopropylaminobutan-2-on.

f) Eine Lösung von 3,9 g N-Boc-L-Asparaginsäure-β-t-butylester in 40 ml Methylenchlorid wird mit 5,5 ml 4-Aethylmorpholin, 3,1 g N-(Dimethylaminopropyl)-N'-äthylcarbodiimid-hydrochlorid und 0,17 g 4-Dimethylaminopyridin versetzt. Zu dieser Lösung gibt man das unter e) erhaltene Material, gelöst in 20 ml Methylenchlorid. Nach Rühren giesst man die Reaktionslösung auf eiskalte 5%ige Kaliumhydrogensulfat-10%ige Kaliumsulfat-Lösung und extrahiert mit Methylenchlorid. Die organische Phase wird mit Kochsalzlösung gewaschen, dann getrocknet und eingedampft und der Rückstand mit Hexan-Methylacetat 2:1 an Kieselgel chromatographiert. Man erhält 3,7 g (S)-N-(2-Acetyläthyl)-3-t-butoxycarbonylamino-N-cyclopropyl-succinamsäure-t-butylester, MS (Ion-Spray): 399,3 $(M+H)^+$.

g) Aus dem Produkt von f) erhält man in Analogie zu Beispiel 2.B)i) den (S)-N-Cyclopropyl-3-(naphthalen-2-ylsulfonylamino)-N-(3-oxo-butyl)-succinamsäure-t-butylester, Fab-MS: 433 (M-Isobutylen).

Beispiel 18

Analog Beispiel 17 erhält man aus dem 4-Cyclopropylamino-2-methylbutan-2-ol-hydrochlorid (Beispiel 17 d), via

a) (S)-3-t-Butoxycarbonylamino-N-cyclopropyl-N-(3-hydroxy-3-methylbutyl)-succinamsäure-t-butylester, MS (Ion-Spray): 415,4 $(M+H)^+$ und

b) (S)-N-Cyclopropyl-N-(3-hydroxy-3-methyl-butyl)-2-naphthalen-2-sulfonylamino)-succinamsäure-t-butylester, MS (Ion-Spray): 505,3 $(M+H)^+$,
das (S)-N4-[(S)-1-(Amino-imino-methyl)-piperidin-3-ylmethyl-N1-cyclopropyl-N1-(3-hydroxy-3-methyl-butyl)-2-(naphthalen-2-ylsulfonylamino)-succinamid-hydrochlorid (1:1), MS (Ion-Spray): 587,4 $(M+H)^+$.

Beispiel 19

Analog Beispiel 7A), jedoch ausgehend von den Diestern des Beispiels 14B)a), b), bzw. e) erhält man folgende Ester:

a) [(S)-3-[(R,S)-4-(Amino-imino-methyl)morpholin-2-ylmethylcarbamoyl]-2-(4-trifluormethyl-phenylsulfonylamino)-propionyl-cyclopropyl-amino]-essigsäure-äthylester-acetat (1:1), MS (Ion-Spray): 607,2 $(M+H)^+$,

b) [(S)-3-[(R,S)-4-Amino-imino-methyl)morpholin-2-ylmethylcarbamoyl]-2-(4-t-butyl-phenylsulfonylamino)-propionyl-cyclopropyl-amino]-essigsäure-äthylester-trifluoracetat (1:1), MS (Ion-Spray): 595,3 $(M+H)^+$, bzw.

c) [(S)-3-[(R,S)-4-(Amino-imino-methyl)morpholin-2-ylmethylcarbamoyl]-2-(biphenyl-4-ylsulfonylamino)-N-cyclopropyl-propionylamino]essigsäureäthylester-trifluoracetat (1:1), MS (Ion-Spray): 615,3 $(M+H)^+$.

Beispiel 20

Analog Beispiel 3 erhält man ausgehend von den Estern von Beispiel 19 folgende Säuren:

a) [(S)-3-[(R,S)-4-(Amino-imino-methyl)morpholin-2-ylmethylcarbamoyl]-2-(4-trifluormethyl-phenylsulfonylamino)-propionyl-cyclopropyl-amino]-essigsäure, MS (Ion-Spray): 579,1 $(M+H)^+$,

b) [(S)-3-[(R,S)-4-(Amino-imino-methyl)morpholin-2-ylmethylcarbamoyl]-2-(t-butyl-phenylSulfonylamino)-N-cyclopropyl-propionylamino]essigsäure, MS (Ion-Spray): 567,4 $(M+H)^+$,

c) [(S)-3-[(R,S)-4-(Amino-imino-methyl)-morpholin-2-ylmethylcarbamoyl]-2-(biphenyl-4-ylsulfonylamino)-N-cyclopropyl-propionylamino]essigsäure, MS (Ion-Spray): 587,3 $(M+H)^+$.

Beispiel 21

Analog Beispiel 9, jedoch ausgehend von N-Methyl-3-picolylamin (an Stelle von N-(2-Hydroxyäthyl)-3-picolylamin erhält man via

t-Butylmethyl (3-pyridinylmethyl)carbamat,

t-Butyl-rac-methyl (3-piperidinylmethyl)carbamat,

t-Butyl-rac-[(1-amidino-3-piperidinyl)methyl]methylcarbamat-bisulfit und

rac-3-[(Methylamino)methyl]-1-piperidincarboxamidin-dihydrochlorid, Fab-MS: 171 $(M+H)^+$,

a) unter Verwendung von
(S)-N-Butyl-N-(2-äthoxycarbonyläthyl-3-(naphthalen-2-ylsulfonylaminsuccinamsäure-t-butylester (Beispiel 2.B)j)11)
das 3-[(S)-3-[(R,S)-[1-(Amino-imino-methyl)piperidin-3-ylmethyl]-N-methylcarbamoyl]-N-butyl-2-(naphthalen-2-ylsulfonylamino)-propionylamino]-propionsäure-äthylester-hydrochlorid (1:1),MS (Ion-Spray): 631,5 $(M+H)^+$,

b) unter Verwendung von
N-[3-(t-Butoxycarbonyl)-N-(2-naphthylsulfonyl)-L-alanyl]-N-cyclopropyl-β-alanin (Beispiel 2.B)j)9)
das 3-[[(S)-3-[[(R,S)-1-(Amino-imino-methyl)piperidin-3-ylmethyl)]-methylcarbamoyl]-2-(naphthalen-2-sulfonylamino)-propionyl]-cyclopropylamino]-propionsäure-äthylester-hydrochlorid (1:1), MS (Ion-Spray): 615,4 $(M+H)^+$.

Beispiel 22

Analog Beispiel 3 erhält man aus den Estern von Beispiel 21 folgende Säuren:

a) 3-[(S)-3-[(R,S)-[1-(Amino-imino-methyl)piperidin-3-ylmethyl]-N-methylcarbamoyl]-N-butyl-2-(naphthalen-2-ylsulfonylamino)-propionylamino]-propionsäure, MS (Ion-Spray): 603,5 $(M+H)^+$,

b) 3-[[(S)-3-[[(R,S)-2-(Amino-imino-methyl)piperidin-3-ylmethyl]-methylcarbamoyl]-2-(naphthalen-2-sulfonylamino)-propionyl]-cyclopropyl-amino]-propionsäure, MS (Ion-Spray): 587,4 $(M+H)^+$.

Beispiel 23

Eine Lösung von (S)-3-[(S)-3-[(4-Chlorbenzyl)-methoxycarbonylmethylcarbamoyl]-3-(naphthalen-2-sulfonylamino)-propionylaminomethylpiperidin-1-carbonsäure-t-butylester in 20 ml Methylenchlorid wird mit 4 ml Trifluoressigsäure versetzt. Nach Rühren wird eingedampft, der Rückstand in 2,7 ml Methanol gelöst und mit 0,93 ml Triäthylamin und 330 mg Formamidinsulfonsäure versetzt. Dann werden nochmals 165 mg Formamidinsulfonsäure und 0,19 ml Triäthylamin zugegeben. Nach Rühren engt man das Reaktionsgemisch ein und chromatographiert den Rückstand an Kieselgel mit Essigester-Aceton-Essigsäure-Wasser 6:2:1:1. Man erhält 516 mg N-[N4-[[(S)-1-Amidino-3-piperidinyl]methyl]-N2-(2-naphthylsulfonyl)-L-asparaginyl]-N-(p-chlorbenzyl)glycinmethylester-acetat (1:1), MS (Ion-Spray): 657 $(M+H)^+$.

Herstellung des Ausgangsmaterials:

a) Zu einer Suspension von 10 g L-Asparaginsäure-β-t-butylester und 11,98 g Naphthalin-2-sulfochlorid in 100 ml Dioxan werden bei 6-10° 52,85 ml 2N Natronlauge zugetropft. Nach Rühren tropft man 53 ml 1N Salzsäure zu. Das Reaktionsgemisch nimmt man in 800 ml Aether auf und wäscht die Aether/Dioxan-Phase mit Wasser. Nach Trocknen und Eindampfen der organischen Phasen wird der Rückstand in Aether kristallisiert. Nach Abfiltrieren der Kristalle erhält man 13,7 g N-(2-Naphthylsulfonyl)-L-asparaginsäure-4-t-butylester, Smp. 141°.

b) Zu 20 g Glycinmethylester-hydrochlorid und 34,8 g Di-t-butyl-dicarbonat in 300 ml Methylenchlorid und 10 ml Wasser tropft man 22,2 ml Triäthylamin. Nach Rühren wird das Reaktionsgemisch eingeengt. Den Rückstand nimmt man in Aether auf und wäscht die Aetherphase nach Zugabe von 5 ml 1N Salzsäure mit Wasser neutral. Nach Trocknen und Eindampfen der Aetherphase erhält man 30,2 g N-BOC-Glycinmethylester. Rf=0,33 (Aether/Hexan 1:1).

c) Zu 1,0 g des Rohprodukts von b) und 937 mg 4-Chlor-benzylchlorid in 10 ml DMF gibt man unter Eiskühlung 242 mg Natriumhydrid (55% in Oel). Nach Rühren nimmt man das Reaktionsgemisch in 100 ml Aether auf und wäscht mit Wasser. Nach Trocknen und Eindampfen der Aetherphase wird der Rückstand über Kieselgel mit Aether/Hexan 1:2 chromatographiert. Man erhält 1,27 g N-BOC-N-(4-chlorbenzyl)-glycinmethylester Rf=0,33 (Aether/Hexan 1:2).

d) 1,275 g des Produkts von c) versetzt man mit 5 ml 10N Salzsäure in Methanol. Das Methanol wird abgedampft und der Rückstand in 20 ml Aether aufgeschlämmt und abfiltriert. Nach Waschen des Rückstandes mit Aether, erhält man 0,93 g N-(4-chlorbenzyl)-glycinmethylester-hydrochlorid, Rf=0,59 (Essigester/Aceton/Wasser/Eisessig 6:2:1:1).

e) Man löst 567 mg des Produkts von a), 394 mg des Produkts von d), 636 mg BOP und 0,5 ml Hünig-Base in 8 ml Methylenchlorid. Nach Rühren nimmt man das Reaktionsgemisch in 100 ml Aether auf und wäscht die Aetherphase mit Salzsäure und Wasser. Nach Trocknen und Eindampfen der Aetherphase chromatographiert man den Rückstand über Kieselgel mit Aether/Hexan 2:1. Man erhält 926 mg (S)-N-(4-Chlorbenzyl)-N-methoxycarbonylmethyl-3-(naphthalen-2-sulfonylamino)-succinamsäure-t-butylester, MS (Ion-Spray): 575 $(M^+)$.

f) 926 mg des Produkts von e) versetzt man mit 6 ml 5-molarer Salzsäure in Dioxan. Nach Rühren nimmt man das Reaktionsgemisch in 100 ml Aether auf und wäscht die Aetherphase mit Wasser. Nach Trocknen und Eindampfen erhält man 877 mg (S)-N-(4-Chlorbenzyl)-N-methoxycarbonylmethyl-3-(naphthalen-2-sulfonylamino)-succinamsäure, $R_f$ =0,7 (Essigester/Eisessig 99:1).

g) 877 mg des Produkts von f), 435 mg [S]-3-Aminomethyl-1-piperidincarbonsäure-t-butylester, 785 mg BOP und 0,58 ml Hünig-Base werden in 12 ml Methylenchlorid gerührt. Das Reaktionsgemisch nimmt man in 100 ml Aether

auf und wäscht die Aetherphase mit 1N Salzsäure und Wasser. Nach Trocknen und Eindampfen der Aetherphasen wird der Rückstand über Kieselgel mit Essigester/Hexan 4:1 chromatographiert. Man erhält 951 mg (S)-3-[(S)-3[(4-Chlorbenzyl)-methoxycarbonylmethylcarbamoyl]-3-(naphthalen-2-sulfonylamino)-propionylaminomethyl]piperidin-1-carbonsäure-t-butylester, MS (Ion-Spray): 715 $(M+H)^+$.

Beispiel 24

Eine Lösung von 300 mg des Esterprodukts von Beispiel 23 in 3 ml THF wird mit 1,25 ml 1N LiOH versetzt. Nach Rühren und Zugabe von 2 ml Essigsäure eingedampft und der Rückstand an Kieselgel mit Essigester-Aceton-Essigsäure-Wasser 6:2:1:1 chromatographiert. Man erhält 154,5 mg N-[N4-[[(S)-1-Amidino-3-piperidinyl]methyl]-N2-(2-naphthylsulfonyl)-L-asparaginyl]-N-(p-chlorbenzyl)glycin, MS (Ion-Spray): 641 (M-H)-.

Beispiel 25

Analog Beispiel 23 stellt man folgende Ester her:

a) N-[N4-[[(S)-1-Amidino-3-piperidinyl]methyl]-N2-(2-naphthylsulfonyl)-L-asparaginyl]-N-(m-chlorbenzyl)glycin-methylester-acetat (1:1), MS (Ion-Spray): 657 $(M+H)^+$,

b) N-[N4-[[(S)-1-Amidino-3-piperidinyl]methyl]-N2-(2-naphthylsulfonyl)-L-asparaginyl]-N-(o-chlorbenzyl)glycin-methylester-acetat (1:1), MS (Ion-Spray): 657 $(M+H)^+$,

c) [N-[(S)-3-[(S)-1-Amidino-piperidin-3-yl-methylcarbamoyl]-2-(naphthalen-2-sulfonylamino)-propionyl]-N-(4-methoxybenzyl)-amino]-essigsäuremethylester-acetat (1:1), MS (Ion-Spray): 653 $(M+H)^+$,

d) [N-[(S)-3-[(S)-1-Amidino-piperidin-3-yl-methylcarbamoyl]-2-(naphthalen-2-sulfonylamino)-propionyl]-N-(pyridin-2-ylmethyl)-amino]-essigsäuremethylester-acetat (1:2), MS (Ion-Spray): 624 $(M+H)^+$,

e) [[(S)-3-[(S)-1-(Amino-imino-methyl)-piperidin-3-ylmethylcarbamoyl]-2-(naphthalen-2-sulfonylamino)-propionyl]-(3-methoxy-benzyl)-amino]-essigsäure-methylester-acetat (1:1), MS (Ion-Spray): 653 $(M+H)^+$.

Beispiel 26

Analog Beispiel 24 erhält man aus den Estern von Beispiel 25 folgende Säuren:

a) N-[N4-[[(S)-1-Amidino-3-piperidinyl]methyl]-N2-(2-naphthylsulfonyl)-L-asparaginyl]-N-(m-chlorbenzyl)glycin, MS (Ion-Spray): 641 $(M-H)^-$,

b) N-[N4-[[(S)-1-Amidino-3-piperidinyl]methyl]-N2-(2-naphthylsulfonyl)-L-asparaginyl]-N-(o-chlorbenzyl)glycin, MS (Ion-Spray): 641 (M-H)-,

c) [N-[(S)-3-[(S)-1-Amidino-piperidin-3-yl-methylcarbamoyl]-2-(naphthalen-2-sulfonylamino)-propionyl]-N-(4-methoxybenzyl)-amino]essigsäure, MS (Ion-Spray): 639 $(M+H)^+$,

d) [N-[(S)-3-[(S)-1-Amidino-piperidin-3-yl-methylcarbamoyl]-2-(naphthalen-2-sulfonylamino)-propionyl]-N-(pyridin-2-ylmethyl)-amino]essigsäureacetat, MS (Ion-Spray): 610 $(M+H)^+$,

e) [[(S)-3-[(S)-1-(Amino-imino-methyl)piperidin-3-ylmethylcarbamoyl]-2-(naphthalen-2-sulfonylamino)-propionyl]-(3-methoxybenzyl)-amino]essigsäure, MS (Ion-Spray): 639 $(M+H)^+$.

Beispiel 27

Analog Beispiel 23, jedoch unter Verwendung der entsprechenden Aminocarbonsäureester an Stelle des N-(4-Chlorbenzyl)glycinmethylesterhydrochlorid (Beispiel 23 d), erhält man folgende Ester:

a) L-N-[N4-[[(S)-1-Amidino-3-piperidinyl]methyl]-N2-(2-naphthylsulfonyl)-L-asparaginyl]-1-phenylglycin-methylester-acetat, (1:1), MS (Ion-Spray): 609 $(M+H)^+$,

b) N-[N4-[[(S)-1-Amidino-3-piperidinyl]methyl]-N2-(2-naphthylsulfonyl)-L-asparaginyl]-L-isoleucin-methylester-acetat (1:1), MS (Ion-Spray): 589 $(M+H)^+$,

c) N-[N4-[[(S)-1-Amidino-3-piperidinyl]methyl]-N2-(2-naphthylsulfonyl)-L-asparaginyl]-L-valin-methylester-acetat (1:1), MS (Ion-Spray): 575 $(M+H)^+$,

d) N-[N4-[[(S)-1-Amidino-3-piperidinyl]methyl]-N2-(2-naphthylsulfonyl)-L-asparaginyl]-D-leucin-methylester-acetat (1:1), MS (Ion-Spray): 589 $(M+H)^+$,

e) N-[N4-[[(S)-1-Amidino-3-piperidinyl]methyl]-N2-(2-naphthylsulfonyl)-L-asparaginyl]-N-methyl-L-valin-methylester-acetat (1:1), MS (Ion-Spray): 589 $(M+H)^+$,

f) N-[N4-[[(S)-1-Amidino-3-piperidinyl]methyl]-N2-(2-naphthylsulfonyl)-L-asparaginyl]-N-methyl-L-isoleucinmethylester-acetat (1:1), MS (Ion-Spray): 603 $(M+H)^+$,

g) (R)-2-[(S)-3-[(S)-1-(Amino-imino-methyl)piperidin-3-ylmethylcarbamoyl]-2-(naphthalen-2-sulfonylamino)-propionylamino]-3-phenyl-propionsäure-methylester-acetat (1:1), MS (Ion-Spray): 623 $(M+H)^+$.

Beispiel 28

Analog Beispiel 24 stellt man ausgehend von den Estern von Beispiel 27 folgende Säure her:

a) L-N-[N4-[[(S)-1-Amidino-3-piperidinyl]methyl]-N2-(2-naphthylsulfonyl)-L-asparaginyl]-2-phenylglycin-acetat, (1:1), MS (Ion-Spray): 595 $(M+H)^+$,

b) N-[N4-[[(S)-1-Amidino-3-piperidinyl]methyl]-N2-(2-naphthylsulfonyl)-L-asparaginyl]-L-isoleucin, MS (Ion-Spray): 575 $(M+H)^+$,

c) N-[N4-[[(S)-1-Amidino-3-piperidinyl]methyl]-N2-(2-naphthylsulfonyl)-L-asparaginyl]-L-valin, MS (Ion-Spray): 561 $(M+H)^+$,

d) N-[N4-[[(S)-1-Amidino-3-piperidinyl]methyl]-N2-(2-naphthylsulfonyl)-L-asparaginyl]-D-leucin, MS (Ion-Spray): 575 $(M+H)^+$,

e) N-[N4-[[(S)-1-Amidino-3-piperidinyl]methyl]-N2-(2-naphthylsulfonyl)-L-asparaginyl]-N-methyl-L-isoleucin, MS (Ion-Spray): 589 $(M+H)^+$,

f) N-[N4-[[(S)-1-Amidino-3-piperidinyl]methyl]-N2-(2-naphthylsulfonyl)-L-asparaginyl]-N-methyl-L-valin, MS (Ion-Spray): 575 $(M+H)^+$,

g) (R)-2-[(S)-3-[(S)-1-(Amino-imino-methyl)piperidin-3-ylmethylcarbamoyl]-2-(naphthalen-2-sulfonylamino)-propionylamino]-3-phenylpropionsäure, MS (Ion-Spray): 609 $(M+H)^+$.

Beispiel 29

Eine Lösung von 1,09 g (S)-3-[(S)-3-[Butyl-[2-(äthoxalylamino-äthyl)]-carbamoyl]-3-(naphthalen-2-sulfonylamino)-propionylaminoäthyl]-piperidin-1-carbonsäure-t-butylester in 20 ml Methylenchlorid wird mit 4 ml Trifluoressigsäure versetzt. Nach Rühren engt man ein, schlämmt den Rückstand mit Aether auf und dekantiert dann den Aether ab. Zum Rückstand gibt man 3 ml Methanol, 1,06 ml Triäthylamin und 377 mg Formamidinsulfonsäure. Nach Rühren gibt man nochmals je 1 Aequivalent Formamidinsulfonsäure und Triäthylamin zu. Das Gemisch wird eingeengt und an Kieselgel mit Essigester-Aceton-Essigsäure-Wasser 6:2:1:1. Man erhält 962 mg [2-[[(S)-3-[(S)-1-(Amino-imino-methyl)piperidin-3-ylmethylcarbamoyl]-2-(naphthalen-2-sulfonylamino)-propionyl]-butyl-amino]äthyl]oxamsäure-methylester-acetat (1:1), MS (Ion-Spray): 646 $(M+H)^+$.

Herstellung des Ausgangsmaterials:

a) 7,0 g 2-Butylamino-äthylchlorid-hydrochlorid (Org.Synth. IV 1963, 333) rührt man zusammen mit 7,9 g Natriumazid in 50 ml DMF bei 50°. Nach Abkühlen tropft man 82 ml 1N Natronlauge zu. Das Gemisch wird in 700 ml Aether aufgenommen, mit Wasser gewaschen und nach Trocknen der Aetherphase mit 25 ml Salzsäure (5 molar

in Dioxan) versetzt. Nach Eindampfen der Aetherphase schlämmt man den Rückstand in Aether auf, filtriert die Kristalle ab und wäscht sie mit Aether. Man erhält 5 g 2-Butylamino-äthylazid-hydrochlorid, Rf=0,14.

b) Zu 8,85 g N-(2-Naphthylsulfonyl)-L-asparaginsäure-4-t-butylester (Beispiel 23e) in 120 ml Methylenchliorid gibt man 5,0 g 2-Butylaminoäthylazid-hydrochlorid, 10,8 g BOP und 11,98 ml Hünig-Base. Nach Rühren nimmt man in 600 ml Aether auf und wäscht die Aetherphase mit 1N Salzsäure und mit Wasser. Nach Trocknen und Eindampfen der Aethernhase chromatographiert man den Rückstand über Kieselgel mit Methylenchlorid/Aether 19:1 und erhält 6,18 g (S)-[3-[(2-Azidoäthyl)-butyl-carbamoyl]-3-naphthalen-2-sulfonylamino)-propionsäure-t-butylester Rf=0,42 (Methylenchlorid/Aether 9:1).

c) 6,18 g des Produkts von b) versetzt man mit 60 ml 5N Salzsäure in Dioxan. Nach Rühren nimmt man in 400 ml Aether auf und wäscht die Aetherphase mit Wasser. Nach Trocknen und Eindampfen erhält man 5,58 g (S)-[3-[(2-Azidoäthyl)-butyl-carbamoyl]-3-naphthalen-2-sulfonylamino)-propionsäure, Rf=0,21 (Essigester).

d) 5,57 g des Produkts von c), 3,3 g (S)-3-Aminomethyl-1-piperidincarbonsäure-t-butylester, 5,97 g BOP und 4,4 ml Hünig-Base rührt man in 80 ml Methylenchlorid. Dann nimmt man das Gemisch in Aether auf und wäscht die Aetherphase mit 1N Salzsäure und mit Wasser. Nach Tocknen und Eindampfen chromatographiert man das Produkt an Kieselgel mit Essigester/Hexan 4:1 und erhält 6,43 g (S)-3-[(S)-3-[(2-Azidoäthyl)-butylcarbamoyl]-3-(naphthalen-2-sulfonylamino)-propionylaminomethyl]piperidin-1-carbonsäure-t-butylester (Essigester/Hexan 4:1).

e) 6,43 g des Produkts von d) versetzt man in 60 ml Methanol mit 650 mg 5% Pd/C und hydriert unter Normalbedingungen. Den Katalysator filtriert man ab und engt das Filtrat ein,. Man erhält 5,86 g (S)-3[(S)-3-[(2-Aminoäthyl)-butyl-carbamoyl]-3-(naphthalen-2-sulfonylamino)-propionylaminomethyl]piperidin-1-carbonsäure-t-butylester, Rf=0,33 (Essigester/Aceton/-Wasser/Essigsäure 6:2:1:1).

f) Zu 1,2 g des Produkts von e) und 0,32 ml Pyridin tropft man bei 0-5° eine Lösung von 0,23 ml Oxalsäure-mono-äthylesterchlorid in 6 ml Methylenchlorid. Nach Rühren nimmt man in 100 ml Aether auf und wäscht die Aetherphase mit IN Salzsäure und mit Wasser. Nach Trocknen und Eindampfen reinigt man das Produkt an Kieselgel mit Essigester. Man erhält 1,09 g (S)-3-[(S)-3-[Butyl-[2-(ethoxalylamino-ethyl)]carbamoyl]-3-(naphthalen-2-sulfonylamino)-propionylamino-ethyl]-piperidin-1-carbonsäure-t-butylester, MS (Ion-Spray). 718 $(M+H)^+$.

Beispiel 30

672 mg des Esterprodukts von Beispiel 29 in 6,7 ml THF werden mit einer Lösung von 2,8 ml 1N Lithiumhydroxyd gerührt. Dann versetzt man das Gemisch mit 4 ml Essigsäure und engt ein. Den Rückstand reinigt man an Kieselgel mit Essigester-Aceton-Essigsäure-Wasser 6:2:1:1, 461 mg [2-[[(S)-3-[(S)-1-(Amino-imino-methyl)piperidin-3-ylmethylcarbamoyl]-2-(naphthalen-2-sulfonylamino)-propionyl]-butyl-amino]äthyloxamsäure, MS (Ion-Spray): 632 $(M+H)^+$.

Beispiel 31

Analog Beispiel 29, jedoch unter Verwendung von a) Acetanhydrid, b) Methansulfochlorid, c) $SO_3$-$N(CH_3)_2$-Komplex bzw. d) Chlorameisensäuremethylester, an Stelle des in Beispiel 29 f) verwendeten Oxalsäure-mono-äthylesterchlorid erhält man folgende Produkte:

a) (S)-N1-(2-Acetylaminoäthyl)-N4-[(S)-1-(amino-imino-methyl)piperidin-3-ylmethyl]-N1-butyl-1-(naphthalen-2-sulfonylamino)-succinamid-acetat (1:1), MS (Ion-Spray): 602 $(M+H)^+$,

b) (S)-N4-[(S)-1-(Amino-imino-methyl)piperidin-3-ylmethyl]-N1-butyl-N1-(2-methansulfonylamino-äthyl)-2-(naphthalen-2-sulfonylamino)-succinamid-acetat (1:1), MS (Ion-Spray): 638 $(M+H)^+$,

c) (S)-N4-[(S)-1-(Amino-imino-methyl)piperidin-3-ylmethyl]-N1-butyl-2-(naphthalen-2-sulfonylamino)-N1-(2-sulfoamino-äthyl)-succinamid, MS (Ion-Spray): 640 $(M+H)^+$,

d) 2-[[(S)-3-[(S)-1-(Amino-imino-methyl)piperidin-3-ylmethylcarbamoyl-2-(naphthalen-2-sulfonylamino)-propionyl]-butyl-amino]-äthyl]-carbamsäuremethylester-acetat (1:1), MS (Ion-Spray): 618 $(M+H)^+$,

Beispiel 32

A) Analog Beispiel 29 bzw. 30 stellt man folgende Produkte her:

a) Essigsäure-3-[(S)-3-[(S)-1-Amino-imino-methyl)piperidin-3-ylmethylcarbamoyl]-2-(naphthalen-2-sulfonylamino)-propionyl-cyclopropylamino]-propylester-acetat (1:1), MS (Ion-Spray): 601,3 $(M+H)^+$, bzw.

b) (S)-N4-[(S)-(1-Amino-imino-methyl)piperidin-3-ylmethyl]-N1-cyclopropyl-N1-(3-hydroxypropyl)-2-(naphthalen-2-sulfonylamino)-succinamidacetat (1:1), MS (Ion-Spray): 559 $(M+H)^+$.

B) Herstellung des an Stelle des 2-Butylamino-äthylazid-hydrochlorid (Beispiel 29 a) verwendeten Ausgangsamins:

a) Zu einer Lösung von 6,86 g N-Boc-Cyclopropylamin und 13,27 g 3-(t-Butyl-dimethylsilyloxy)propylbromid in 70 ml DMF gibt man bei 0-5° 2,0 g Natriumhydrid (55% in Oel). Nach Rühren nimmt man in Aether auf und wäscht die Aetherphase mit Wasser. Nach Trocknen und Eindampfen der Aetherphase und Chromatographie an Kieselgel mit Aether/Hexan 1:9 erhält man 11,73 g [3-(t-Butyl-dimethyl-silanyloxy)propyl]-cyclopropylcarbamidsäure-t-butylester, Rf=0,38 (Aether/Hexan 1:4).

b) Man löst 11,73 g des Produkts von a) in 42,7 ml einer 1M Lösung von Tetrabutylammoniumfluorid in in THF. Nach Rühren nimmt man in Aether auf und wäscht die Aetherphase mit Wasser. Nach Trocknen und Eindampfen erhält man 7,02 g N-Boc-3-Cyclopropylamino-propanol, Rf=0,47 (Methylenchlorid/Aether 1:1).

c) Man versetzt eine Lösung von 1,92 g des Produkts von b) in 19 ml Methylenchlorid mit 1,44 ml Pyridin und 0,89 ml Acetanhydrid. Nach Rühren nimmt man in Aether auf und wäscht die Aetherphase mit IN Salzsäure und mit Wasser. Nach Trocknen und Eindampfen der Aetherphase und Chromatographie an Kieselgel mit Aether/Hexan 1:2 erhält man 2,3 g N-Boc-3-Cyclopropylamino-propylacetat, Rf=0,18 (Aether/Hexan 1:2).

d) 2,3 g des Produkts von c) werden mit 23 ml 4,3M Salzsäure in Dioxan versetzt. Nach Abdampfen des Lösungsmittels, schlämmt man den Rückstand mit Aether auf und dekantiert anschliessend den Aether ab. Nach dem Trocknen erhält man 1,61 g 3-Cyclopropylamino-propionsäuremethylester-hydrochlorid (1:1) Rf=0,17 (Essigester, Aceton, Essigsäure, Wasser 6:2:1:1).

Beispiel 33

Analog Beispiel 12 erhält man aus dem (R)-3-[[(S)-3-Benzyl-methylcarbamoyl)-3-(naphthalen-2-sulfonylamino)-propionyl]-äthoxycarbonylmethyl-aminomethyl]-piperidin-1-carbonsäure-t-butylester
das [[(R)-1-(Amino-imino-methyl)piperidin-3-ylmethyl]-[(S)-3-(benzylmethyl-carbamoyl)-3-(naphthalen-2-sulfonylamino)propionyl]-amino]-3-essigsäure-äthylester-sulfit (2:1), MS (Ion-Spray): 651,3 $(M+H)^+$.

Herstellung des Ausgangsmaterials:

a) Zu einer Lösung von 7,6 g N-(2-Naphthylsulfonyl)-L-asparaginsäure-4-t-butylester (Beispiel 23 a) in 80 ml Methylenchlorid gibt man 8,1 ml 4-Aethylmorpholin, 4,6 g N-(Dimethylaminopropyl)-N'-äthylcarbodiimidhydrochlorid, 0,24 g 4-Dimethylaminopyridin und 2,6 ml N-Benzylmethylamin. Nach Rühren wird das Reaktionsgemisch auf eiskalte 5%ige Kaliumhydrogensulfat-10%ige Kaliumsulfat-Lösung gegossen und mit Methylenchlorid extrahiert. Die organische Phase wird mit Kochsalzlösung gewaschen, getrocknet und eingedampft. Der Rückstand wird mit Hexan-Essigester (3:1) an Kieselgel chromatographiert. Man isoliert 3,4 g (S)-N-Benzyl-N-methyl-3-(naphthalen-2-ylsulfonylamino)succinamidsäure-1-t-butylester, MS (Ion-Spray): 483,4 $(M+H)^+$.

b) Aus dem Produkt von a) erhält man analog Beispiel 12 i) den (R)-3-[[(S)-3-Benzyl-methyl-carbamoyl)-3-(naphthalen-2-sulfonylamino)-propionyl]-äthoxycarbonylmethyl-aminomethyl]-piperidin-1-carbonsäure-t-butylester, MS (Ion-Spray): 709,5 $(M+H)^+$.

Beispiel 34

Eine Lösung von 0,2 g des Esters von Beispiel 33 in 10 ml Methanol wird mit 1,4 1N Natronlauge versetzt.

Nach Rühren wird die Reaktionslösung mit 6 ml 1N Salzsäure versetzt und eingedampft. Der Rückstand wird mit einem Wasser-Acetonitril Gradienten an RP-18 chromatographiert. Man isoliert 0,1 g [[(R)-1-(Amino-imino-methyl)piperidin-3-ylmethyl]-[(S)-3-(benzyl methyl-carbamoyl)-3-(naphthalen-2-sulfonylamino)-propionyl]-amino)-3-essigsäure-hydrochlorid (1:1), MS (Ion-Spray): 623,3 $(M+H)^+$.

Beispiel 35

Analog Beispiel 9 jedoch ausgehend von (S)-N-Benzyl-N-methyl-3-(naphthalen-2-ylsulfonylamino)succinamidsäure-1-t-butylester (Beispiel 33 a) erhält man das (S)-N4-[(R,S)-1-(Amino-imino-methyl)piperidin-3-ylmethyl)]-N1-benzyl-N4-(2-hydroxyäthyl)-N1-methyl-2-(naphthalen-2-sulfonylamino)-succinamid-hydrochlorid (1:1), MS (Ion-Spray): 609,3 $(M+H)^+$.

Beispiel 36

Analog Beispiel 1 stellt man aus (S)-N-Cyclopropyl-N-(2-tetrazol-5-yl-ethyl)-3-naphthalin-2-ylsulfonylamino-succinamsäure-tert-butylester und aus (S)-1-Amidino-3-(aminomethyl)piperidin-dihydrochlorid das (S)-N4-[(S)-1-(Amino-imino-methyl)-piperidin-3-ylmethyl]-N1-cyclopropyl-N1-(2-tetrazol-5-yl-ethyl)-2-(naphthyl-2-ylsulfonylamino)-succinamid her, MS (ISP): 597.4 $(M+H)^+$.

Herstellung der Ausgangsmaterialien:

Aa) Analog Beispiel 2B)e) jedoch unter Verwendung von 3-Cyclopropylamino-propionitril an Stelle von Sarcosinethylester erhält man den (S)-3-tert-Butoxycarbonylamino-N-cyclopropyl-N-2-(cyano-ethyl)-succinamsäuretert-butylester, MS (ISP): 382.2 $(M+H)^+$.

Ab) Analog Beispiel 2B)i) jedoch unter Verwendung des Esters von a) an Stelle von (S)-3-t-Butoxycarbonylamino-N-äthoxycarbonylmethyl-N-methylsuccinamsäure-t-butylester erhält man (S)-N-Cyclopropyl-N-(2-cyanoethyl)-3-naphthalin-2-sulfonylaminosuccinamsäure-tert-butylester, MS (FAB): 414 (M-Isobutylen).

Ac) Zu einer Lösung von 2.3 g des unter b) erhaltenen Materials in 25 ml DMF gibt man nacheinander 0.7 g Ammoniumchlorid und 0.86 g Natriumazid. Das Reaktionsgemisch wird 24 Stunden bei 80° gerührt, abgekühlt, filtriert und das Filtrat eingedampft. Nach Chromatografie des Rückstandes an Kieselgel mit Essigester + 0.5 % Essigsäure erhält man 0.3 g (S)-N-Cyclopropyl-N-(2-tetrazol-5-yl-ethyl)-3-naphthalin-2-ylsulfonylaminosuccinamsäure tert-butylester, MS (ISP): 515.4 $(M+H)^+$.

Ba) Eine Lösung von 42.5 g N-(3-Pyridinylmethyl)-benzamid in 220 ml Ethanol und 220 ml 1N Salzsäure werden mit 4.2 g Palladium auf Kohle versetzt und während 24 Stunden über 100 bar Wasserstoff bei Raumtemperatur hydriert. Dann wird vom Katalysator filtriert und das Filtrat eingedampft. Der Rückstand wird in Methylenchlorid aufgenommen und mit 1N Natronlauge geschüttelt. Die organissche Phase wird mit Wasser gewaschen, getrocknet und eingedampft. Man erhält 36.1 g (RS)-N-Piperidin-3-ylmethyl-benzamid, MS (FAB): 218 $M^+$.

Bb) 36.1 g des unter Ba) erhaltenen Materials werden in 800 ml Methylenchlorid gelöst und mit 25.2 g D-Mandelsäure versetzt. Zur entstandenen Lösung tropft man unter Rühren 380 ml Ether. Nach Animpfen kristallisieren 32.5 g Salz aus. Nochmaliges Umkristallisieren aus 420 ml Methylenchlorid, 10 ml Methanol und 140 ml Ether ergeben 19.5 g (R)-N-Piperidin-3-ylmethyl-benzamid-(R)-hydroxy-phenyl-acetat (1:1), Smp: ab 75°, Zersetzung.

Bc) 19.3 g des unter Bb) erhaltenen Mandelsäuresalzes werden in 193 ml DMF suspendiert, mit 21.7 ml Triethylamin und 7.75 g Formamidinsulfonsäure versetzt und bei Raumtemperatur gerührt. Das Reaktionsgemisch wird eingedampft und der Rückstand an RP-18 Kieselgel mit einem Wasser-Acetonitril Gradienten chromatografiert. Man isoliert 13.4 g (S)-N-[1-(Amino-imino-methyl)-piperidin-3-ylmethyl]-benzamid-(R)-hydroxyphenyl-acetat (1:1), MS (FAB): 218 M-($H_2N$-CN).

Bd) 13.4 g des unter Bc) erhaltenen Mandelsäuresalzes werden in 267 ml konzentrierter Salzsäure gelöst und unter Rückfluss gekocht. Nach Abkühlen wird die Lösung mit Ether extrahiert, dann die Wasserphase eingedampft und mit Ethanol azeotropiert. Der Rückstand wird in 50 ml Ethanol aufgeschlämmt, im Eisbad abgekühlt und genutscht. Man erhält 4.6 g (S)-1-Amidino-3-(aminomethyl)piperidin-dihydrochlorid, $[\alpha]_D$ -16.3° (c=1.0, Wasser).

Beispiel 37

Analog Beispiel 1 jedoch unter Verwendung des Nitrils von Beispiel 36Ab) an Stelle von t-Butyl-(S)-N-Cyclohexyl-N-[(äthoxycarbonyl)-methyl]-3-(2-naphthylsulfonamido)succinamat erhält man

das (S)-N4-[(S)-1-(Amino-imino-methyl)-piperidin-3-ylmethyl]-N1-(2-carbamoyl-ethyl)-N1-cyclopropyl-2-(naphthyl-2-sulfonylamino)-succinamidhydrochlorid. MS (ISP): 572.3 $(M+H)^+$.

Beispiel 38

38A) Analog Beispiel 1 stellt man folgende Verbindungen her:

38Aa) aus (S)-3-(4-Cyclopentyl-benzolsulfonylamino)-N-cyclopropyl-N-(2-ethoxycarbonyl-ethyl)-succinamsäure-tert-butylester

das 3-[Cyclopropyl-[(S)-3-[(S)-1-(amino-imino-methyl)-piperidin-3-ylmethyl]-2-(4-cyclopentyl-phenylsulfonylamino)-propionyl]-amino]-propionsäureethylester-hydrochlorid, MS (FAB): 619.2 $(M+H)^+$

38Ab) aus (S)-2-[2-tert-Butoxycarbonyl-1-[cyclopropyl-(2-ethoxycarbonyl-ethyl)-carbamoyl]-ethylsulfamoyl]-benzoesäuremethylester

das 2-[(S)-2-[(S)-1-(Amino-imino-methyl)-piperidin-3-ylmethylcarbamoyl]-1-[cyclopropyl-(2-ethoxycarbonyl-ethyl)-carbamoyl]-ethylsulfamoyl]-benzoesäuremethylester-hydrochlorid, MS (ISP): 609.4 $(M+H)^+$

38Ac) aus (S)-N-Cyclopropyl-N-ethoxycarbonylmethyl-3-naphthalin-1-ylsulfonylamino-succinamsäure-tert-butylester

das [[(S)-3-[(S)-(1-Amino-imino-methyl)-piperidin-3-ylmethylcarbamoyl]-2-(naphthalen-1-ylsulfonylamino)-propionyl]-cyclopropyl-amino]-essigsäureethylester-hydrochlorid, MS (FAB): 587.4 $(M+H)^+$

38Ad) aus (S)-N-Cyclopropyl-N-(2-ethoxycarbonyl-ethyl)-3-(4-trifluormethoxybenzolsulfonylamino)-succinamsäure-tert-butylester

das 3-[[(S)-3-[(S)-1-(Amino-imino-methyl)-piperidin-3-ylmethylcarbamoyl-2-(4-trifluormethoxy-benzolsulfonylamino)-propionyl]-cyclopropyl-amino]-propionsäureethylester-hydrochlorid, MS (ISP): 635.5 $(M+H)^+$

38Ae) aus (S)-3-(4-Cyäno-benzolsulfonylamino)-N-cyclopropyl-N-(2-ethoxycarbonyl-ethyl)-succinamsäure-tert-butylester

das 3-[[(S)-3-[(S)-1-(Amino-imino-methyl)-piperidin-3-ylmethylcarbamoyl]-2-(4-cyano-phenylsulfonylamino)-propionyl]-cyclopropyl-amino]-propionsäureethylester-hydrochlorid, MS (ISP): 576.7 $(M+H)^+$

38Af) aus (S)-N-Cyclopropyl-N-(2-ethoxycarbonyl-ethyl)-3-methansulfonylamino-succinamsäure-tert-butylester

das 3-[[(S)-3-[(S)-1-(Amino-imino-methyl)-piperidin-3-ylmethylcarbamoyl]-2-methylsulfonylamino-propionyl]-cyclopropyl-amino]-propionsäureethylester-hydrochlorid, MS (ISP): 489.4 $(M+H)^+$

38Ag) aus (S)-N-Cyclopropyl-N-(2-ethoxycarbonyl-ethyl)-3-(pyridin-3-ylsulfonylamino)-succinamsäure-tert-butylester

das 3-[[(S)-3-[(S)-1-(Amino-imino-methyl)-piperidin-3-ylmethylcarbamoyl]-2-pyridin-3-ylsulfonylamino-propionyl]-cyclopropyl-amino]-propionsäureethylester-trifluoracetat. MS (ISP): 552.6 $(M+H)^+$

38B) Herstellung des Ausgangsmaterial:

Die Ausgangsdiester erhält man analog dem Verfahren in Beispiel 2B)i) aus (S)-3-t-Butoxycarbonylamino-N-cyclopropyl-N-(2-ethoxycarbonylethyl)-succinamsäure-t-butylester (Beispiel 2B)h)1) durch Verwendung der entsprechenden Arylsulfochloride anstelle von 2-Naphthylsulfochlorid:

38Ba) (S)-3-(4-Cyclopentyl-benzolsulfonylamino)-N-cyclopropyl-N-(2-ethoxycarbonyl-ethyl)-succinamsäure-tert-butylester, MS (FAB): 481 (M-Isobutylen)

38Bb) (S)-2-[2-tert-Butoxycarbonyl-1-[cyclopropyl-(2-ethoxycarbonyl-ethyl)-carbamoyl]-ethylsulfamoyl]-benzoesäuremethylester, MS (FAB): 471 (M-Isobutylen)

38Bc) (S)-N-Cyclopropyl-N-ethoxycarbonylmethyl-3-naphthalin-1-ylsulfonylamino-succinamsäure-tert-butylester, MS (ISP): 505.3 $(M+H)^+$

38Bd) (S)-N-Cyclopropyl-N-(2-ethoxycarbonyl-ethyl)-3-(4-trifluormethoxybenzolsulfonylamino)-succinamsäure-tert-butylester, MS (FAB): 497 (M-Isobutylen)

38Be) (S)-3-(4-Cyano-benzolsulfonylamino)-N-cyclopropyl-N-(2-ethoxycarbonyl-ethyl)-succinamsäure-tert-butylester, MS (ISP): 494.2 $(M+H)^+$

38Bf) (S)-N-Cyclopropyl-N-(2-ethoxycarbonyl-ethyl)-3-methanesulfonylaminosuccinamsäure-tert-butylester, MS (FAB): 361 ($M^+$-OEt)

38Bg) (S)-N-Cyclopropyl-N-(2-ethoxycarbonyl-ethyl)-3-(pyridin-3-ylsulfonylamino)-succinamsäure-tert-butylester, MS (ISP): 470.2 $(M+H)^+$.

Beispiel 39

Analog Beispiel 3 jedoch ausgehend von den Estern von Beispiel 38A erhält man folgende Säuren:

a) 3-[Cyclopropyl-[(S)-3-[(S)-1-(amino-imino-methyl)-piperidin-3-ylmethyl]-2-(4-cyclopentyl-phenylsulfonylamino)-propionyl]-amino]-propionsäure, MS (FAB): 591.3 $(M+H)^+$

b) 2-[(S)-2-[(S)-1-(Amino-imino-methyl)-piperidin-3-ylmethylcarbamoyl]-1-[cyclopropyl-(2-carboxy-ethyl)-carbamoyl]-ethylsulfamoyl]-benzoesäure, MS (ISP): 567.2 $(M+H)^+$

c) [[(S)-3-[(S)-1-(Amino-imino-methyl)-piperidin-3-ylmethylcarbamoyl]-2-naphthalen-1-ylsulfonylamino-propionyl]-cyclopropyl-amino]-essigsäure, MS (FAB): 559.4 $(M+H)^+$

d) 3-[N-Cyclopropyl-N-[(S)-3-[(S)-1-(amino-imino-methyl)-piperidin-3-ylmethylcarbamoyl]-2-(4-trifluoromethoxy-phenylsulfonylamino)-propionyl]-amino]-propionsäure, MS (FAB): 607.2 $(M+H)^+$

e)1) 3-[[(S)-3-[(S)-1-(Amino-imino-methyl)-piperidin-3-ylmethylcarbamoyl]-2-(4-cyano-phenylsulfonylamino)-propionyl]-cyclopropyl-amino]-propionsäure, MS (ISP): 548.5 $(M+H)^+$

e)2) 3-[[(S)-3-[(S)-1-(Amino-imino-methyl)-piperidin-3-ylcarbamoyl]-2-(4-carbamoyl-phenylsulfonylamino)-propionyl]-cyclopropyl-amino]-propionsäure, MS (ISP): 566.6 $(M+H)^+$

f) 3-[[(S)-3-[(S)-1-(Amino-imino-methyl)-piperidin-3-ylmethylcarbamoyl]-2-methylsulfonylamino-propionyl]-cyclopropyl-amino]-propionsäure, MS (ISP): 461.3 $(M+H)^+$

g) 3-[[(S)-3-[(S)-1-(Amino-imino-methyl)-piperidin-3-ylmethylcarbamoyl]-2-pyridin-3-ylsulfonylamino-propionyl]-cyclopropyl-amino]-propionsäure, MS (ISP): 524.3 $(M+H)^+$.

Beispiel 40

Analog Beispiel 1 stellt man das 3-[(S)-3-[(S)-1-(Amino-imino-methyl)-piperidin-3-ylmethylcarbamoyl]-N-cyclopropyl-2-(4-tetrazol-5-yl-phenylsulfonylamino)-propionylamino]-propionsäureethylester-acetat her, MS (ISP): 619.4 $(M+H)^+$.

Herstellung des Ausgangsmaterials:

1.4 g des in Beispiel 38B)e) hergestellten Diesters werden in 14 ml DMF gelöst, mit 410 mg Ammoniumchlorid und 500 mg Natriumazid versetzt und 24 Stunden bei 80° gerührt. Nach Abkühlen auf Raumtemperatur wird das Reaktionsgemisch filtriert und das Filtrat eingedampft. Man isoliert 1.8 g (S)-N-Cyclopropyl-N-(2-ethoxycarbonyl-ethyl)-3-(4-tetrazol-5-yl-phenyl-sulfonylamino)-succinamsäure-tert-butylester, MS (ISP): 537.4 $(M+H)^+$.

Beispiel 41

Analog Beispiel 3 erhält man aus dem Ester von Beispiel 40 die 3-[(S)-3-[(S)-1-(Amino-imino-methyl)-piperidin-3-ylmethylcarbamoyl]-N-cyclopropyl-2-[4-(tetrazol-5-yl)-phenylsulfonylamino]-propionylamino]-propionsäure, MS (ISP): 591.4 $(M+H)^+$.

Beispiel 42

Analog Beispiel 1, jedoch unter Verwendung der folgenden Enantiomeren stellt man folgende Verbindungen her:

a) aus N-Boc-D-Asparaginsäure-β-t-butylester anstelle von N-Boc-L-Asparaginsäure-β-t-butylester:
das [[(R)-3-[(S)-1-(Amino-imino-methyl)-piperidin-3-ylmethylcarbamoyl]-2-(naphthalen-2-ylsulfonylamino)-propionyl]-cyclopropylamino]-essigsäureethylester-hydrochlorid, MS (ISN): 585.4 (M-H)-

b) aus (R)-1-Amidino-3-(aminomethyl)-piperidin-dihydrochlorid anstelle von (S)-1-Amidino-3-(aminomethyl)-piperidin-dihydrochlorid:
das [[(S)-3-[(R)-1-(Amino-imino-methyl)-piperidin-3-ylmethylcarbamoyl]-2-(naphthalen-2-ylsulfonylamino)-propionyl]-cyclopropyl-amino]-essigsäureethylester-hydrochlorid, MS (ISN): 585.7 (M-H)-

c) aus N-Boc-D-Asparaginsäure-β-t-butylester anstelle von N-Boc-L-Asparaginsäure-β-t-butylester und (R)-1-Amidino-3-(aminomethyl)-piperidin-dihydrochlorid anstelle von (S)-1-Amidino-3-(aminomethyl)-piperidin-dihydrochlorid
das [[(R)-3-[(R)-1-(Amino-imino-methyl)-piperidin-3-ylmethylcarbamoyl]-2-(naphthalen-2-ylsulfonylamino)-propionyl]-cyclopropyl-amino]-essigsäureethylester-hydrochlorid, MS (ISP): 587.6 $(M+H)^+$.

Herstellung des Ausgangsguanidins von Beispiel 42b):
In einem zu Beispiel 36B) analogen Verfahren, jedoch unter Verwendung von L-Mandelsäure anstelle von D-Mandelsäure erhält man via

a) (S)-N-Piperidin-3-ylmethyl-benzamid-(S)-hydroxy-phenyl-acetat (1:1), MS (FAB): 218 $M^+$ und

b) (R)-N-[1-(Amino-imino-methyl)-piperidin-(3)-ylmethyl]-benzamid-(S)-hydroxy-phenyl-acetat (1:1), MS (ISP): 261.4 $(M+H)^+$
das (R)-1-Amidino-3-(aminomethyl)-piperidin-dihydrochlorid, $[\alpha]_D^{20}$ = +17.6° (c=1.0, Wasser).

Beispiel 43

Analog Beispiel 3 stellt man aus den Estern von Beispiel 42 folgende Säuren her:

a) [[(R)-3-[(S)-1-(Amidino-imino-methyl)-piperidin-3-ylmethylcarbamoyl]-2-(naphthalen-2-ylsulfonylamino)-propionyl]-cyclopropyl-amino]-essigsäure, MS (ISP): 559.5 $(M+H)^+$

b) [[(S)-3-[(R)-1-(Amidino-imino-methyl)-piperidin-3-ylmethylcarbamoyl]-2-(naphthalen-2-ylsulfonylamino)-propionyl]-cyclopropyl-amino]-essigsäure, MS (ISP): 559.5 $(M+H)^+$

c) [[(R)-3-[(R)-1-(Amidino-imino-methyl)-piperidin-3-ylmethylcarbamoyl]-2-(naphthalen-2-ylsulfonylamino)-propionyl]-cyclopropyl-amino]-essigsäure, MS: (ISP): 559.5 $(M+H)^+$.

Beispiel 44

Analog Beispiel 1 erhält man aus den entsprechenden t-Butylestern und unter Verwendung von rac-2-(Aminomethyl)-4-morpholinecarboxamidin-trifluoracetat (Beispiel 7Bc)) anstelle des (S)-Amidino-3-(aminomethyl)-piperidin-dihydrochlorids die folgenden Produkte:

a) aus (S)-N-Cyclopropyl-N-(2-tetrazol-5-yl-ethyl)-3-naphthalin-2-ylsulfonylamino-succinamsäure tert-butylester (Beispiel 36Ac) ):
das (S)-N(4)-[4-(Amino-imino-methyl)-morpholin-2-ylmethyl]-N(1)-cyclopropyl-N(1)-[2-(tetrazol-5-yl)-ethyl]-2-(naphthalin-2-ylsulfonyl)-succinamid ((1:1)-Epimerengemisch, MS (ISP): 599.5 $(M+H)^+$

b) aus (S)-N-Cyclopropyl-N-[2-(5-methyl-1,2,4-oxadiazol-3-yl)-ethyl]-3-(naphthalen-2-ylsulfonylamino)-succinamsäure tert-butylester (Beispiel 47e)):
das (S)-N(4)-[4-(Amino-imino-methyl)-morpholin-2-ylmethyl]-N(1)-cyclopropyl-N(1)-[2-(5-methyl-1,2,4-oxadiazol-3-yl)-ethyl]-2-(naphthalen-2-ylsulfonylamino)-succinamid-hydrochlorid (1:1), (1:1)(1:1)-Epimerengemisch, MS (ISP): 613.7 $(M+H)^+$.

Beispiel 45

Analog Beispiel 1 erhält man aus den entsprechenden Estern unter Verwendung von rac-2-(Aminomethyl)-4-morpholinecarboxamidintrifluoracetat an Stelle von (S)-1-Amidino-3-(aminomethyl)piperidindihyrochlorid die folgenden Produkte:

a) aus (S)-N-Cyclopropyl-N-ethoxycarbonylmethyl-3-(naphthalen-2-sulfonylamino)-succinamsäure t-butylester (Beispiel 2B)d)1)):
das [[(S)-3-[4-(Amino-imino-methyl)-morpholin-2-ylmethylcarbamoyl]2-(naphthalin-2-yl-sulfonyl)-propionyl]-cyclopropyl-amino]-essigsäure-ethylester-hydrochlorid (1:1), (1:1)(1:1)-Epimerengemisch, MS (ISP): 589.5 $(M+H)^+$

b) aus dem Ester von Beispiel 38 Bb):
das 2-[(S)-2-[4-(Amino-imino-methyl)-morpholin-2-ylmethylcarbamoyl]-1-[(2-ethoxycarbonyl-ethyl)-cyclopropyl-carbamoyl]-ethylsulfamoyl]-benzoesäuremethylester-trifluoracetat (1:1), (1:1)-Epimerengemisch, MS (ISP): 611.6 $(M+H)^+$.

Beispiel 46

Analog Beispiel 3 jedoch ausgehend von den Estern von Beispiel 45 erhält man folgende Säuren:
a) [[(S)-3-[4-(Amino-imino-methyl)-morpholin-2-ylmethylcarbamoyl]-2-(naphthalen-2-yl-sulfonyl)-propionyl]-cyclopropyl-amino]-essigsäure, (1:1)-Epimerengemisch, MS (ISP): 561.4 $(M+H)^+$

b)1) 2-[(S)-2-[4-(Amino-imino-methyl)-morpholin-2-ylmethylcarbamoyl]-1-[cyclopropyl-(2-ethoxy-carbonyl-ethyl)-carbamoyl]-ethylaminosulfonyl]-benzoesäure, (1:1)-Epimerengemisch, MS (ISP): 597.5 $(M+H)^+$

b)2) 2-[(S)-2-[4-(Amino-imino-methyl)-morpholin-2-ylmethylcarbamoyl ]-1-[(2-carboxy-ethyl)-cyclopropyl-carbamoyl]-ethylaminosulfonyl]-benzoesäure, (1:1)-Epimerengemisch, MS (ISP): 569.4$(M+H)^+$.

Beispiel 47

Analog Beispiel 1 stellt man das (S)-N4-[(S)-1-(Amino-imino-methyl)-piperidin-3-ylmethyl]-N(1)-cyclopropyl-N(1)-[2-(5-methyl-1,2,4-oxadiazol-3-yl)-ethyl]-2-(naphthyl-2-ylsulfonylamino)-succinamid-hydrochlorid her, MS (ISP): 597.4 $(M+H)^+$

Herstellung des Ausgangsmaterials:

a) Zu einer Lösung von 29.2 g 3-Cyclopropylamino-propionitril in 300 ml Dioxan tropft man bei Raumtemperatur eine Lösung von 57.8 g Di-tert-butyldicarbonat in 300 ml Dioxan. Die Lösung wird über Nacht bei Raumtemperatur gerührt und dann eingedampft. Man erhält 58.1 g rohen (2-Cyano-ethyl)-cyclopropyl-carbaminsäure-tert-butylester, MS (FAB): 154 (M-isobutylen).

b) Zu einer Lösung von 20.0 g des unter a) erhaltenen Nitrils in 57 ml Ethanol und 23 ml Wasser gibt man 6.6 g Hydroxylamin-hydrochlorid und 13.6 g Natriumcarbonat-decahydrat. Das Reaktionsgemisch wird unter Rückfluss gekocht, eingedampft, der Rückstand in heissem Ethanol suspendiert und filtriert. Das Filtrat wird eingedampft und der Rückstand aus Isopropanol-Hexan umkristallisiert. Die erhaltenen Kristalle werden in 10 ml Acetanhydrid gelöst und bei 80° gerührt. Anschliessend wird das Reaktionsgemisch eingedampft, der Rückstand mit gesättigter Natriumcarbonat-Lösung versetzt und mit Essigester extrahiert. Die organischen Phasen werden eingedampft und der Rückstand mit Hexan-Essigester (3:1) an Kieselgel chromatografiert. Man erhält 5.4 g Cyclopropyl-2-(5-methyl-1,2,4-oxadiazol-3-yl)-ethyl-carbaminsäure-tert-butylester, MS (FAB): 211 (M-isobutylen).

c) 5.2 g des unter b) erhaltenen Materials werden in 30 ml Essigester gelöst, mit einer 4 molaren Salzsäurelösung in Essigester versetzt und bei Raumtemperatur gerührt. Die Lösung wird eingedampft, der Rückstand in Essigester aufgeschlämmt und abfiltriert. Man isoliert 3.7 g Cyclopropyl-2-(5-methyl-1,2,4-oxadiazol-3-yl)-ethyl-amin-hydrochlorid, MS (FAB): 167 $(M^+)$.

d) Analog dem Verfahren von Beispiel 2.B)e) jedoch unter Verwendung des unter c) erhaltenen Amin-hydrochlorids

anstelle von Sarcosinethylester Hydrochlorid erhält man (S)-3-tert-Butoxycarbonylamino-N-cyclopropyl-N-[2-(5-methyl-1,2,4-oxadiazol-3-yl)-ethyl]-succinamsäure-tert-butylester, MS (ISP): 439.6 $(M+H)^+$.

e) Analog dem Verfahren von Beispiel 2.B)i) jedoch unter Verwendung des unter d) erhaltenen Diesters anstelle von (S)-3-t-Butoxycarbonylamino-N-ethoxy-carbonylmethyl-N-methylsuccinamsäure-t-butylester stellt man (S)-N-Cyclopropyl-N-[2-(5-methyl-1,2,4-oxadiazol-3-yl)-ethyl]-3-(naphthalen-2-ylsulfonylamino)-succinamsäure-tert-butylester her, MS (FAB): 473 (M-isobutylen).

Beispiel 48

Analog Beispiel 1 erhält man aus (S)-N-Cyclopropyl-N-ethoxycarbonylmethyl-3-(naphthalen-2-sulfonylamino)-succinamsäure-t-butylester (Beispiel 2.B)d)1)) unter Verwendung von (S)-(3-Aminomethyl-piperidin-1-yl)-iminomethylcarbaminsäureethylester-hydrochlorid an Stelle von (S)-1-Amidino-3-(aminomethyl)piperidin-dihydrochlorid

den [Cyclopropyl-[(S)-3-[(S)-1-(ethoxycarbonyl-amino-imino-methyl)-piperidin-3-ylmethylcarbamoyl]-2-(naphthalen-2-ylsulfonylamino)-propionyl]-amino]-essigsäureethylester, MS (ISP): 659.6 $(M+H)^+$.

Herstellung des Ausgangsmaterials:

a) Zu einer Lösung von 10,0 g (S)-3-Aminomethyl-1-piperidincarbonsäure-t-butylester in 400 ml Hexan und 100 ml Wasser gibt man 3,7 g Tetrabutylammoniumhydrogensulfat und 100 ml 1N Natronlauge. Zu dieser Mischung tropft man 9,3 ml Benzylchloroformiat und rührt während 3 Stunden bei Raumtemperatur. Anschliessend wird die organische Phase abgetrennt, mit Wasser, 10%iger Zitronensäure, Wasser und gesättigter Natriumbicarbonatlösung gewaschen, getrocknet und eingedampft. Man erhält t-Butyl-(S)-3-[(1-benzyloxy)formamido]methyl]-1-piperidincarboxylat.

b) 11,3 g des unter a) erhaltenen Materials werden in 120 ml Essigester gelöst, bei 4° mit 120 ml einer 4 molaren Lösung von Salzsäure in Essigester versetzt und 5 Stunden bei Raumtemperatur gerührt. Anschliessend wird die Reaktionslösung eingeengt, der Rückstand in 265 ml DMF gelöst, mit 18 ml Triäthylamin und 4,3 g Formamidinsulfonsäure versetzt und 17 Stunden bei Raumtemperatur gerührt. Das Lösungsmittel wird eingedampft, der Rückstand mit IN Salzsäure versetzt, wiederum eingeengt und mit Wasser-Acetonitril an einer RP-18 Säule chromatographiert. Man isoliert so 5.4 g Benzyl-[[(S)-1-amidino-3-piperidinyl]methyl]carbamat-hydrochlorid.

c) Zu einer Lösung von 2.0 g Benzyl-[[(S)-1-amidino-3-piperidinyl]methylcarbamat-hydrochlorid in 200 ml Methylenchlorid tropft man 0.55 ml Chlorameisensäureethylester. Das Reaktionsgemisch wird auf 0° gekühlt. Unter Rühren werden 113 ml 0.1 N Natronlauge zugetropft. Anschliessend wird im Eisbad gerührt, die Phasen werden getrennt, die organische Phase mit Wasser gewaschen, getrocknet und eingedampft. Man erhält 1.5 g (S)-1-(Ethoxycarbonylamino-imino-methyl)-piperidin-3-ylmethylcarbamin-säurebenzylester, MS (FAB): 363.2 $(M+H)^+$.

d) 1.5 g des unter c) erhaltenen Materials werden in 30 ml Ethanol und 30 ml IN Salzsäure gelöst, mit 0.2 g Palladium auf Kohle versetzt und hydriert. Man erhält 1.4 g (S)-(3-Aminomethyl-piperidin-1-yl)-imino-methylcarbaminsäure ethylester-hydrochlorid, MS (ISP): 229.4 $(M+H)^+$.

Beispiel 49

Analog Beispiel 48 jedoch unter Verwendung von Chlorameisensäureisobutylester an Stelle von Chlorameisensäureäthylester (im Beispiel 48c) erhält man via (S)-(3-Benzyloxycarbonylaminomethyl-piperidin-1-yl)-imino-methylcarbaminsäureisobutylester, MS (FAB): 390 $M^+$ und via [(S)-3-Aminomethyl-piperidin-1-yl]-imino-methylcarbaminsäureisobutylesterhydrochlorid (1:1), MS (Thermospray): 257 $(M+H)^+$,

den [Cyclopropyl-[(S)-3-[(S)-1-(isobutoxycarbonylamino-imino-methyl)-piperidin-3-ylmethylcarbamoyl]-2-(naphthalen-2-ylsulfonylamino)-propionyl]-amino]-essigsäureethylester oder dem Amidinogruppe-Tautomer, MS (ISP) 687.7 $(M+H)^+$.

Beispiel 50

Analog Beispiel 48 jedoch unter Verwendung von (RS)-(2-Aminomethyl-morpholin-4-yl)-imino-methylcarbaminsäureethylesterhydrochlorid an Stelle von (S)-(3-Aminomethyl-piperidin-1-yl)-iminomethylcarbaminsäureethylester-hydrochlorid erhält man

den [Cyclopropyl-[(S)-3-[4-(ethoxycarbonylamino-imino-methyl)-morpholin-2-ylmethylcarbamoyl]-2-(naphthalen-2-ylsulfonylamino)-propionyl]-amino]-essigsäureethylester, (1:1)(1:1)-Epimerengemisch, MS (ISP): 661.5 $(M+H)^+$

Herstellung des Ausgangsmaterials

a) Zu einer Suspension von 10.3 g tert-Butyl rac-[(4-amidino-2-morpholinyl)methyl]carbamat hemisulfit (Beispiel 7Bb) in 1030 ml Methylenchlorid gibt man 3.15 ml Chlorameisensäureethylester. Man fühlt das Reaktionsgemisch auf 4 ° und tropft 637.2 ml 0.1 N Natronlauge zu. Anschliessend wird bei 5° gerührt, dann die organische Phase abgetrennt, mit Wasser gewaschen, getrocknet und eingedampft. Man isoliert 10.5 g (RS)-[2-(tert-Butoxycarbonylaminomethyl)-morpholin-4-yl]-imino-methylcarbaminsäureethylester, MS (ISP): 331.4 $(M+H)^+$.

b) 8.9 g des unter a) erhaltenen Materials wird in 50 ml Essigester gelöst, mit 50 ml 4 molarer Salzsäure in Essigester versetzt und bei Raumtemperatur gerührt. Nach Eindampfer der entstandenen Suspension erhält man 7.3 g (RS)-(2-Aminomethyl-morpholin-4-yl)-imino-methylcarbaminsäureethylester-hydrochlorid, MS (ISP): 231.4 $(M+H)^+$

Beispiel 51

Analog Beispiel 50 stellt man aus den entsprechenden Estern folgende Verbindungen her:

a) aus (S)-N-Cyclopropyl-N-(2-tetrazol-5-yl-ethyl)-3-naphthalin-2-ylsulfonylamino-succinamsäure-tert-butylester (Beispiel 36Ac)
den [2-[(S)-3-[Cyclopropyl-2-(tetrazol-5-yl)-ethyl-carbamoyl]-3-(naphthalen-2-ylsulfonylamino)-propionylaminomethyl]-morpholin-4-yl]-imino-methylcarbaminsäureethylester, (1:1)(1:1)-Epimerengemisch, MS (ISP): 671.6 $(M+H)^+$

b) aus N-[3-(t-Butoxycarbonyl)-N-(2-naphthylsulfonyl)-L-alanyl]-N-cyclopropyl-β-alanin-ethylester (Beispiel 2.B)j) 9))
das 3-[[(S)-3-[4-(Ethoxycarbonylamino-imino-methyl)-morpholin-2-ylmethylcarbamoyl]-2-(naphthalen-2-ylsulfonylamino)-propionyl]-cyclopropyl-amino]-propionsäureethylester oder der Amidinogruppe-Tautomer, (1:1)-Epimerengemisch, MS (ISN): 673.5 (M-H)-.

Beispiel 52

Analog Beispiel 48 erhält man aus (S)-N-Cyclopropyl-N-(2-tetrazol-5-yl-ethyl)-3-naphthalin-2-ylsulfonylamino-succinamsäure tert-butylester (Beispiel 36Ac) und den folgenden Aminomethyl-piperidin Derivaten die entsprechenden Produkte:

a) aus (S)-(3-Aminomethyl-piperidin-1-yl)-imino-methylcarbaminsäure ethylester Hydrochlorid (Beispiel 48d)
das [(S)-3-[(S)-3-[Cyclopropyl-2-(tetrazol-5-yl)-ethyl-carbamoyl]-3-(naphthalen-2-ylsulfonamino)-propionylaminomethyl]-piperidin-1-yl]-iminomethylcarbaminsäureethylester, MS (ISP): 669.6 $(M+H)^+$

b) aus [(S)-3-Aminomethyl-piperidin-1-yl]-imino-methylcarbaminsäureisobutylester-hydrochlorid (1:1) (Beispiel 49)
das (S)-N(1)-Cyclopropyl-N(4)-[(S)-1-isobutoxycarbonylamino-iminomethyl)-piperidin-3-ylmethyl]-2-(naphthalen-2-ylsulfonylamino)-N(1)-[2-(tetrazol-5-yl)-ethyl]-succinamid, MS (ISP): 697.5 $(M+H)^+$.

Beispiel 53

Analog Beispiel 48 erhält man unter Verwendung der entsprechenden Arylsulfonylestern anstelle von (S)-N-Cyclopropyl-N-ethoxycarbonylmethyl-3-(naphthalen-2-sulfonylamino)-succinamsäure t-butylester folgende Produkte:

a) aus N-[3-(t-Butoxycarbonyl)-N-(2-naphthylsulfonyl)-L-alanyl]-N-cyclopropyl-β-alanin-ethylester (Beispiel 2.B)j) 9)
den 3-[[(S)-3-[(S)-1-(Ethoxycarbonylamino-imino-methyl)-piperidin-3-ylmethylcarbamoyl]-2-(naphthalen-2-ylsulfonylamino)-propionyl]-cyclopropyl-amino]-propionsäureethylester oder das Amidinogruppe-Tautomere, MS (ISP): 673.5 $(M+H)^+$

b) aus (S)-2-[2-tert-Butoxycarbonyl-1-[cyclopropyl-(2-ethoxycarbonyl-ethyl)-carbamoyl]-ethylsulfamoyl]-benzoesäure methylester (Beispiel 38Bb)

den 3-[[(S)-3-[(S)-1-(Ethoxycarbonylamino-imino-methyl)-piperidin-3-ylmethylcarbamoyl]-2-(2-methoxycarbonyl-phenylsulfonylamino)-propionyl]-cyclopropyl-amino]-propionsäureethylester oder das Amidinogruppe-Tautomere, MS (ISP): 681.5 $(M+H)^+$.

Beispiel 54

Analog Beispiel 3 jedoch unter Verwendung der entsprechenden Ester erhält man:

54a) aus dem Ester von Beispiel 51b)
die 3-[[(S)-3-[4-(Ethoxycarbonylamino-imino-methyl)-morpholin-2-ylmethylcarbamoyl]-2-(naphthalen-2-ylsulfonylamino)-propionyl]-cyclopropyl-amino]-propionsäure oder das Amidinogruppe-Tautomere, (1:1)-Epimerengemisch, MS (ISN): 645.2 (M-H)-

54b) aus dem Ester von Beispiel 53b)

54b)1. die 2-[(S)-1-[(2-Carboxy-ethyl)-cyclopropyl-carbamoyl]-2-[(S)-1-(ethoxycarbonylamino-imino-methyl)-piperidin-3-ylmethylcarbamoyl]-ethyl-sulfamoyl]-benzoesäure oder das Amidinogruppe-Tautomere, MS (ISP) 639.5 $(M+H)^+$ und

54b)2. die 2-[(S)-1-[(2-Ethoxycarbonyl-ethyl)-cyclopropyl-carbamoyl]-2-[(S)-1-(ethoxycarbonylamino-imino-methyl)-piperidin-3-ylmethylcarbamoyl]-ethylsulfamoyl]-benzoesäure oder das Amidinogruppe-Tautomere, MS (ISP) 667.6 $(M+H)^+$.

Beispiel 55

Analog Beispiel 29 wird via

a) Benzyloxycarbonylamino-essigsäurecyclopropylamid, MS (EI): 248 (M)

b) 2-Cyclopropylamino-ethylcarbaminsäurebenzylester-hydrochlorid (1:1), MS (EI): 234 (M)

c) (S)-3-[(S)-3-[(2-Amino-ethyl)-cyclopropyl-carbamoyl]-3-(naphthalen-2-ylsulfonylamino)-propionylaminomethyl]-piperidin-1-carbonsäure-tertbutylester-hydrochlorid (1:1) und

d) (S)-3-[(S)-3-[Cyclopropyl-(2-pyrazin-2-ylcarbonylamino-ethyl)-carbamoyl]-3-(naphthalen-2-ylsulfonylamino)-propionylaminomethyl]-piperidin-1-carbonsäure-tert-butylester, MS (ISP): 708.8 (M+H)
das (S)-N4-[(S)-1-(Amino-imino-methyl)-piperidin-3-ylmethyl]-N1-cyclopropyl-2-(naphthalen-2-ylsulfonylamino)-N1-[2-(pyrazin-2-ylcarbonylamino)-ethyl]-succinamid-acetat (1:3), MS (ISP): 650.7 (M+H) hergestellt.

Herstellung des Ausgangsmaterials

a) Zu 24 g N-Benzyloxycarbonyl-glycin und 8 ml Cyclopropylamin in 240 ml Methylenchlorid, gibt man unter Rühren 23.1 g N-(3-Dimethylaminopropyl)-N'-ethylcarbodiimid-hydrochlorid (EDC) und rührt 4 Stunden bei Raumtemperatur. Das Gemisch wird in Aether aufgenommen, mit 1N Salzsäure, mit Bicarbonatlösung und mit Wasser gewaschen. Nach Trocknen und Eindampfen der Aetherphase erhält man 23 g Benzyloxycarbonylamino-essigsäurecyclopropylamid.

b) Zu 23 g des Produkts von a) in 250 ml THF tropft man bei 10-23° 17.6 ml Boran-Dimethylsulfid. Das Gemisch wird unter Rückfluss erhitzt, dann auf -10° abgekühlt. Unter Eiskühlung tropft man 75 ml 2N Salzsäure zu und nimmt das Gemisch bei Raumtemperatur in Aether auf. Die Aetherphase wäscht man mit Wasser. Die wässrigen Phasen stellt man mit 90 ml 2N Natronlauge wieder basisch und extrahiert mit Aether. Die Aetherphasen wäscht man mit Wasser, stellt sie dann mit 2N Salzsäure (in Dioxan) sauer (pH 2) und engt sie ein. Den Rückstand schlämmt man in Aether auf und filtriert. Man erhält 11 g 2-Cyclopropylamino-ethylcarbaminsäurebenzylester-hydrochlorid.

c) Analog Beispiel 29b)c)d)e) erhält man das (S)-3-[(S)-3-[(2-Amino-ethyl)-cyclopropyl-carbamoyl]-3-(naphthalen-2-ylsulfonylamino)-propionylaminomethyl]-piperidin-1-carbonsäure-tert-butylester-hydrochlorid.

d) Zu 400 mg des Amin-hydrochlorids von c) und 0.11 ml Hünig's Base in 4 ml Methylenchlorid gibt man 94 mg Pyrazincarbonsäure und 127 mg EDC. Das Gemisch rührt man 20 Stunden bei Raumtemperatur und nimmt es dann in Essigester auf. Die Essigesterphase wäscht man mit Wasser. Nach Trocknen und Eindampfen chromatographiert man das Rohprodukt über Kieselgel mit Essigester/Methanol (9:1). Man erhält 288 mg (S)-3-[(S)-3-[Cyclopropyl-(2-pyrazin-2-ylcarbonylamino-ethyl)-carbamoyl]-3-(naphthalen2-ylsulfonylamino)-propionylaminomethyl]-piperidin-1-carbonsäure-tert-butylester.

Beispiel 56

Analog Beispiel 55 jedoch unter Verwendung der entsprechenden Säuren oder Säurederivaten an Stelle der im Beispiel 55d) verwendeten Pyrazincarbonsäure, nämlich unter Verwendung von a) Oxalsäuremonomethylester-chlorid, b) dem $SO_3$, $N(CH_3)_3$-Komplex, c) Benzylchloroformiat, d) Chloressigsäure, e) Phenoxyessigsäurechlorid, f) Phenylglyoxylsäure, g) Brenztraubensäure, h) Nicotinsäure, i) Nicotinsäure-N-oxyd bzw. j) 3,4-Dihydroxyphenylessigsäure, erhält man folgende Verbindungen:

a) N-[2-[[(S)-3-[(S)-1-(Amino-imino-methyl)-piperidin-3-ylmethylcarbamoyl]-2-(naphthalen-2-ylsulfonylamino)-propionyl]-cyclopropyl-amino]-ethyl[-oxamsäuremethylester-acetat (1:1), MS (ISP): 630.5 (M+H)

b) 2-[[(S)-3-[(S)-1-(Amino-imino-methyl)-piperidin-3-ylmethylcarbamoyl]-2-(naphthalen-2-ylsulfonylamino)-propionyl]-cyclopropyl-amino]-ethylsulfaminsäure, MS (ISP): 624.5 (M+H)

c) 2-[[(S)-3-[(S)-1-(Amino-imino-methyl)-piperidin-3-ylmethylcarbamoyl]-2-(naphthalen-2-ylsulfonylamino)-propionyl]-cyclopropyl-amino]-ethylcarbaminsäurebenzylester-acetat (1:1), MS (ISP): 678.5 (M+H)

d) (S)-N4-[(S)-1-(Amino-imino-methyl)-piperidin-3-ylmethyl]-N1-(2-chloroacetylamino-ethyl)-N1-cyclopropyl-2-(naphthalen-2-ylsulfonylamino)-succinamid-acetat (1:1), MS (ISP): 620.4 (M+H)

e) (S)-N4-[(S)-1-(Amino-imino-methyl)-piperidin-3-ylmethyl]-N1-cyclopropyl-2-(naphthalen-2-ylsulfonylamino)-N1-(2-phenoxyacetylamino-ethyl)-succinamid-acetat (1:1), MS (ISP): 678.6 (M+H)

f) (S)-N4-[(S)-1-(Amino-imino-methyl)-piperidin-3-ylmethyl]-N1-cyclopropyl-2-(naphthalen-2-ylsulfonylamino)-N1-[2-(2-oxo-2-phenyl-acetylamino)-ethyl]-succinamid-acetat (1:2), MS (ISP): 676.6 (M+H)

g) (S)-N4-[(S)-1-(Amino-imino-methyl)-piperidin-3-ylmethyl]-N1-cyclopropyl-2-(naphthalen-2-ylsulfonylamino)-N1-[2-(2-oxo-propionylamino)-ethyl]-succinamid-acetat (1:1), MS (ISP): 614.6 (M+H)

h) (S)-N4-[(S)-1-(Amino-imino-methyl)-piperidin-3-ylmethyl]-N1-cyclopropyl-2-(naphthalen-2-ylsulfonylamino)-N1-[2-(pyridin-3-ylcarbonylamino)-ethyl]-succinamid-acetat (1:2), MS (ISP): 649.1 (M+H)

i) (S)-N4-[(S)-1-(Amino-imino-methyl)-piperidin-3-ylmethyl]-N1-cyclopropyl-2-naphthalen-2-ylsulfonylamino-N1-[2-(1-oxy-nicotinoylamino)-ethyl]-succinamid-acetat (1:1), MS (ISP): 666.5 (M+H)

j) (S)-N4-[(S)-1-(Amino-imino-methyl)-piperidin-3-ylmethyl]-N1-cyclopropyl-N1-[2-(3,4-dihydroxy-phenylacetylamino)-ethyl]-2-(naphthalen-2-ylsulfonylamino)-succinamid-acetat (1:1), MS (ISP): 694.6 (M+H).

Beispiel 57

Analog Beispiel 3 erhält man aus dem Ester von Beispiel 56a) folgende Säure:

N-[2-[[(S)-3-[(S)-1-(Amino-imino-methyl)-piperidin-3-ylmethylcarbamoyl]-2-(naphthalen-2-ylsulfonylamino)-propionyl]-cyclopropyl-amino]-ethyl]-oxamsäure, MS (ISP): 616.5 (M+H).

Beispiel 58

Analog Beispiel 29 und 55, jedoch unter Verwendung von [(S)-3-Aminomethyl-piperidin-1-yl]-imino-methylcarbaminsäuremethylester-hydrochlorid (1:2) an Stelle von (S)-3-Aminomethyl-1-piperidincarbonsäure-t-butylester (Beispiel 29d) erhält man

den [(S)-3-[(S)-3-[(2-Chloroacetylamino-ethyl)-cyclopropyl-carbamoyl]-2-(naphthalen-2-ylsulfonylamino)-propionylaminomethyl]-piperidin-1-yl]-imino-methylcarbaminsäuremethylester, MS (ISP): 648.4 (M+H).

Herstellung des Ausgangsmaterials

Analog Beispiel 48 jedoch unter Verwendung von Chlorameisensäuremethylester an Stelle von Chlorameisensäureäthylester (Beispiel 48c) erhält man das [(S)-3-Aminomethyl-piperidin-1-yl]-imino-methylcarbaminsäuremethylester-hydrochlorid (1:2), MS (Thermospray): 215 (M+H).

Beispiel 59

Analog Beispiel 58 werden folgende Verbindungen hergestellt:

a) N-[2-[Cyclopropyl-[(S)-3-[(S)-1-(imino-methoxycarbonylamino-methyl)-piperidin-3-ylmethylcarbamoyl]-2-(naphthalen-2-ylsulfonylamino)-propionyl]-amino]-ethyl]-oxamsäuremethylester, MS (ISP): 702.6 (M+H)

b) 2-[Cyclopropyl-[[(S)-3-[(S)-1-(methoxycarbonylamino-imino-methyl)-piperidin-3-ylmethylcarbamoyl]-2-(naphthalen-2-ylsulfonylamino)-propionyl]-amino]-ethylcarbaminsäurebenzylester, MS (ISP): 736.7 (M+H)

c) [(S)-3-[(S)-3-[Cyclopropyl-(2-methylsulfonylamino-ethyl)-carbamoyl]-3-(naphthalen-2-ylsulfonylamino)-propionylaminomethyl]-piperidin-1-yl]-imino-methylcarbaminsäure-methylester, MS (ISP): 680.5 (M+H).

Beispiel 60

Analog Beispiel 29 jedoch unter Verwendung von N-Cyclopropylglycinäthylester an Stelle von 2-Butyl-aminoäthylazid-hydrochlorid (in Beispiel 29b) erhält man via

[[(S)-3-[(S)-1-tert-Butoxycarbonyl-piperidin-3-ylmethylcarbamoyl]-2-(naphthalen-2-sulfonylamino)-propionyl]-cyclopropyl-amino]-essigsäure und

(S)-3-[(S)-3-(Cyclopropyl-methoxycarbamoylmethyl-carbamoyl)-3-(naphthalen-2-ylsulfonylamino)-propionylaminomethyl]-piperidin-1-carbonsäure-tert-butylester,
das (S)-N4-[(S)-1-(Amino-imino-methyl)-piperidin-3-ylmethyl]-N1-cyclopropyl-N1-methoxycarbamoylmethyl-2-(naphthalen-2-ylsulfonylamino)-succinamid-acetat (1:1), MS (ISP): 588.6 (M+H).

Herstellung des Ausgangsmaterials:

500 mg [[(S)-3-[(S)-1-tert-Butoxycarbonyl-piperidin-3-ylmethylcarbamoyl]-2-(naphthalen-2-sulfonylamino)-propionyl]-cyclopropyl-amino]-essigsäure werden zusammen mit 71 mg O-Methylhydroxylamin-hydrochlorid, 0.28 ml N-Methylmorpholin und 376 mg BOP in 10 ml Methylenchlorid 20 Stunden bei Raumtemperatur gerührt. Das Gemisch nimm man in Essigester auf, wäscht mit 1N Salzsäure und dann mit Wasser. Nach Trocknen und Eindampfen, reinigt man das Produkt über Kieselgel mit Essigester/Methanol (9:1). Man erhält 282 mg (S)-3-[(S)-3-([Cyclopropylmethoxycarbamoylmethyl-carbamoyl)]-3-(naphthalen-2-ylsulfonylamino)-propionylaminomethyl]-piperidin-1-carbonsäure-tert-butylester, MS (ISP): 646.6 (M+H).

Beispiel 61

Analog Beispiel 29 und 60 erhält man folgende Verbindungen:

a) (S)-N4-[(S)-1-(Amino-imino-methyl)-piperidin-3-ylmethyl]-N1-benzyloxycarbamoylmethyl-N1-cyclopropyl-2-(naphthalen-2-ylsulfonylamino)-succinamid-acetat (1:1), MS (ISP): 664.5 (M+H)

b) (S)-N4-[(S)-1-(Amino-imino-methyl)-piperidin-3-yl-methyl]-N1-cyclopropyl-N1-methylsulfonylcarbamoylmethyl-2-(naphthalen-2-ylsulfonylamino)-succinamid-acetat (1:1), MS (ISP): 636.5 (M+H)

c) (S)-N4-[(S)-1-(Amino-imino-methyl)-piperidin-3-ylmethyl]-N1-cyclo-propyl-N1-cyclopropylcarbamoylmethyl-2-(naphthalen-2-ylsulfonylamino)succinamid-acetat (1:1), MS (ISP): 598.6 (M+H)

d) (S)-N4-[(S)-1-(Amino-imino-methyl)-piperidin-3-ylmethyl]-N1-cyclopropyl-2-(naphthalen-2-ylsulfonylamino)-N1-(pyridin-3-ylmethylcarbamoylmethyl)-succinamid-acetat (1:2), MS (ISP): 649.5 (M+H)

e) (S)-N4-[(S)-1-(Amino-imino-methyl)-piperidin-3-ylmethyl]-N1-cyclopropyl-N1-[2-(3,4-dihydroxy-phenyl)-ethylcarbamoylmethyl]-2-(naphthalen-2-ylsulfonylamino)-succinamid-acetat (1:2), MS (ISP): 694.5 (M+H)

f) (S)-N4-[(S)-1-(Amino-imino-methyl)-piperidin-3-yl]-N1-cyclopropyl-N1-(2-hydroxy-ethylcarbamoylmethyl)-2-(naphthalen-2-ylsulfonylamino)-succinamid-acetat (1:1), MS (ISP): 602.2 (M+H).

Beispiel 62

2,0 g (S)-N-(3-Benzyloxy-propyl)-N-cyclopropyl-3-naphthalen-2-ylsulfonylamino-succinaminsäure und 1,35 g [(S)-3-Aminomethyl-piperidin-1-yl]-imino-methylcarbaminsäuremethylester-hydrochlorid (1:2) (Beispiel 58) rührt man bei Raumtemperatur zusammen mit 1,91 g BOP und 2,34 ml 1,8-Diazabicyclo(5.4.0)undec-7-en (DBU) in 20 ml Methylenchlorid. Nach Eindampfen und Chromatographie über Kieselgel mit Essigester/Methanol (19:1) erhält man 2,35 g (S)-3-[(S)-3-[(3-Benzyloxy-propyl)-cyclopropylcarbamoyl]-3-(naphthalen-2-ylsulfonylamino)-propionylaminomethyl]-piperidin-1-yl]-imino-methylcarbaminsäuremethylester.

Herstellung des Ausgangsmaterials:

a) 8.58 g N-BOC-Cyclopropylamin und 15 g O-Benzyl-3-brom-1-propanol in 90 ml DMF versetzt man bei 0-5° mit 2,5 g Natriumhydrid (55% in Oel). Man rührt das Gemisch 1 Stunde bei 0-5° und 3 Stunden bei Raumtemperatur und versetzt es dann bei 0-5° mit wässriger Ammoniumchlorid-Lösung. Das Gemisch wird in Aether/Wasser verteilt, die Aetherphasen werden mit Wasser gewaschen, dann getrocknet und eingeengt. Nach Chromatographie über Kieselgel mit Aether/Hexan (1:4) werden die 11,5 g Produkt mit 120 ml 4,8-molarer Salzsäure in Dioxan gerührt. Nach Einengen kristallisiert man den Rückstand in Aether, filtriert die Kristalle und wäscht sie mit Aether. Man erhält 9,0 g (3-Benzyloxy-propyl)-cyclopropyl-amin-hydrochlorid, MS (EI): 206 (M+H).

b) 9,0 g Produkt von a) und 11,77 g N-(2-Naphthylsulfonyl)-L-asparaginsäure-4-t-butylester (Beispiel 23a) rührt man zusammen mit 14,4 g BOP und 15,9 ml Hünig's Base in 200 ml Methylenchlorid. Das Gemisch nimmt man in Aether auf, wäscht die Aetherphase mit 1N Salzsäure und dann mit Wasser. Nach Trocknen und Eindampfen der Aetherphase chromatographiert man den Rückstand über Kieselgel mit Essigester/Hexan (1:2). Man erhält 14,85 g (S)-N-(3-Benzyloxy-propyl)-N-cyclopropyl-3-naphthalen-2-ylsulfonylamino-succinamsäure-tert-butylester, MS (ISN): 565.8 (M-H).

c) 14,85 g des Produkts von b) löst man in 60 ml Dioxan und versetzt diese Lösung mit 120 ml 4,8-molarer Salzsäure in Dioxan. Das Gemisch rührt man bei Raumtemperatur, nimmt es in Aether auf und wäscht es mit Wasser. Nach Trocknen und Eindampfen der Aetherphase erhält man 12,87 g (S)-N-(3-Benzyloxy-propyl)-N-cyclopropyl-3-naphthalen-2-ylsulfonylamino-succinaminsäure, MS (ISN): 509.2 (M-H).

Beispiel 63

970 mg (S)-3-[(S)-3-[(3-Benzyloxy-propyl)-cyclopropyl-carbamoyl]-3-(naphthalen-2-ylsulfonylamino)-propionylaminomethyl]-piperidin-1-yl]-imino-methylcarbaminsäuremethylester löst man in 5 ml Methylenchlorid und versetzt die Lösung mit 5 ml einer 0,5-molaren Bortribromid-Lösung in Methylenchlorid. Nach 1,5 Stunden Rühren bei Raumtemperatur versetzt man das Gemisch mit 20 ml gesättigter Natriumbicarbonatlösung. Das Gemisch verteilt man zwischen Essigester und Wasser. Nach Trocknen und Eindampfen reinigt man das Rohprodukt über Kieselgel mit Essigester/Methanol 9:1. Man erhält 465 mg reines [(S)-3-[(S)-3-[Cyclopropyl-(3-hydroxy-propyl)-carbamoyl]-2-(naphthalen-2-ylsulfonylamino)-propionylaminomethyl]-piperidin-1-yl]-imino-methylcarbaminsäuremethylester, MS (ISP): 617.7 (M+H).

Beispiel 64

274 mg [(S)-3-[(S)-3-[Cyclopropyl-(3-hydroxy-propyl)-carbamoyl]-2-(naphthalen-2-ylsulfonylamino)-propionylaminomethyl]-piperidin-1-yl]-imino-methylcarbaminsäuremethylester (Beispiel 63), 1 ml 1N Methyljodid in THF, 2 ml 1-molare DBU-Lösung in THF und 2 ml Methylenchlorid werden zusammen bei Raumtemperatur gerührt. Das Gemisch engt man ein und chromatographiert den Rückstand über Kieselgel mit Essigester-Methanol 9:1. Man erhält 120 mg (S)-N1-Cyclopropyl-N4-[(S)-1-(iminomethoxycarbonylamino-methyl)-piperidin-3-ylmethyl]-N1-(3-methoxypropyl)-2-(naphthalen-2-ylsulfonylamino)-succinamid, MS (ISO): 631.6 (M+H).

Beispiel 65

Analog Beispiel 29 jedoch unter Verwendung von a) Phosphorsäurediphenylesterchlorid bzw. b) Phosphorsäurediäthylesterchlorid an Stelle von Oxalsäure-monoäthylesterchlorid (Beispiel 29f) erhält man

a) 2-[[(S)-3-[(S)-1-(Amino-imino-methyl)-piperidin-3-ylmethylcarbamoyl]-2-(naphthalen-2-sulfonylamino)-propionyl]-butylamino]-ethylamidophosphorsäurediphenylester-acetat (1:1), MS (ISP): 792.4 (M+H),

b) (S)-N4-[(S)-1-(Amino-imino-methyl)-piperidin-3-yl]-N1-butyl-N1-(2-diethoxyphosphorylamino-ethyl)-2-(naphthalen-2-sulfonylamino)-succinamid-acetat (1:1), MS (ISP): 696.2 (M+H).

Beispiel 66

Analog Beispiel 48 jedoch unter Verwendung von Di-t-butyl-dicarbonat an Stelle von Chlorameisensäureäthylester (in Beispiel 48c) erhält man via

(S)-(3-Benzyloxycarbonylaminomethyl-piperidin-1-yl)-imino-methylcarbaminsäure-tert-butylester, MS (Thermospray): 391 $(M+H)^+$, und via

(S)-(3-Aminomethyl-piperidin-1-yl)-imino-methylcarbaminsäure-tert-butylester, MS (ISP): 257.2 $(M+H)^+$

den [[(S)-3-[(S)-1-(tert-Butoxycarbonylamino-imino-methyl)-piperidin-3-ylmethylcarbamoyl]-2-(naphthalen-2-ylsulfonylamino)-propionyl]-cyclo-propyl-amino]-essigsäureethylester, MS (ISP): 687.5 $(M+H)^+$.

Beispiel 67

Analog Beispiel 1 jedoch unter Verwendung von [(4-Aminomethylpiperidin-1-yl)-imino-methyl]amin-dihydrochlorid anstelle von (S)-1-Amidino-3-(aminomethyl)piperidin-dihydrochlorid stellt man das (S)-3-[[3-[1-(Amino-imino-methyl)-piperidin-4-ylmethylcarbamoyl]-2-(naphthalen-2-ylsulfonylamino)-propionyl]-cyclohexyl-amino]-essigsäure-ethylester-hydrochlorid her, MS (ISP): 629.6 $(M+H)^+$.

Herstellung des Ausgangsmaterials:

a) Eine Lösung von 130 g (4-Piperidinylmethyl)-carbaminsäure-1,1-dimethylethylester in 1300 ml DMF wird mit 138 ml Triethylamin und 61.8 g Formamidinsulfinsäure versetzt und über Nacht bei Raumtemperatur gerührt. Das ausgefallene Material wird abfiltriert, in 500 ml Ethanol-aufgeschlämmt, wieder filtriert und getrocknet. Man erhält 65.6 g 1-(Amino-imino-methyl)-piperidin-4-ylmethylcarbaminsäure-tert-butylesterhemisulfit, MS (ISP): 257.4 $(M+H)^+$.

b) 65.6 g des unter a) erhaltenen Materials werden in 656 ml 1N Salzsäure gelöst und während 5 Stunden bei 50° gerührt. Das Lösungsmittel wird eingedampft, der Rückstand in 500 ml Ethanol aufgeschlämmt, genutscht und getrocknet. Man erhält 48.5 g [(4-Aminomethyl-piperidin-1-yl)-iminomethyl]-amin-dihydrochlorid, MS (ISP): 573.5 $(M+H)^+$.

Beispiel 68

Analog Beispiel 67 stellt man aus den entsprechenden tert-Butylestern die folgenden Produkte her:

68a) [[(S)-3-[1-Amino-imino-methyl)-piperidin-4-ylmethylcarbamoyl]-2-(naphthalen-2-ylsulfonylamino)-propionyl]-cyclopropyl-amino]-essigsäureethylester-trifluoracetat, MS (ISP): 587.8 $(M+H)^+$

68b) [[(S)-3-[1-(Amino-imino-methyl)-piperidin-4-ylmethylcarbamoyl]-2-(naphthalen-2-ylsulfonylamino)-propionyl]-benzyl-amino]-essigsäureethylester-hydrochlorid (1:1), MS (ISP): 637.4 $(M+H)^+$

68c) [[(S)-3-[1-(Amino-imino-methyl)-piperidin-4-ylmethylcarbamoyl]-2-(naphthalen-2-ylsulfonylamino)-propionyl]-cyclohexylmethyl-amino]-essigsäureethylester-hydrochlorid, MS (ISP): 643.6 $(M+H)^+$

68d) [[(S)-3-[1-(Amino-imino-methyl)-piperidin-4-ylmethylcarbamoyl]-2-(naphthalen-2-ylsulfonylamino)-propionyl]-butyl-amino]-essigsäureethylester-hydrochlorid (1:1), MS (ISP): 603.4 $(M+H)^+$

68e) (S)-3-[[3- [ 1-(Amino-imino-methyl)-piperidin-4-ylmethylcarbamoyl]-2-(naphthalen-2-ylsulfonylamino)-propionyl]-cyclopropyl-amino]-propionsäureethylester-hydrochlorid, MS (ISP): 601.6 $(M+H)^+$

68f) (S)-3-[[3-[1-(Amino-imino-methyl)-piperidin-4-ylmethylcarbamoyl]-2-(naphthalen-2-ylsulfonylamino)-propionyl]-benzyl-amino]-propionsäureethylester-hexafluorophosphat (1:1), MS (ISP): 651.6 $(M+H)^+$

68g) (S)-3-[[3-[1-(Amino-imino-methyl)-piperidin-4-ylmethylcarbamoyl]-2-(naphthalen-2-ylsulfonylamino)-propionyl]-cyclohexylmethyl-amino]-propionsäureethylester-hexafluorophosphat (1:1), MS (ISP): 657.5 $(M+H)^+$

68h) (S)-N4-[1-(Amino-imino-methyl)-piperidin-4-ylmethyl]-N1-cyclopropyl-2-(naphthalen-2-ylsulfonylamino)-N1-(3-oxo-butyl)-succinamid-hydrochlorid, MS (ISP): 571.6 $(M+H)^+$.

Beispiel 69

Analog Beispiel 3 stellt man aus den Estern von Beispiel 67 und 68 die folgenden Säuren her:

a) (S)-3-[[3-[1-(Amino-imino-methyl)-piperidin-4-ylmethylcarbamoyl]-2-(naphthalen-2-ylsulfonylamino)-propionyl]-cyclohexyl-amino]-essigsäurehydrochlorid, MS (ISP): 601.6 $(M+H)^+$

b) (S)-[[3-[1-(Amino-imino-methyl)-piperidin-4-ylmethylcarbamoyl]-2-(naphthalen-2-ylsulfonylamino)-propionyl]-cyclopropyl-amino]-essigsäure, MS (ISP): 559.6 $(M+H)^+$

c) [[(S)-3-[1-(Amino-imino-methyl)-piperidin-4-ylmethylcarbamoyl]-2-(naphthalen-2-ylsulfonylamino)-propionyl]-benzyl-amino]essigsäure, MS (ISP): 609.5 $(M+H)^+$

d) (S)-[[3-[1-(Aimno-imino-methyl)-piperidin-4-ylmethylcarbamoyl]-2-(naphthalen-2-ylsulfonylamino)-propionyl]-cyclohexylmethyl-amino]-essigsäure, MS (ISP): 615.5 $(M+H)^+$

e) [[(S)-3-[1-(Amino-imino-methyl)-piperidin-4-ylmethylcarbamoyl]-2-(naphthalen-2-ylsulfonylamino)-propionyl]-butyl-amino]-essigsäure, MS (ISP): 575.5 $(M+H)^+$

f) (S)-3-[[3-[1-(Aimno-imino-methyl)-piperidin-4-ylmethylcarbamoyl]-2-(naphthalen-2-ylsulfonylamino)-propionyl]-cyclopropyl-amino]-propionsäure-hydrochlorid, MS (ISP): 573.5 $(M+H)^+$

g) (S)-3-[[3-[1-(Amino-imino-methyl)-piperidin-4-ylmethylcarbamoyl]-2-(naphthalen-2-ylsulfonylamino)-propionyl]-benzyl-amino]-propionsäure, MS (ISP): 623.6 $(M+H)^+$

h) (S)-3-[[3-[1-(Amino-imino-methyl)-piperidin-4-ylmethylcarbamoyl]-2-(naphthalen-2-ylsulfonylamino)-propionyl]-cyclohexylmethyl-amino]-propionsäure, MS (ISP): 629.5.

Beispiel 70

1.0 g des N-[N4-[[(S)-1-Amidino-3-piperidinyl]methyl]-N2-(2-naphthylsulfonyl)-L-asparaginyl]-N-cyclopropylglycin (Beispiel 4a) wird in 10 ml DMF gelöst, mit 0,2 ml Morpholin, 0.8 g BOP und 1.1 ml 4-Ethylmorpholin versetzt und über Nacht bei Raumtemperatur gerührt. Das Reaktionsgemisch wird mit 20 ml 1N Salzsäure versetzt, eingedampft und der Rückstand mit einem Wasser-Acetonitril-Gradienten an einer RP-18-Säule chromatographiert. Man erhält 0.5 g (S)-N4-[(S)-1-(Amino-imino-methyl)-piperidin-3-ylmethyl]-N1-cyclopropyl-N1-morpholin-4-ylcarbonylmethyl-2-(naphthalen-2-ylsulfonylamino)-succinamid-hydrochlorid (1:1), MS (ISP): 628.5 $(M+H)^+$.

Beispiel 71

Eine Lösung von 0.8 des 2-[[(S)-3-[(S)-1-(Amino-imino-methyl)-piperidin-3-ylmethylcarbamoyl]-2-(naphthalen-2-ylsulfonylamino)-propionyl]-cyclopropyl-amino]-ethylcarbaminsäurebenzylesters (Beispiel 56c) in 20 ml Methanol wird nach Zugabe von 0.2 g Palladium auf Kohle während 24 Stunden bei Raumtemperatur hydriert. Der Katalysator wird abfiltriert, das Filtrat eingedampft und der Rückstand getrocknet. 0.57 g des so erhaltenen Materials in 30 ml THF werden bei 0° zu einer Lösung von 0.57 g 3,4-Bis(2-propenyloxy)-3-cyclobuten-1,2-dion in 20 ml THF zugetropft und das Reaktionsgemisch 5 Stunden bei Raumtemperatur gerührt. Das Lösungsmittel wird eingedampft und der Rückstand mit Essigester-Aceton-Essigsäure-Wasser (6:2:1:1) auf Kieselgel chromatographiert. Die Produktfraktionen werden eingedampft und man erhält nach Trocknen des Rückstandes 0.6 g (S)-N1-[2-[2-Allyloxy-3,4-dioxo-cyclobut-1-enylamino)-ethyl]-N4-[(S)-1-(amino-imino-methyl)-piperidin-3-ylmethyl]-N1-cyclopropyl-2-(naphthalen-2-ylsulfonylamino)-succinamid-acetat (1:1), MS (ISP): 680.6 $(M+H)^+$.

Beispiel 72

0.1 g des unter Beispiel 71 erhaltenen Materials werden in 10 ml Acetonitril unter Zugabe von 1 Tropfen Wasser gelöst und diese Lösung mit 0.03 g Palladium(II)-acetat und 0.08 ml Triethylphosphit versetzt. Anschliessend werden 0.13 ml 2N Natrium-2-ethylcapronat in Wasser zugegeben und das Reaktionsgemisch wird bei Raumtemperatur 1.5 Stunden gerührt. Das ausgefallene Material wird abfiltriert, mit Ether-Hexan gewaschen und der Filterrückstandgetrocknet. Man isoliert 0.090 g (S)-N4-[(S)-1-(Amino-iminomethyl)-piperidin-3-ylmethyl]-N1-cyclopropyl-N1-[2-(3,4-dioxo-2-hydroxycyclobut-1-enylamino)-ethyl]-2-(naphthalen-2-ylsulfonylamino)-succinamidacetat (1:1), MS (ISP): 640.5 $(M+H)^+$.

Beispiel 73

Analog Beispiel 3 werden folgende Säuren aus den entsprechenden Estern hergestellt:

a) aus dem Ester von Beispiel 49 die Cyclopropyl-[(S)-3-[(S)-1-(isobutoxycarbonylamino-imino-methyl)-piperidin-3-ylmethylcarbamoyl]-2-(naphthalen-2-ylsulfonylamino)-propionyl]-amino]-essigsäure oder das Amidinogruppe-Tautomere, MS (ISP): 659.5 $(M+H)^+$

b) aus dem Ester von Beispiel 66 das [[(S)-3-[(S)-1-(Imino-tert-butoxycarbonylamino-methyl)-piperidin-3-ylmethyl-carbamoyl]-2-(naphthalen-2-ylsulfonylamino)-propionyl]-cyclopropyl-amino]-essigsäure oder das Amidinogruppe-Tautomere, MS (ISP): 659.7 $(M+H)^+$.

Eine Verbindung der Formel I, ein Solvat oder Salz davon kann man in an sich bekannter Weise als Wirkstoff zur Herstellung von pharmazeutischen Präparaten, z.B. von Tabletten und Kapseln folgender Zusammen) setzung, verwenden:

| Beispiel A | |
|---|---|
| | pro Tablette |
| Wirkstoff | 200 mg |
| mikrokristalline Cellulose | 155 mg |
| Maisstärke | 25 mg |
| Talk | 25 mg |
| Hydroxypropylmethylcellulose | 20 mg |
| | 425 mg |

| Beispiel B | |
|---|---|
| | pro Kapsel |
| Wirkstoff | 100,0 mg |
| Maisstärke | 20,0 mg |
| Milchzucker | 95,0 mg |
| Talk | 4,5 mg |
| Magnesiumstearat | 0.5 mg |
| | 220,0 mg |

## Patentansprüche

1. Sulfonamidocarboxamide der Formel

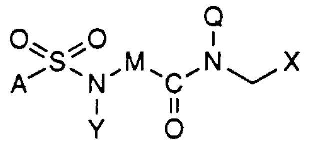 I

worin

X eine Gruppe der Formel $X^1$ oder $X^2$:

($X^1$) 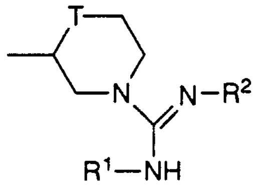 ($X^2$) 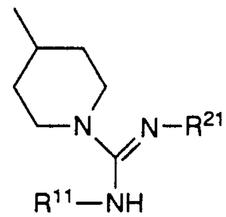

| | |
|---|---|
| T | $CH_2$ oder O, |
| $R^1$, $R^2$, $R^{11}$ und $R^{21}$ | unabhängig voneinander H oder COO-nieder-Alkyl, |
| Y | H oder, falls X eine Gruppe $X^2$ ist oder falls X eine Gruppe $X^1$ ist, in der zumindest eins von $R^1$ und $R^2$ nicht H ist, dann Y auch $CH_2COOH$ oder $SO_2$-A' sein kann, |
| A und A' | Aryl, Heteroaryl, Heterocyclyl, Alkyl oder Cycloalkyl, |
| Q | H, nieder-Alkyl oder nieder-Alkyl(OH, COOH oder COO-niederalkyl), |
| M | eine Gruppe der Formel $M^1$ ist oder, falls X eine Gruppe $X^2$ ist, oder falls X eine Gruppe $X^1$ ist und zumindest eins von $R^1$, $R^2$ und Q nicht H ist, und/oder falls A Alkyl oder Cycloalkyl ist, dann M auch eine Gruppe einer der Formeln $M^2$ bis $M^8$ sein kann, |

($M^1$) 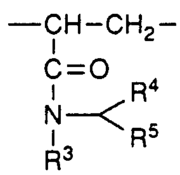 ($M^2$) 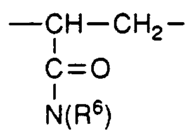

$-CH_2CH(NH(CO)_{1-2}R^7)-$ ($M^3$)

$-CH_2CH$(NHC(O)O-Benzyl)- ($M^4$)

$=CH(CH_2)_{1-2}R^7$ ($M^5$)

$=CHCH_2C(O)R^8$ ($M^6$)

$=CHCH_2NH(CO)_{1-2}R^7$ ($M^7$)

$=CHCH_2$NHC(O)O-Benzyl ($M^8$)

$R^3$ H, nieder-Alkyl oder -Alkenyl, Aryl, Heteroaryl, Cycloalkyl oder (Aryl, Heteroaryl oder

Cycloalkyl)-niederalkyl,

| | |
|---|---|
| $R^4$ | H, nieder-Alkyl, Aryl, Cycloalkyl, oder (Aryl oder Cycloalkyl)-niederalkyl, |
| $R^5$ | H, nieder-Alkyl oder gegebenenfalls über nieder-Alkylen gebundenes COOH, COO-nieder-Alkyl, nieder-Alkanoyl, OH, nieder-Alkanoyloxy, nieder-Alkoxy, Aryl-niederalkoxy, $CONH_2$, $CONHCH_2CH_2OH$, CONHOH, $CONHOCH_3$, CONHO-Benzyl, $CONHSO_2$-nieder-Alkyl, $CONHCH_2CH_2$-Aryl, CONH-Cycloalkyl, $CONHCH_2$-Heteroaryl, $NH_2$, NHCOO-nieder-Alkyl, NHCOO-nieder-Aralkyl, $NHSO_3H$, ($NHSO_2$ oder $NHSO_3$)-nieder-Alkyl, NH-nieder-Alkanoyl, NHCOCOOH, NHCOCOO-nieder-Alkyl, NH-Cycloalkyl, NH-(3,4-Dioxo-2-hydroxy-cyclobut-1-enyl), NH-[2-nieder-(Alkoxy oder -Alkenyloxy)-3,4-dioxocyclobut-1-enyl], $NHCH_2$-Heteroaryl, NHCOCO-(Aryl oder nieder-Alkyl), $NHCOCH_2Cl$, $NHCOCH_2O$-Aryl, $NHCOCH_2$-Aryl, NHCO-(Aryl oder Heteroaryl), $NHPO_3(R^9,R^{10})$, Heteroaryl oder gegebenenfalls durch O oder S unterbrochenes und gegebenenfalls durch bis zu 2 Substituenten aus der Gruppe von nieder-Alkyl, COOH, COO-nieder-Alkyl, $CH_2OH$ und $CH_2O$-Benzyl ringsubstituiertes $CON(CH_2)_{4-9}$, |
| $R^9$ und $R^{10}$ | H, nieder-Alkyl oder Phenyl, wobei falls Q, $R^1$, $R^2$, $R^3$ und $R^5$ gleichzeitig H sind, $R^4$ nicht Phenyl sein soll, |
| $N(R^6)$ | Benzylamino oder gegebenenfalls durch O oder S unterbrochenes und gegebenenfalls durch bis zu 2 Substituenten aus der Gruppe von nieder-Alkyl, COOH, COO-nieder-Alkyl, $CH_2OH$, $CH_2O$-Benzyl ringsubstituiertes $N(CH_2)_{4-9}$, |
| $R^7$ und $R^8$ | Aryl, Heteroaryl, Cycloalkyl oder Heterocyclyl, oder |
| $R^8$ | gegebenenfalls durch bis zu 2 Substituenten aus der Gruppe von Oxo, COO-nieder-Alkyl, $(CH_2)_{0-1}OH$, $(CH_2)_{0-1}OCO$-nieder-Alkyl, $CONH_2$, CONH-nieder-Alkyl oder CON (nieder-Alkyl)$_2$ ringsubstituiertes $N(CH_2)_{4-9}$ sind, wobei der Ausdruck "nieder" Gruppen mit bis zu 6 C-Atomen, "Aryl" gegebenenfalls durch Halogen, nieder-Alkyl, nieder-Alkoxy, OH, Phenyl, $CF_3$, $OCF_3$, Cyclopentyl, CN, COOH, $COOCH_3$, $COOC_2H_5$, $CONH_2$ oder Tetrazolyl substitutiertes Phenyl oder 1- oder 2-Naphthy] "Heteroaryl" gegebenenfalls durch nieder-Alkyl oder Halogen substituierte 5- bis 10-gliedrige aromatische Gruppen, die aus einem oder 2 Ringen bestehen und ein oder mehrere N-und/oder O-Atom(e) enthalten, und "Heterocyclyl" gegebenenfalls durch nieder-Alkyl substituierte 5- bis 10-gliedrige nicht aromatische, teilweise oder vollständig gesättigte Gruppen, die ein oder zwei Ringe und zumindest ein Heteroatom enthalten, bezeichnen, |

sowie Hydrate oder Solvate und physiologisch verträgliche Salze davon.

**2.** Verbindungen nach Anspruch 1, worin

| | |
|---|---|
| X | eine Gruppe $X^1$ ist, in der die Guanidinogruppe ungeschützt ist, |
| Y | H, |
| A | Aryl, Heteroaryl oder Heterocyclyl, |
| Q | die gleiche Bedeutung wie in Anspruch 1 hat und |
| M | entweder eine Gruppe $M^1$, in der $R^3$ und $R^4$ die gleiche Bedeutung wie in Anspruch 1 haben, wobei falls Q, $R^3$ und $R^5$ gleichzeitig H sind, $R^4$ nicht H oder Phenyl sein soll, und |
| $R^5$ | H, nieder-Alkyl oder gegebenenfalls über nieder-Alkylen gebundenes COOH, COO-nieder-Alkyl, nieder-Alkanoyl, OH, nieder-Alkanoyloxy, $NH_2$, NHCOO-nieder-Alkyl, $NHSO_3H$, ($NHSO_2$ oder $NHSO_3$)-nieder-Alkyl, NH-nieder-Alkanoyl, NHCOCOOH, NHCOCOO-nieder-Alkyl oder $NHPO_3(R^9,R^{10})$, oder falls Q nicht H ist, dann |
| M | auch eine Gruppe $M^2$ sein kann, in der $N(R^6)$ gegebenenfalls durch COOH oder COO-nieder-Alkyl ringsubstituiertes $N(CH_2)_{4-9}$ ist. |

**3.** Verbindungen nach Anspruch 1, worin Y H, X eine Gruppe $X^1$ und M eine Gruppe $M^1$ ist und, falls zumindest eins von $R^1$ und $R^2$ (in $X^1$) nicht H ist und/oder falls Q nicht H ist und/oder falls A Alkyl oder Cycloalkyl ist, dann M auch eine Gruppe $M^2$ sein kann.

**4.** Verbindungen nach Anspruch 1, worin Y H, X eine Gruppe $X^2$ und M eine Gruppe $M^1$ oder $M^2$ ist.

**5.** Verbindungen nach Anspruch 1, worin Y H, X eine Gruppe $X^1$ und M eine Gruppe $M^5$ oder $M^6$ ist, mit der Bedingung, dass zumindest eins von $R^1$ und $R^2$ (in $X^1$) nicht H ist und/oder dass Q nicht H ist und/oder dass A Alkyl oder Cycloalkyl ist.

**6.** Verbindungen nach Anspruch 1, worin Y H, X eine Gruppe $X^1$ und M eine Gruppe $M^3$ oder $M^7$ ist, mit der Bedingung, dass zumindest eins von $R^1$ und $R^2$ (in $X^1$) nicht H ist und/oder dass Q nicht H ist und/oder dass A Alkyl oder Cycloalkyl ist.

**7.** Verbindungen nach Anspruch 3, worin Y und Q H sind, X eine Gruppe $X^1$ und M eine Gruppe $M^1$ ist und, falls zumindest eins von $R^1$ und $R^2$ (in $X^1$) nicht H ist und/oder falls A Alkyl oder Cycloalkyl ist, dann M auch eine Gruppe $M^2$ sein kann.

**8.** Verbindungen nach Anspruch 3, worin Y H, Q nieder-Alkyl(OH, COOH oder COO-niederalkyl), X eine Gruppe $X^1$ und M eine Gruppe $M^1$ oder $M^2$ ist.

**9.** Verbindungen nach Anspruch 1, worin A Naphthyl, Methylchinolyl, Methyltetrahydrochinolyl, Methyl, Pyridyl oder durch t-Butyl, $CF_3$, Phenyl, Cyclopentyl, Carboxy, Methoxycarbonyl, Ethoxycarbonyl, $OCF_3$, CN, $CONH_2$ oder Tetrazolyl substituiertes Phenyl ist.

**10.** Verbindungen nach Anspruch 1, worin Q H, $CH_3$, $CH_2COOH$, $CH_2CH_2OH$ oder $CH_2COOC_2H_5$ ist.

**11.** Verbindungen nach Anspruch 1, worin X eine Gruppe $X^1$, T $CH_2$, eins von $R^1$ und $R^2$ H und das andere H oder COO-(Methyl, Ethyl, Isobutyl oder t-Butyl) ist.

**12.** Verbindungen nach Anspruch 1, worin X eine Gruppe $X^1$, T O, eins von $R^1$ und $R^2$ H und das andere H oder $COOC_2H_5$ ist.

**13.** Verbindungen nach Anspruch 1, worin X eine Gruppe $X^2$ und $R^{11}$ und $R^{21}$ H sind.

**14.** Verbindungen nach Anspruch 1, worin M eine Gruppe $M^1$, $R^3$ H, $CH_3$, Propyl, Isopropyl, Butyl, Pentyl, Allyl, Cyclopropyl, Cyclopentyl, Cyclohexyl, Cyclopropylmethyl, Cyclohexylmethyl, Pyridylmethyl oder gegebenenfalls durch Chlor oder Methoxy substituiertes Benzyl und $R^4$ H, Isopropyl, 2-Butyl, Isobutyl, Phenyl, Benzyl oder Cyclohexyl ist.

**15.** Verbindungen nach Anspruch 1 oder 14, worin $R^5$ eine Gruppe $(CH_2)_{0-2}$-$R^{50}$ und $R^{50}$ H, OH, $C(CH_3)_2OH$, $COCH_3$, $OCOCH_3$, COO-(H, $CH_3$ oder $C_2H_5$), $NHCOOCH_3$, $NHCOCH_3$, Tetrazolyl, $CONH_2$, Methyloxadiazolyl, $OCH_3$, Benzyloxy, Morpholinocarbonyl, $CONHOCH_3$, CONHO-Benzyl, $CONHSO_2CH_3$, $CONHCH_2$-Pyridyl, CONH-Cyclopropyl, $CONHCH_2CH_2$-$C_6H_3(OH)_2$, $CONHCH_2CH_2OH$, NHCOCOOH, $NHCOCOOCH_3$, $NHCOCOOC_2H_5$, $NHSO_3H$, $NHSO_2CH_3$, NHCOO-Benzyl, $NHCOCH_2Cl$, $NHCOCH_2OC_6H_5$, $NHCOCOC_6H_5$, $NHCOCOCH_3$, NHCO-Pyridyl, NHCO-Pyridyl-N-oxid, NHCO-Pyrazinyl, $NHCOCH_2C_6H_3(OH)_2$, $NHPO(OC_6H_5)_2$, $NHPO(OC_2H_5)_2$, NH-(3,4-Dioxo-2-hydroxycyclobut-1-enyl) oder NH-(2-Allyloxy-3,4-dioxocyclobut-1-enyl) ist.

**16.** Verbindungen nach Anspruch 1, worin M die Gruppe $M^2$ und N($R^6$) Hexamethylenimino ist.

**17.** Verbindungen nach Anspruch 1 oder 2 aus der Gruppe der folgenden:

N-[N4-[[(S)-1-Amidino-3-piperidinyl]methyl]-N2-(2-naphthylsulfonyl)-L-asparaginyl]-N-cyclopropylglycin,

(S)-[[3-[(S)-1-(Amino-imino-methyl)piperidin-3-ylmethylcarbamoyl]-2-(naphthalin-2-sulfonylamino)propionyl] cyclopropylamino]propionsäure,

[(S)-3-[(S)-1-(Amino-imino-methyl)piperidin-3-ylmethylcarbamoyl]-2-(4-trifluormethyl-phenylsulfonylamino)-propionyl-cyclopropyl-amino]essig-säure.

**18.** Verbindungen nach Anspruch 1 oder 2 aus der Gruppe der folgenden:

(S)-N4-[(S)-1-(Amino-imino-methyl)piperidin-3-ylmethyl]-N1-carboxymethyl-N1-cyclopentyl-2-(naphthalin-2-sulfonylamino)succinamid,

[(S)-3-[(S)-2-(Amino-imino-methyl)piperidin-3-ylmethylcarbamoyl]-2-(naphthalen-2-sulfonylamino)propionyl]-propyl-aminoessigsäure,

N-[N4-[[(S)-1-Amidino-3-piperidinyl]methyl]-N2-(2-naphthylsulfonyl)-L-asparaginyl]-N-(o-chlorbenzyl)glycin,

[2-[[(S)-3-[(S)-1-(Amino-imino-methyl)piperidin-3-ylmethylcarbamoyl]-2-(naphthalen-2-sulfonylamino)-propionyl]-butyl-amino]äthyl]oxamsäure,

(S)-N4-[(S)-1-(Amino-imino-methyl)piperidin-3-ylmethyl]-N1-butyl-2-(naphthalen-2-sulfonylamino)-N1-(2-sulfoamino-äthyl)-succinamid,

[(S)-3-[(S)-1-(Amino-imino-methyl)piperidin-3-ylmethylcarbamoyl]-2-(4 -t-butylphenylsulfonylamino)-propionyl-cyclopropyl-amino]-essigsäure.

**19.** Verbindungen nach Anspruch 1 aus der Gruppe der folgenden:

3-[[(S)-3-[(S)-1-(Amino-imino-methyl)-piperidin-3-ylcarbamoyl]-2-(4-carbamoyl-phenylsulfonylamino)-propionyl]-cyclopropyl-amino]-propionsäure,

(S)-N4-[(S)-1-(Amino-imino-methyl)-piperidin-3-ylmethyl]-N1-cyclopropyl-2-(naphthalen-2-ylsulfonylamino)-N1-[2-(pyrazin-2-ylcarbonylamino)-ethyl]-succinamid,

(S)-N4-[(S)-1-(Amino-imino-methyl)-piperidin-3-ylmethyl]-N1-cyclo-propyl-N1-[2-(3,4-dihydroxy-phenyl)-ethylcarbamoylmethyl]-2-(naphthalen-2-ylsulfonylamino)-succinamid.

**20.** Verbindungen nach Anspruch 1 aus der Gruppe der folgenden:

2-[(S)-2-[(S)-1-Amino-imino-methyl)-piperidin-3-ylmethylcarbamoyl]-1-[cyclopropyl-(2-carboxy-ethyl)-carbamoyl]-ethylsulfamoyl]-benzoesäure,

3-[[(S)-3-[(S)-1-(Amino-imino-methyl)-piperidin-3-ylcarbamoyl]-2-(4-cyano-phenylsulfonylamino)-propionyl]-cyclopropyl-amino]-propionsäure,

(S)-N(4)-[4-(Amino-imino-methyl)-morpholin-2-ylmethyl]-N(1)-cyclopropyl-N(1)-[2-(tetrazol-5-yl)-ethyl]-2-(naphthalin-2-ylsulfonyl)-succinamid,

[[(S)-3-[4-(Amino-imino-methyl)-morpholin-2-ylmethylcarbamoyl]-2-(naphthalin-2-yl-sulfonyl)-propionyl]-cyclopropyl-amino]-essigsäure-ethylester,

[[(S)-3-[4-(Amino-imino-methyl)-morpholin-2-ylmethylcarbamoyl]-2-(naphthalen-2-yl-sulfonyl)-propionyl]-cyclopropyl-amino]-essigsäure,

2-[[(S)-3-[(S)-1-(Amino-imino-methyl)-piperidin-3-ylmethylcarbamoyl]-2-(naphthalen-2-ylsulfonylamino)-propionyl]-cyclopropyl-amino]-ethyl-sulfaminsäure,

(S)-N4-[(S)-1-(Amino-imino-methyl)-piperidin-3-ylmethyl]-N1-(2-chloroacetylamino-ethyl)-N1-cyclopropyl-2-(naphthalen-2-ylsulfonylamino)-succinamid,

(S)-N4-[(S)-1-(Amino-imino-methyl)-piperidin-3-ylmethyl]-N1-cyclopropyl-2-(naphthalen-2-ylsulfonylamino)-N1-(2-phenoxyacetylamino-ethyl)-succinamid,

(S)-N4-[(S)-1-(Amino-imino-methyl)-piperidin-3-ylmethyl]-N1-cyclopropyl-2-(naphthalen-2-ylsulfonylamino)-N1-[2-(2-oxo-2-phenyl-acetylamino)-ethyl]-succinamid,

(S)-N4-[(S)-1-(Amino-imino-methyl)-piperidin-3-ylmethyl]-N1-cyclopropyl-2-(naphthalen-2-ylsulfonylamino)-N1-[2-(2-oxo-propionylamino)-ethyl]-succinamid,

(S)-N4-[(S)-1-(Amino-imino-methyl)-piperidin-3-ylmethyl]-N1-cyclopropyl-2-(naphthalen-2-ylsulfonylamino)-N1-[2-(pyridin-3-ylcarbonylamino)-ethyl]-succinamid,

(S)-N4-[(S)-1-(Amino-imino-methyl)-piperidin-3-ylmethyl]-N1-cyclopropyl-2-naphthalen-2-ylsulfonylamino-N1-[2-(1-oxy-nicotinoylamino)-ethyl]-succinamid.

**21.** Verbindungen nach einem der Ansprüche 1 bis 20 zur Verwendung als Heilmittel, insbesondere als Hemmer der durch Thrombin induzierten Plättchenaggregation und Gerinnung von Fibrinogen im Blutplasma.

**22.** Verfahren zur Herstellung der Verbindungen nach einem der Ansprüche 1 bis 20, dadurch gekennzeichnet, dass man

a) eine Säure der Formel

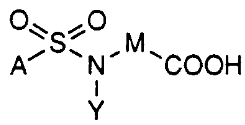

II

mit einem Amin der Formel

$$Q\text{-}NHCH_2\text{-}X \qquad III$$

oder einem Salz davon unter intermediärem Schutz von in den Gruppen A, Y und M (in II) und Q (in III) enthaltenen funktionellen Gruppen umsetzt oder

b) ein Amin der Formel

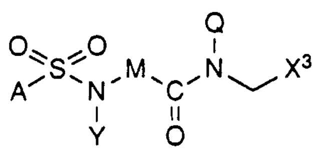

IV

worin $X^3$ eine Gruppe $X^{31}$ oder $X^{32}$ ist:

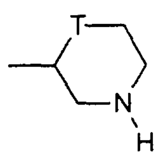

($X^{31}$)

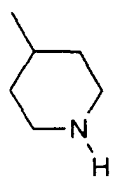

($X^{32}$)

mit einem Amidinierungsmittel umsetzt und

c) gewünschtenfalls eine in der Gruppe M oder Q einer Verbindung der Formel I enthaltene reaktionsfähige Gruppe funktionell abwandelt, und

d) gewünschtenfalls eine Verbindung der Formel I in ein physiologisch verträgliches Salz überführt oder ein Salz einer Verbindung der Formel I in die freie Säure oder Base überführt.

**23.** Pharmazeutische Präparate enthaltend eine Verbindung nach einem der Ansprüche 1 bis 20 als Wirkstoff.

**24.** Verwendung einer Verbindung nach einem der Ansprüche 1 bis 20 zur Herstellung von Arzneimitteln zur Behandlung oder Prophylaxe von Krankheiten, die durch Thrombin induzierter Plättchenaggregation oder Gerinnung von

Fibrinogen im Blutplasma verursacht sind.

**25.** Die Verbindungen der Formel III gemäss Anspruch 22, worin X eine Gruppe $X^1$ und zumindest eins von $R^1$, $R^2$ und Q nicht H ist oder worin X eine Gruppe $X^2$ ist, sowie die Verbindungen der Formel IV gemäss Anspruch 22, worin M eine Gruppe $M^1$ ist oder, falls $X^3$ eine Gruppe $X^{32}$ ist, oder falls $X^3$ eine Gruppe $X^{31}$ ist und gleichzeitig Q nicht H ist, und/oder falls A Alkyl oder Cycloalkyl ist, dann M auch eine der Gruppen $M^2$ bis $M^8$ sein kann.

## Claims

**1.** Sulphonamidocarboxamides of the formula

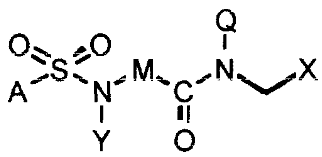 I

wherein

X is a group of formula $X^1$ or $X^2$:

($X^1$) 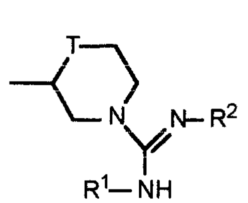 ($X^2$) 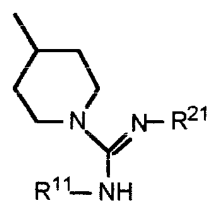

| | |
|---|---|
| T | is $CH_2$ or O, |
| $R^1$, $R^2$, $R^{11}$ and $R^{21}$ | are each independently H or COO-lower-alkyl, |
| Y | is H or, where X is a group $X^2$ or where X is a group $X^1$ in which at least one of $R^1$ and $R^2$ is not H, then Y can also be $CH_2COOH$ or $SO_2$-A', |
| A and A' | are aryl, heteroaryl, heterocyclyl, alkyl or cycloalkyl, |
| Q | is H, lower-alkyl or lower-alkyl(OH, COOH or COO-lower alkyl), |
| M | is a group of the formula $M^1$ or, where X is a group $X^2$ or where X is a group $X^1$ and at least one of $R^1$, $R^2$ and Q is not H and/or where A is aflkyl or cycloalkyl, then M can also be a group of one of the formulae $M^2$ to $M^8$: |

($M^1$) 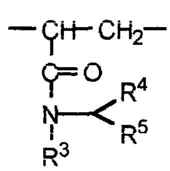 ($M^2$) 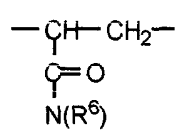

$-CH_2CH(NH(CO)_{1-2}R^7)-$ ($M^3$)

$-CH_2CH(NHC(O)O\text{-benzyl})-$ ($M^4$)

$=CH(CH_2)_{1-2}R^7$ ($M^5$)

$=CHCH_2C(O)R^8$ ($M^6$)

$=CHCH_2NH(CO)_{1-2}R^7$ ($M^7$)

$=CHCH_2NHC(O)O$-benzyl ($M^8$)

$R^3$ is H, lower-alkyl or -alkenyl, aryl, heteroaryl, cycloalkyl of (aryl, heteroaryl or cycloalkyl)-lower alkyl,

$R^4$ is H, lower-alkyl, aryl, cycloalkyl, or (aryl or cycloalkyl)-lower alkyl,

$R^5$ is H, lower-alkyl or (optionally bonded via lower-alkylene) COOH, COO-lower-alkyl, lower-alkanoyl, OH, lower-alkanoyloxy, lower-alkoxy, aryl-lower alkoxy, $CONH_2$, $CONHCH_2CH_2OH$, CONHOH, $CONHOCH_3$, CONHO-benzyl, $CONHSO_2$-lower-alkyl, $CONHCH_2CH_2$-aryl, CONH-cycloalkyl, $CONHCH_2$-heteroaryl, $NH_2$, NHCOO-lower-alkyl, NHCOO-lower-aralkyl, $NHSO_3H$, ($NHSO_2$ or $NHSO_3$)-lower-alkyl, NH-lower-alkanoyl, NHCOCOOH, NHCOCOO-lower-alkyl, NH-cycloalkyl, NH-(3,4-dioxo-2-hydroxy-cyclobut-1-enyl), NH-[2-lower-(alkoxy or -alkenyloxy)-3,4-dioxocyclobut-1-enyl], $NHCH_2$-heteroaryl, NHCOCO-(aryl or lower-alkyl), $NHCOCH_2Cl$, $NHCOCH_2O$-aryl, $NHCOCH_2$-aryl, NHCO-(aryl or heteroaryl), $NHPO_3(R^9,R^{10})$, heteroaryl or $CON(CH_2)_{4-9}$ optionally interrupted by O or S and optionally ring-substituted by up to 2 substituents from the group of lower-alkyl, COOH, COO-lower-alkyl, $CH_2OH$ and $CH_2O$-benzyl,

$R^9$ and $R^{10}$ are H, lower-alkyl or phenyl,
whereby $R^4$ must not be phenyl when Q, $R^1$, $R^2$, $R^3$ and $R^5$ are simultaneously H,

$N(R^6)$ is benzylamino or $N(CH_2)_{4-9}$ optionally interrupted by O or S and optionally ring-substituted by up to 2 substituents from the group of lower-alkyl, COOH, COO-lower-alkyl, $CH_2OH$ and $CH_2O$-benzyl,

$R^7$ and $R^8$ are aryl, heteroaryl, cycloalkyl or heterocyclyl, or

$R^8$ is $N(CH_2)_{4-9}$ optionally ring-substituted by up to 2 substituents from the group of oxo, COO-lower-alkyl, $(CH_2)_{0-1}OH$, $(CH_2)_{0-1}OCO$-lower-alkyl, $CONH_2$, CONH-lower-alkyl or $CON(lower\text{-}alkyl)_2$,

whereby the term "lower" denotes groups with up to 6 C atoms, "aryl" denotes phenyl or 1or 2-naphthyl optionally substituted by halogen, lower-alkyl, lower-alkoxy, OH, phenyl, $CF_3$, $OCF_3$, cyclopentyl, CN, COOH, $COCH_3$, $COOC_2H_5$, $CONH_2$ or tetrazolyl, "heteroaryl" denotes 5- to 10-membered aromatic groups which consist of one or two rings and contain one or more N and/or O atoms and which are optionally substituted by lower-alkyl or halogen and "heterocyclyl" denotes 5- to 10-membered non-aromatic, partially or fully saturated groups which contain one or two rings and at least one hetero atom and which are optionally substituted by lower-alkyl, as well as hydrates or solvates and physiologically compatible salts thereof.

**2.** Compounds according to claim 1, wherein

x is a group $X^1$ in which the guanidino group is unprotected,
Y is H,
A is aryl, heteroaryl or heterocyclyl,
Q has the same significance as in claim 1 and
M is either a group $M^1$ in which $R^3$ and $R^4$ have the same significance as in claim 1, whereby $R^4$ must not be H or phenyl when Q, $R^3$ and $R^5$ are simultaneously H, and
$R^5$ is H, lower-alkyl or (optionally bonded via lower-aikylene) COOH, COO-lower-alkyl, lower-alkanoyl, OH, lower-alkanoyloxy, $NH_2$, NHCOO-lower-alkyl, $NHSO_3H$, ($NHSO_2$ or $NHSO_3$)-lower-alkyl, NH-lower-alkanoyl, NHCOCOOH, NHCOCOO-lower-alkyl or $NHPO_3(R^9,R^{10})$, or, where Q is not H, then
M can also be a group $M^2$ in which $N(R^6)$ is $N(CH_2)_{4-9}$ optionally ring-substituted by COOH or COO-lower-alkyl.

**3.** Compounds according to claim 1, wherein Y is H, X is a group $X^1$ and M is a group $M^1$ and, where at least one of

$R^1$ and $R^2$ (in $X^1$) is not H and/or where Q is not H and/or where A is alkyl or cycloalkyl, then M can also be a group $M^2$.

**4.** Compounds according to claim 1, wherein Y is H, X is a group $X^2$ and M is a group $M^1$ or $M^2$.

**5.** Compounds according to claim 1, wherein Y is H, X is a group X' and M is a group $M^5$ or $M^6$, with the proviso that at least one of $R^1$ and $R^2$ (in $X^1$) is not H and/or that Q is not H and!or that A is alkyl or cycloalkyl.

**6.** Compounds according to claim 1, wherein Y is H, X is a group X' and M is a group $M^3$ or $M^7$, with the proviso that at least one of $R^1$ and 2 (in $X^1$) is not H and/or that Q is not H and/or that A is alkyl or cycloalkyl.

**7.** Compounds according to claim 3, wherein Y and Q are H, X is a group $X^1$ and M is a group $M^1$ and, where at least one of $R^1$ and $R^2$ (in $X^1$) is not H and/or where A is alkyl or cycloalkyl, then M can also be a group $M^2$.

**8.** Compounds according to claim 3, wherein Y is H, Q lower-alkyl(OH, COOH or COO-lower alkyl), X is a group $X^1$ and M is a group $M^1$ or $M^2$.

**9.** Compounds according to claim 1, wherein A is naphthyl, methylquinolyl, methyltetrahydroquinolyl, methyl, pyridyl or phenyl substituted by t-butyl, $CF_3$, phenyl, cyclopentyl, carboxy, methoxycarbonyl, ethoxycarbonyl, $OCF_3$, CN, $CONH_2$ or tetrazolyl.

**10.** Compounds according to claim 1, wherein Q is H, $CH_3$, $CH_2COOH$, $CH_2CH_2OH$ or $CH_2COOC_2H_5$.

**11.** Compounds according to claim 1, wherein X is a group $X^1$, T is $CH_2$, one of $R^1$ and $R^2$ is H and the other is H or COO-(methyl, ethyl, isobutyl or t-butyl).

**12.** Compounds according to claim 1, wherein X is a group $X^1$, T is O, one of $R^1$ and $R^2$ is H and the other is H or $COOC_2H_5$.

**13.** Compounds according to claim 1, wherein X is a group $X^2$ and $R^{11}$ and $R^{21}$ are H.

**14.** Compounds according to claim 1, wherein M is a group $M^1$, $R^3$ is H, $CH_3$, propyl, isopropyl, butyl, pentyl, allyl, cyclopropyl, cyclopentyl, cyclohexyl, cyclopropylmethyl, cyclohexylmethyl, pyridylmethyl or benzyl optionally substituted by chlorine or methoxy and $R^4$ is H, isopropyl, 2-butyl, isobutyl, phenyl, benzyl or cyclohexyl.

**15.** Compounds according to claim 1 or 14, wherein $R^5$ is a group $(CH_2)_{0-2}$-$R^{50}$ and $R^{50}$ is H, OH, $C(CH_3)_2OH$, $COCH_3$, $OCOCH_3$, COO(H, $CH_3$ or $C_2H_5$), $NHCOOCH_3$, $NHCOCH_3$, tetrazolyl, $CONH_2$, methyloxadiazolyl, $OCH_3$, benzyloxy, morpholinocarbonyl, $CONHOCH_3$, CONHO-benzyl, $CONHSO_2CH_3$, $CONHCH_2$-pyridyl, CONH-cyclopropyl, $CONHCH_2CH_2$-$C_6H_3(OH)_2$, $CONHCH_2CH_2OH$, NHCOCOOH, $NHCOCOOCH_3$, $NHCOCOOC_2H_5$, $NHSO_3H$, $NHSO_2CH_3$, NHCOO-benzyl, $NHCOCH_2Cl$, $NHCOCH_2OC_6H_5$, $NHCOCOC_6H_5$, $NHCOCOCH_3$, NHCO-pyridyl, NHCO-pyridyl N-oxide, NHCO-pyrazinyl, $NHCOCH_2C_6H_3(OH)_2$, $NHPO(OC_6H_5)_2$, $NHPO(OC_2H_5)_2$, NH-(3,4-dioxo-2-hydroxycyclobut-1-enyl) or NH- (2-allyloxy-3,4-dioxocyclobut-1-enyl).

**16.** Compounds according to claim 1, wherein M is the group $M^2$ and $N(R^6)$ is hexamethyleneimino.

**17.** Compounds according to claim 1 or 2 from the following group:

N- [N4-[[(S)-1-Amidino-3-piperidinyl] methyl]-N2-(2-naphthylsulphonyl)-L-asparaginyl] -N-cvdopropylglycine,

(S)-[[3-[(S)-1-(amino-imino-methyl)piperidin-3-ylmethylcarbamoyl]-2-(naphthalene-2-sulphonylamino)propionyl]cyclopropylamino]propionic acid,

[(S) -3-[(S)-1-(amino-imino-methyl)piperidin-3-ylmethylcarbamoyl]-2-(4-trifluoromethyl-phenylsulphonylamino)-propionyl-cyclopropyl-amino|acetic acid.

**18.** Compounds according to claim 1 or 2 from the following group:

(S)-N4-[(S)-1-(Amino-imino-methyl)piperidin-3-ylmethyl]-N1-carboxymethyl-N1-cyclopentyl-2-(naphthalene-2-sulphonylamino)succinamide,

[(S)-3-[(S)-2-(amino-imino-methyl)piperidin-3-ylmethylcarbamoyl]-2-(naphthalene-2-sulphonylamino)propionyl|-propyl-aminoacetic acid, N-[N4-[[(S)-1-amidino-3-piperidinyl]methyl]-N2-(2-naphthylsulphonyl]-L-asparaginyl]-N-(o-chlorobenzyl)glycine,

[2-[[(S)-3-[(S)-1-(amino-imino-methyl)piperidin-3-ylmethylcarbamoyl]-2-(naphthalene-2-sulphonylamino)-propionyl]-butyl-amino] ethyl]oxamic acid,

(S)-N4-[(S)-1-(amino-imino-methyl)piperidin-3-ylmethyl]-N1-butyl-2-(naphthalene-2-sulphonylamino)-N1-(2-sulphoamino-ethyl)-succinamide,

[(S)-3-[(S)-1-(amino-imino-methyl)piperidin-3-ylmethylcarbamoyl]-2-(4-t-butylphenylsulphonylamino)-propionyl-cyclopropyl-amino]-acetic acid.

**19.** Compounds according to claim 1 from the following group:

3-[[(S)-3-[(S)-1-(Amino-imino-methyl)-piperidin-3-ylcarbamoyl]-2-(4-carbamoyl-phenylsulphonylamino)-propionyl]-cyclopropyl-amino]-propionic acid,

(S)-N4-[(S)-1-(amino-imino-methyl)-piperidin-3-ylmethyl]-N1-cyclopropyl-2-(naphthalen-2-ylsulphonylamino)-N1-|2-(pyrazin-2-ylcarbonylamino) -ethyl|-succinamide,

(S) -N4- [(S)-1-(amino-imino-methyl) -piperidin-3-ylmethyl]-N1-cyclopropyl-N1-[2-(3,4-dihydroxy-phenyl)-ethylcarbamoylmethyl]-2-(naphthalen-2-ylsulphonylamino)-succinamide.

**20.** Compounds according to claim 1 from the following group:

2- [(S)-2-[(S)-1-Amino-imino-methyl) -piperidin-3-ylmethylcarbamoyl]-1-[cyclopropyl-(2-carboxy-ethyl)-carbamoyl]-ethylsulphamoyl]-benzoic acid,

3-[[(S)-3-[(S)-1-(amino-imino-methyl)-piperidin-3-ylcarbamoyl]-2-(4-cyano-phenylsulphonylamino)-propionyl|-cyclopropyl-amino|-propionic acid,

(S)-N4-[4-(amino-imino-methyl)-morpholin-2-ylmethyl]-N(1)-cyclopropyl-N(1)-[2-(tetrazol-5-yl)-ethyl]-2-(naphthalen-2-ylsulphonyl)-succinamide,

ethyl [[(S)-3-[4-(amino-imino-methyl)-morpholin-2-ylmethylcarbamoyl]-2-(naphthalen-2-ylsulphonyl)-propionyl]-cyclopropyl-amino]-acetate,

[[(S)-3-[4-(amino-imino-methyl)-morpholin-2-ylmethylcarbamoyl]-2-(naphthalen-2-ylsulphonyl)-propionyl] -cyclopropyl-amino] -acetic acid,

2-[[(S)-3-[(S)-1-(amino-imino-methyl)-piperidin-3-ylmethylcarbamoyl]-2-(naphthalen-2-ylsulphonylamino)-propionyl]-cyclopropyl-amino]-ethylsulphamic acid,

(S)-N4-[(S)-1-(amino-imino-methyl)-piperidin-3-yimethyl]-N1-(2-chloro-acetylamino-ethyl)-N1-cyclopropyl-2-(naphthalen-2-ylsulphonylamino)-succinamide,

(S)-N4-[(S)-1-(amino-imino-methyl)-piperidin-3-ylmethyl]-N1-cyciopropyl-2-(naphthalen-2-ylsulphonylamino)-N1-(2-phenoxyacetylamino-ethyl)-succinamide,

(S)-N4-[(S)-1-(amino-imino-methyl)-piperidin-3-ylmethyl]-N1-cyclopropyl-2-(naphthalen-2-ylsulphonylamino)-N1-[2-(2-oxo-2-phenyl-acetylamino)-ethyl]-succinamide,

(S) -N4-[(S)-1-(amino-imino-methyl)-piperidin-3-ylmethyl] -N1-cyclopropyl-2-(naphthalen-2-ylsulphonylamino)-N1-[2-(2-oxo-propionylamino)-ethyl]-succinamide,

(S)-N4-[(S)-1-(amino-imino-methyl)-piperidin-3-ylmethyl] -N-cyclopropyl-2-(naphthalen-2-ylsulphonylamino)-N1-[2-(pyridin-3-ylcarbonylamino)-ethyl]-succinamide,

(S)-N4-[(S)-1-(amino-imino-methyl)-piperidin-3-ylmethyl]-N1-cyclopropyl-2-naphthalen-2-ylsulphonylamino-N1- [2-(1-oxy-nicotinoylamino)-ethyl]-succinamide.

**21.** Compounds according to anyone of claims 1 to 20 for use as medicaments, especially as inhibitors of thrombin-induced platelet aggregation and clotting of fibrinogen in blood plasma.

**22.** A process for the manufacture of the compounds according to any one of claims 1 to 20, characterized by

a) reacting an acid of the formula

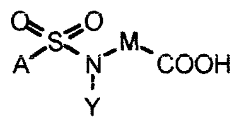

II

with an amine of the formula

$$Q\text{-}NHCH_2\text{-}X \qquad III$$

or a salt thereof, with intermediary protection of functional groups present in the groups A, Y and M (in II) and Q (in III), or

b) reacting an amine of the formula

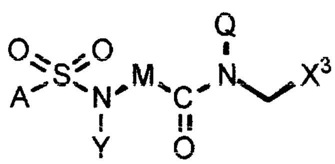

IV

wherein $X^3$ is a group $X^{31}$ or $X^{32}$:

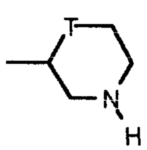

($X^{31}$)

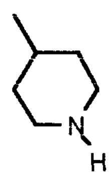

($X^{32}$)

with an amidinating agent, and

c) if desired, functionally modifying a reactive group present in group M or Q of a compound of formula I, and

d) if desired, converting a compound of formula I into a physiologically compatible salt or converting a salt of a compound of formula I into the free acid or base.

**23.** Pharmaceutically preparations containing a compound according to any one of claims 1 to 20 as the active ingredient.

**24.** The use of a compound according to any one of claims 1 to 20 for the production of medicaments for the treatment of prophylaxis of illnesses which are caused by thrombin-induced platelet aggregation or clotting of fibrinogen in blood plasma.

**25.** The compounds of formula III in accordance with claim 22, wherein X is a group $X^1$ and at least one of $R^1$, $R^2$ and Q is not H or wherein X is a group $X^2$, as well as the compounds of formula IV in accordance with claim 22, wherein M is a group $M^1$ or, where $X^3$ is a group $X^{32}$ or where $X^3$ is a group $X^{31}$ and simultaneously Q is not H and/or where A is alkyl or cycloalkyl, then M can also be one of the groups $M^2$ to $M^8$.

## Revendications

**1.** Sulfonamidocarboxamides de formule :

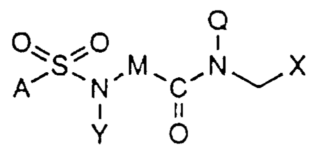 I

dans laquelle

X représente un groupe de formule $X^1$ ou $X^2$ :

($X^1$) 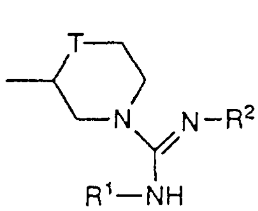 ($X^2$) 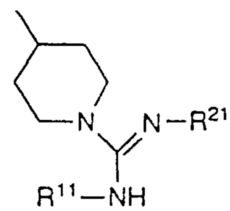

T représente $CH_2$ ou O,

$R^1$, $R^2$, $R^{11}$ et $R^{21}$ représentent indépendamment l'un de l'autre H ou un COO-alkyle inférieur,

Y représente H ou si X représente un groupe $X^2$ ou si X représente un groupe $X^1$ dans lequel au moins l'un des radicaux $R^1$ ou $R^2$ ne représente pas H, alors Y peut également représenter $CH_2COOH$ ou $SO_2$-A',

A et A' représentent un aryle, hétéroaryle, hétérocyclyle, alkyle ou cycloalkyle,

Q représente H, un alkyle inférieur ou un alkyle inférieur (OH, COOH ou COO-alkyle inférieur),

M représente un groupe de formule $M^1$ ou, si X est un groupe $X^2$, ou si X est un groupe $X^1$ et au moins l'un des radicaux $R^1$, $R^2$ et Q ne représentent pas H, et/ou si A représente un alkyle ou un cycloalkyle, alors M peut également être un groupe d'une des formules $M^2$ à $M^8$,

($M^1$) 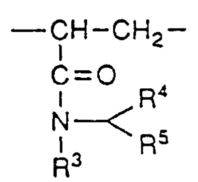 ($M^2$) 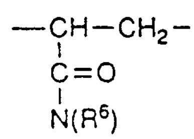

$-CH_2CH(NH(CO)_{1-2}R^7)-$ $(M^3)$

$-CH_2CH(NHC(O)O\text{-benzyl})-$ $(M^4)$

$= CH(CH_2)_{1-2}R^7$ $(M^5)$

$=CHCH_2C(O)R^8$ $(M^6)$

$=CHCH_2NH(CO)_{1-2}R^7$ $(M^7)$

$=CHCH_2NHC(O)O\text{-benzyle}$ $(M^8)$

$R^3$ représente H, un alkyle ou alcényle inférieur, un aryle, un hétéroaryle, un cycloalkyle ou un (aryle, hétéroaryle ou cycloalkyle)-alkyle inférieur,

$R^4$ représente H, un alkyle inférieur, un aryle, un cycloalkyle ou un (aryle ou cycloalkyle)-alkyle inférieur,

$R^5$ représente H, un alkyle inférieur ou un radical, éventuellement lié par l'intermédiaire d'un alkylène inférieur, qui est un COOH, COO-alkyle inférieur, alcanoyle inférieur, OH, alcanoyloxy inférieur, alcoxy inférieur, arylalcoxy inférieur, $CONH_2$, $CONHCH_2CH_2OH$, CONHOH, $CONHOCH_3$, CONHO-benzyle, $CONHSO_2$-alkyle inférieur, $CONHCH_2CH_2$-aryle, CONH-cycloalkyle, $CONHCH_2$-hétéroaryle, $NH_2$, NHCOO-alkyle inférieur, NHCOO-aralkyle inférieur, $NHSO_3H$, ($NHSO_2$ ou $NHSO_3$)-alkyle inférieur, NH-alcanoyle inférieur, NHCOCOOH, NHCOCOO-alkyle inférieur, NH-cyclo-alkyle, NH-(3,4-dioxo-2-hydroxycyclobut-1-ényle), NH-[2-(alcoxy ou alcényl-oxy inférieur)-3,4-dioxocyclobut-1-ényle], $NHCH_2$-hétéroaryle, NHCOCO-(aryle ou alkyle inférieur), $NHCOCH_2Cl$, $NHCOCH_2O$-aryle, $NHCOCH_2$-aryle, NHCO-(aryle ou hétéroaryle), $NHPO_3(R^9,R^{10})$, un hétéroaryle ou $CON(CH_2)_{4-9}$, éventuellement interrompu par O ou S et éventuellement substitué dans le noyau par jusqu'à deux substituants du groupe constitué par alkyle inférieur, COOH, COO-alkyle inférieur, $CH_2OH$ et $CH_2O$-benzyle,

$R^9$ et $R^{10}$ représentent H, un alkyle inférieur ou un phényle,

où si Q, $R^1$, $R^2$, $R^3$ et $R^5$ représentent simultanément H, $R^4$, ne doit pas être un phényle,

$N(R^6)$ représente un benzylamino ou $N(CH_2)_{4-9}$ éventuellement interrompu par O ou S et éventuellement substitué dans le noyau par jusqu'à deux substituants du groupe constitué par alkyle inférieur, COOH, COO-alkyle inférieur, $CH_2OH$, $CH_2O$-benzyle,

R7 et R8 représentent un aryle, un hétéroaryle, un cycloalkyle ou un hétéro-cyclyle ou

$R^8$ représente un $N(CH_2)_{4-9}$ éventuellement substitué dans le noyau par jusqu'à deux substituants du groupe constitué par oxo, COO-alkyle inférieur, $(CH_2)_{0-1}$-OH, $(CH_2)_{0-1}$OCO-alkyle inférieur, $CONH_2$, CONH-alkyle inférieur ou CON-alkyle inférieur$)_2$, où l'expression "inférieur" désigne des groupes comportant jusqu'à 6 atomes de carbone, "aryle" représente un phényle ou un 1- ou 2-naphtyle éventuellement substitué par un halogène, un alkyle inférieur, un alcoxy inférieur, OH, un phényle, $CF_3$, $OCF_3$, un cyclopentyle, CN, COOH, $COOCH_3$, $COOC_2H_3$, $CONH_2$, ou un tétrazolyle, "hétéroaryle" représente des groupes aromatiques à 5 à 10 chaînons éventuellement substitués par un alkyle inférieur ou un halogène, qui se composent de un ou deux noyaux et contiennent un ou plusieurs atomes d'azote et/ou d'oxygène, et "hétéro-cyclyle" représente des groupes non aromatiques, partiellement ou entièrement saturés, à 5 à 10 chaînons, éventuellement substitués par un alkyle inférieur, qui contiennent un ou deux noyaux, et au moins un hétéroatome,
ainsi que leurs hydrates ou produits de solvatation et leurs sels physiologiquement acceptables.

**2.** Composés selon la revendication 1, dans lesquels :

X représente un groupe $X^1$ dans lequel le groupe guanidino est non protégé, Y représente H,

A représente un aryle, un hétéroaryle ou un hétérocyclyle,

Q a la même signification que dans la revendication 1, et

M représente ou bien un groupe $M^1$ dans lequel $R^3$ et $R^4$ ont la même signification que dans la revendication 1, où si Q, $R^3$ et $R^5$ représentent simultanément H, $R^4$ ne représente pas H ou un phényle, et

$R^5$ représente H, un alkyle inférieur ou un COOH éventuellement lié par l'intermédiaire d'un alkylène inférieur, un COO-alkyle inférieur, un alcanoyle inférieur, OH, un alcanoyloxy inférieur, un $NH_2$, un NHCOO-alkyle inférieur, $NHSO_3H$, ($NHSO_2$ ou $NHSO_3$)-alkyle inférieur, NH-alcanoyle inférieur, NHCOCOOH, NHCOCOO-alkyle inférieur ou $NHPO_3(R^9,R^{10})$, ou si Q ne représente pas H, alors

M peut également représenter un groupe $M^2$, dans lequel $N(R^6)$ est un $N(CH_2)_{4-9}$ éventuellement substitué dans le noyau par COOH ou un COO-alkyle inférieur.

**3.** Composés selon la revendication 1, dans lesquels Y représente H, X représente un groupe $X^1$ et M un groupe $M^1$ et, si au moins l'un des radicaux $R^1$ et $R^2$ (dans $X^1$) n'est pas un hydrogène, et/ou si Q ne représente pas H et/ou si A est un alkyle ou un cycloalkyle, alors M peut également représenter un groupe $M^2$.

**4.** Composés selon la revendication 1, dans lesquels Y représente H, X un groupe $X^2$ et M un groupe $M^1$ ou $M^2$.

**5.** Composés selon la revendication 1, dans lesquels Y représente H, et X un groupe $X^1$ et M un groupe M5 ou $M^6$, sous réserve qu'au moins l'un des radicaux $R^1$ ou $R^2$ (dans $X^1$) ne représente pas H et/ou que Q ne représente pas H et/ou que A représente un alkyle ou un cycloalkyle.

**6.** Composés selon la revendication 1, dans lesquels Y représente H, X représente un groupe $X^1$ et M représente un groupe M3 ou $M^7$, sous réserve qu'au moins l'un des radicaux $R^1$ et $R^2$ (dans $X^1$) ne représente pas H ou que Q ne représente pas H et/ou que A représente un alkyle ou un cycloalkyle.

**7.** Composés selon la revendication 3, dans lesquels Y et Q représentent H, X représente un groupe $X^1$ et M un groupe $M^1$ et, si au moins un des radicaux $R^1$ et $R^2$ (dans $X^1$) ne représente pas H et/ou si A représente un alkyle ou un cycloalkyle, alors M peut également représenter un groupe $M^2$.

**8.** Composés selon la revendication 3, dans lesquels Y représente H, Q représente un alkyle inférieur (OH, COOH ou COO-alkyle inférieur), X représente un groupe $X^1$ et M représente un groupe $M^1$ ou $M^2$.

**9.** Composés selon la revendication 1, dans lesquels A représente un naphtyle, un méthylquinoléyle, un méthyltétrahydroquinoléyle, un méthyle, un pyridyle ou un phényle substitué par un t-butyle, $CF_3$, un phényle, un cyclopentyle, un carboxy, un méthoxycarbonyle, un éthoxycarbonyle, $OCF_3$, CN, $CONH_2$ ou un tétrazolyle.

**10.** Composés selon la revendication 1, dans lesquels Q représente H, $CH_3$, $CH_2COOH$, $CH_2CH_2OH$ ou $CH_2COOC_2H_5$.

**11.** Composés selon la revendication 1, dans lesquels X représente un groupe $X^1$, T représente $CH_2$, l'un des radicaux $R^1$ et $R^2$ représente H et l'autre représente H ou un COO-(méthyle, éthyle, isobutyle ou t-butyle).

**12.** Composés selon la revendication 1, dans lesquels X représente un groupe $X^1$, T représente O, l'un des radicaux $R^1$ et $R^2$ représente H et l'autre représente H ou $COOC_2H_5$.

**13.** Composés selon la revendication 1, dans lesquels X représente un groupe $X^2$ et $R^{11}$ et $R^{21}$ représentent H.

**14.** Composés selon la revendication 1, dans lesquels M représente un groupe M', $R^3$ représente H, $CH_3$, un propyle, isopropyle, butyle, pentyle, allyle, cyclo-propyle, cyclopentyle, cyclohexyle, cyclopropylméthyle, cyclohexylméthyle, pyridylméthyle ou un benzyle éventuellement substitué par un chlore ou un méthoxy et $R^4$ représente H, un isopropyle, 2-butyle, isobutyle, phényle, benzyle ou cyclohexyle.

**15.** Composés de la revendication 1 ou 14, dans lesquels $R^5$ représente un groupe $(CH_2)_{0-2}$-$R^{50}$ et $R^{50}$ représente H, OH, $C(CH_3)_2OH$, $COCH_3$, OCO-$CH_3$, COO-(H-$CH_3$ ou $C_2H_5$), $NHCOOCH_3$, $NHCOCH_3$, un tétrazolyle, CO-$NH_2$, un méthyloxadiazolyle, $OCH_3$, un benzyloxy, morpholinocarbonyle, $CONHOCH_3$, CONHO-benzyle, $CONHSO_2CH_3$, $CONHCH_2$-pyridyle, CO-NH-cyclopropyle, $CONHCH_2CH_2C_6H_3(OH)_2$, $CONHCH_2CH_2OH$, NHCO-COOH, $NHCOCOOCH_3$, $NHCOCOOC_2H_5$, $NHSO_3H$, $NHSO_2CH_3$, NH-COO-benzyle, $NHCOCH_2Cl$, $NHCOCH_2OC_6H_5$, $NHCOCOC_6H_5$, NHCO-$COCH_3$, NHCO-pyridyle, NHCO-pyridyl-N-oxyde, NHCO-pyrazinyle, NH-$COCH_2C_6H_3(OH)_2$, $NHPO(OC_6H_5)_2$, $NHPO(OC_2H_5)_2$, NH-(3,4-dioxo-2-hydroxycyclobut-1-ényle) ou NH-(2-allyloxy-3,4-dioxocyclobut-1-ényle).

**16.** Composés selon la revendication 1, dans lesquels M représente le groupe $M^2$ et $N(R^6)$ est un hexaméthylène imino.

**17.** Composés selon la revendication 1 ou 2 du groupe des composés suivants :

N-[N4-[[(S)-1-amidino-3-pipéridinyl]-méthyl]-N2-(2-naphtylsulfonyl)-L-asparginyl]-N-cyclopropylglycine,

acide (S)-[[3-[(S)-1-aminoiminométhyl)-pipéridin-3-ylméthylcarbamoyl]-2-(naphtalène-2-sulfonylamino)-propionyl]-cyclopropylamino]-propionique,

acide [(S)-3-[(S)-1-aminoiminométhyl)-pipéridin-3-ylméthylcarbamoyl]-2-(4-trifluorométhylphénylsulfonylamino)-propionyl-cyclopropylamino]-acétique.

**18.** Composés selon la revendication 1 ou 2 du groupe des composés suivants :

(S)-N4-[(S)-1-(aminoiminométhyl)-pipéridin-3-ylméthyl]-N1-carboxyméthyl-N 1-cyclopentyl-2-(naphtalène-2-sulfonylamino)-succinamide,

acide [(S)-3-[(S)-2-(aminoiminométhyl)-pipéridin-3-ylméthylcarbamoyl]-2-(naphtaiène-2-sulfonylamino)-propionyl]-propylaminoacétique,

N-[N4-[[(S)-1-amidino-3-pipéridinyl]-méthyl]-N2-(2-naphtylsulfonyl)-L-asparginyl]-N-(o-chlorobenzyl)-glycine,

acide [2-[[(S)-3-[(S)-1-(aminoiminométhyl)-pipéridin-3-ylméthylcarbamoyl]-2-(naphtalène-2-sulfonylamino)-propionyl]-butylamino]-éthyl]-oxamique, (S)-N4-[(S)-1-(aminoiminométhyl)-pipéridin-3-ylméthyl]-N1-butyl-2-(naphtalène-2-sulfonylamino)-N1-(2-sulfoaminoéthyl)-succinamide,

acide [(S)-3-[(S)-1-(aminoiminométhyl)-pipéridine-3-ylméthylcarbamoyl]-2-(4-t-butylphénylsulfonylamino)-propionylcyclopropylamino]-acétique.

**19.** Composés selon la revendication 1 du groupe des composés suivants :

acide 3-[[(S)-3-[(S)-1-(aminoiminométhyl)-pipéridin-3-ylcarbamoyl]-2-(4-carbamoylphénylsulfonylamino)-propionyl]-cyclopropylamino]-propionique,

(S)-N4-[(S)-1-(aminoiminométhyl)-pipéridin-3-ylméthyl]-N1-cyclopropyl-2-(naphtalène-2-ylsulfonylamino)-N1-[2-(pyrazin-2-ylcarbonylamino)-éthyl]-succinamide,

(S)-N4-[(S)-1-(aminoiminométhyl)-pipéridin-3-ylméthyl]-N1-cyclopropyl-N1-[2-(3,4-dihydroxyphényl)-éthylcarbamoylméthyl]-2-(naphtalène-2-ylsulfonylamino)-succinamide.

**20.** Composés selon la revendication 1 du groupe des composés suivants :

acide 2-[(S)-2-[(S)-1-(aminoiminométhyl)-pipéridin-3-ylméthylcarbamoyl]-1-[cyclopropyl-(2-carboxyéthyl)-carbamoyl]-éthylsulfamoyl]-benzoïque,

acide 3-[[(S)-3-[(S)-1-(aminoiminométhyl)-pipéridin-3-carbamoyl]-2-(4-cyanophénylsulfonylamino)-propionyl]-cyclopropylamino]-propionique,

(S)-N(4)-[4-(aminoiminométhyl)-morpholin-2-ylméthyl]-N(1)-cyclopropyl-N(1)-[2(tétrazol-5-yl)-éthyl]-2-(naphtalène-2-ylsulfonyl)-succinamide,

ester éthylique de l'acide [[(S)-3-[4-(aminoiminométhyl)-morpholino-2-ylméthylcarbamoyl]-2-(naphtalène-2-ylsulfonyl)-propionyl]-cyclopropylamino]-acétique,

acide [[(S)-3-[4-(aminoiminométhyl)-morpholino-2-ylméthylcarbamoyl]-2-(naphtalène-2-ylsulfonyl)-propionyl]-cyclopropylamino]acétique,

acide 2-[[(S)-3-[(S)-1-(aminoiminométhyl)-pipéridin-3-ylméthylcarbamoyl]-2-(naphtalène-2-ylsulfonylamino)-propionyl]-cyclopropylamino]-éthylsulfamique,

(S)-N4-[(S)-1-(aminoiminométhyl)-pipéridin-3-ylméthyl]-N1-(2-chloro-acétylaminoéthyl)-N 1 -cyclopropyl-2-(naphtalène-2-ylsulfonyl-amino)-succinamide,

(S)-N4-[(S)-1-(aminoiminométhyl)-pipéridin-3-ylméthyl]-N1-cyclopropyl-2-(naphtalène-2-ylsulfonylamino)-N1-(2-phénoxyacétyl-aminoéthyl)-succinamide,

(S)-N4-[(S)-1-(aminoiminométhyl)-pipéridin-3-ylméthyl]-N1-cyclopropyl-2-(naphtalène-2-ylsulfonylamino)-N1-[2-(2-oxo-2-phénylacétylamino)-éthyl]-succinamide,

(S)-N4-[(S)-1-(aminoiminométhyl)-pipéridine-3-ylméthyl]-N1-cyclopropyl-2-(naphtalène-2-ylsulfonylamino)-N1-[2-(2-oxopropionyl-amino)-éthyl]-succinamide,

(S)-N4-[(S)-1-(aminoiminométhyl)-pipéridin-3-ylméthyl]-N1-cyclopropyl-2-(naphtalène-2-ylsulfonylamino)-N1-[2-(pyridin-3-ylcarbonyl-amino)-éthyl]-succinamide,

(S)-N4-[(S)-1-(aminoiminométhyl)-pipéridin-3-ylméthyl]-N1-cyclopropyl-2-naphtalène-2-ylsulfonylamino-N1-[2-(1-oxynicotinoyl-amino)-éthyl]-succinamide.

**21.** Composés selon l'une des revendications 1 à 20 aux fins d'utilisation comme médicaments, en particulier comme inhibiteurs de l'agglutination des plaquettes induite par la thrombine et de la coagulation du fibrinogène dans le plasma sanguin.

**22.** Procédé de préparation des composés selon l'une des revendications 1 à 20, caractérisé en ce que :

a) on fait réagir un acide de formule :

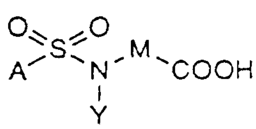

II

avec une amine de formule :

$$Q\text{-}NHCH_2\text{-}X \qquad III$$

ou un de ses sels avec protection intermédiaire des groupes fonctionnels contenus dans l'un des groupes A, Y et M (dans II) et Q (dans III) ou

b) on fait réagir une amine de formule :

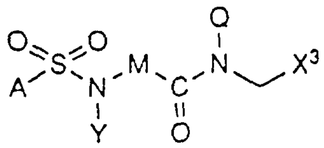

IV

dans laquelle $X^3$ est un groupe $X^{31}$ ou $X^{32}$ ;

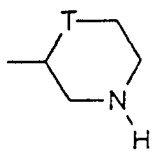

($X^{31}$)

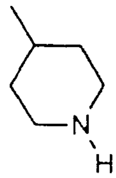

($X^{32}$)

avec un agent d'amidination et

c) si on le désire on transforme fonctionnellement un groupe réactif contenu dans le groupe M ou Q d'un composé de formule I, et

d) si on le désire on transforme un composé de formule I en un sel physiologiquement acceptable ou un sel d'un composé de formule I en l'acide ou la base libre.

**23.** Préparations pharmaceutiques contenant un composé selon l'une des revendications 1 à 20 comme substance active.

**24.** Utilisation d'un composé selon l'une des revendications 1 à 20 pour la préparation de médicaments de traitement ou de prophylaxie des maladies provoquées par l'agglutination des plaquettes induite par la thrombine ou par la coagulation du fibrinogène dans le plasma sanguin.

**25.** Les composés de formule III selon la revendication 22, dans lesquels X représente un groupe $X^1$ et au moins l'un des radicaux $R^1$, $R^2$ et Q ne représente pas H ou bien où X est un groupe $X^2$, ainsi que les composés de formule IV selon la revendication 22, dans lesquels M est un groupe $M^1$, ou, si $X^3$ est un groupe $X^{32}$, ou bien si $X^3$ est un groupe $X^{31}$ et simultanément Q ne représente pas H, et/ou si A représente un alkyle ou un cycloalkyle, alors M peut également être l'un des groupes $M^2$ à $M^8$.